(19) Europäisches Patentamt
European Patent Office
Office européen des brevets

(11) EP 4 387 412 B1

(12) **EUROPEAN PATENT SPECIFICATION**

(45) Date of publication and mention of the grant of the patent:
**09.04.2025 Bulletin 2025/15**

(21) Application number: **22213851.3**

(22) Date of filing: **15.12.2022**

(51) International Patent Classification (IPC):
***H10K 50/14*** *(2023.01)* ***H10K 50/15*** *(2023.01)*
***H10K 50/16*** *(2023.01)* ***H10K 50/165*** *(2023.01)*
***H10K 50/19*** *(2023.01)* ***H10K 85/60*** *(2023.01)*

(52) Cooperative Patent Classification (CPC):
**H10K 85/654; H10K 85/615; H10K 85/6572;**
H10K 50/16; H10K 50/19; H10K 71/30

(54) **ORGANIC ELECTROLUMINESCENT DEVICE AND DISPLAY DEVICE COMPRISING THE ORGANIC ELECTROLUMINESCENT DEVICE**

ORGANISCHE ELEKTROLUMINESZENZVORRICHTUNG UND ANZEIGEVORRICHTUNG MIT DER ORGANISCHEN ELEKTROLUMINESZENZVORRICHTUNG

DISPOSITIF ÉLECTROLUMINESCENT ORGANIQUE ET DISPOSITIF D'AFFICHAGE COMPRENANT LE DISPOSITIF ÉLECTROLUMINESCENT ORGANIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**19.06.2024 Bulletin 2024/25**

(73) Proprietor: **Novaled GmbH**
**01099 Dresden (DE)**

(72) Inventors:
- **HUANG, Qiang**
  **01099 Dresden (DE)**
- **SCHOLZ, Johannes**
  **01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

(56) References cited:
**EP-A1- 3 828 951**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

The present invention relates to an organic electroluminescent device and to a display device comprising the organic electroluminescent device.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode (organic electroluminescent device) may be affected by characteristics of an organic semiconductor layer comprised therein, and among them, may be affected by characteristics of an organic material of the organic semiconductor layer.

Particularly, development of an organic semiconductor layer arrangement being capable of improving electron transport, electron injection and electron generation properties is needed. Thereby, operating voltage in OLEDs may be reduced. Lower operating voltage is important for reduced power consumption and improved battery life, esp. of mobile devices.

Further, development of an organic electronic device with improved efficiency is needed. Increased efficiency is important for reducing power consumption and increasing battery life, for example of a mobile display device.

There remains a need to improve the performance of organic electroluminescent devices, in particular to achieve a multi-stack OLED with a low operational voltage, high efficiency and a low voltage rise over time.

The document EP3828951 A1 discloses an organic electroluminescent device comprising a charge generation layer.

### DISCLOSURE

An aspect of the present invention provides an organic electroluminescent device comprising an anode layer, a cathode layer, a first emission layer, a second emission layer, a first charge generation layer, and a first electron transport layer,
wherein

- the first charge generation layer is arranged between the first emission layer and the second emission layer;
- the first electron transport layer is arranged between the first emission layer and the second emission layer;
- the first charge generation layer comprises a first n-type charge generation layer and a first p-type charge generation layer;
- the first n-type charge generation layer is closer to the anode layer than the first p-type charge generation layer and the first p-type charge generation layer is closer to the cathode layer than the first n-type charge generation layer;
- the first electron transport layer is arranged in direct contact with the first n-type charge generation layer;
- the first electron transport layer is free of 8-hydroxyquinolinolato-lithium;
- the first n-type charge generation layer comprises a metal dopant and the metal dopant is Yb;
- the first n-type charge generation layer comprises a compound of formula (I)

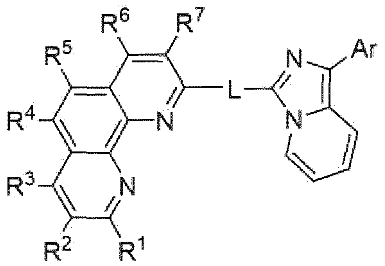

(I);

wherein in formula (I)

- Ar is selected from substituted or unsubstituted $C_6$ to $C_{24}$ aryl or substituted or unsubstituted $C_2$ to $C_{24}$ heteroaryl;

- $R^1$ to $R^7$ are independently selected from H, D, substituted or unsubstituted $C_1$ to $C_{16}$ alkyl, substituted or unsubstituted $C_6$ to $C_{24}$ aryl or substituted or unsubstituted $C_2$ to $C_{24}$ heteroaryl, CN, halogen, F, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, or $PX^1(R^8)_2$ wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O; and

- L is selected from substituted or unsubstituted $C_6$ to $C_{24}$ aryl, substituted or unsubstituted $C_2$ to $C_{24}$ heteroaryl.

Another aspect of the present invention provides a display device comprising the organic electroluminescent device according to the present invention.

It should be noted that throughout the application and the claims any generic labeling, such as $R^n$, Ar or L always refer to the same moieties, unless otherwise noted.

In the present specification, when a definition is not otherwise provided, "substituted" refers to one substituted with a deuterium, $C_1$ to $C_{12}$ alkyl and $C_1$ to $C_{12}$ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a $C_1$ to $C_{12}$ alkyl group. More specifically, the alkyl group may be a $C_1$ to $C_{10}$ alkyl group or a $C_1$ to $C_6$ alkyl group. For example, a $C_1$ to $C_4$ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenylyl, and polycyclic groups comprising fused rings, like naphtyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one

ring hydrogen from a saturated cycloalkyl ring in a compound comprising at least one such ring.

The term "fused aryl rings" or "condensed aryl rings" is understood the way that two aryl rings are considered fused or condensed when they share at least two common sp2-hybridized carbon atoms In the present specification, the single bond refers to a direct bond.

In the context of the present invention, "different" means that the compounds do not have an identical chemical structure.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

**Advantageous Effects**

It was surprisingly found that a multi-stack OLED comprising an n-type charge generation layer comprising Ytterbium as metal dopant a compound comprising a imidazo[1,5-a]pyridine group and a phenanthroline group as the host (matrix) in direct contact with a LiQ-free electron transport layer has an reduced voltage rise over time.

Especially, the use of a compound comprising a imidazo[1,5-a]pyridine group and phenanthroline group as n-CGL matrix for Ytterbium and an LiQ-free ETL contacting directly said n-CGL results synergistically in an improved voltage increase over time of a multi-stack OLED.

First charge generation layer

The thickness of the first n-type charge generation layer may be in the range from 0.5 nm to 50 nm; alternatively in the range from 1 nm to about 40 nm; or alternatively in the range of 2 nm to 30 nm; or alternatively 5 nm to 15 nm.

The first charge generation layer comprises a first n-type charge generation layer and a first p-type charge generation layer. The first n-type charge generation layer comprises a compound of formula (I)

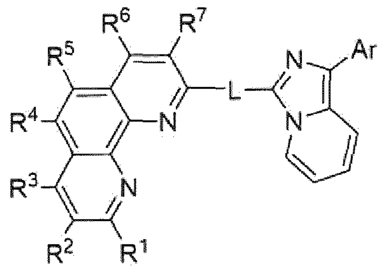

(I).

According to an embodiment, the compound of formula (I) is present in the first n-type charge generation layer in an amount of ≥0.1 wt.-%, preferably ≥1 wt.-%, preferably ≥2 wt.-%, more preferably ≥3 wt.-%, more preferably ≥5 wt.-%, more preferably ≥8 wt.-%, more preferably ≥20 wt.-%, more preferably ≥30 wt.-%, more preferably ≥40 wt.-%, more preferably ≥50 wt.-%, based on the total weight of the first n-type charge generation layer.

According to an embodiment, the metal dopant is present in the first n-type charge generation layer in amount of ≤99.9 wt.-%, preferably ≤99 wt.-%, more preferably ≤98 wt.-%, more preferably ≤97 wt.-%, more preferably ≤95 wt.-%, more preferably ≤92 wt.-%, more preferably ≤80 wt.-%, more preferably ≤70 wt.-%, more preferably ≤60 wt.-%, more preferably ≤55 wt.-%, more preferably ≤50 wt.-%, based on the total weight of the first n-type charge generation layer.

According to an embodiment, the compound of formula (I) is present in the first n-type charge generation layer in an amount of ≥ 50 wt.-% to ≤99.9 wt.-%, preferably ≥50 wt.-% to ≤99 wt.-%, more preferably ≥50 wt.-% to ≤98 wt.-%, more preferably ≥50 wt.-% to ≤ 97 wt.-% to, based on the total weight of the first n-type charge generation layer.

According to an embodiment, the metal dopant is present in the first n-type charge generation layer in amount of ≥0.1 wt.-% to ≤50 wt.-%, preferably ≥1 wt.-% to ≤50 wt.-%, more preferably ≥2 wt.-% to ≤50 wt.-%, more preferably ≥3 wt.-% to ≤50 wt.-%, based on the total weight of the first n-type charge generation layer.

According to one embodiment of the present invention the first n-type charge generation layer is non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

The metal dopant in terms of the present disclosure may be a redox n-dopant. Under a redox n-dopant, it is understood a compound which, if embedded into an electron transport matrix, improves, in comparison with the neat matrix under the same physical conditions, the electronic properties of the formed organic material, in particular in terms of electron injection, electron generation and/or electron conductivity. Preferably, the redox n-dopant is non-emissive.

In the context of the present invention "embedded into an electron transport matrix" means the redox n-dopant forms a mixture with the electron transport matrix.

Ar of formula (I)

Ar is selected from substituted or unsubstituted $C_6$ to $C_{24}$ aryl or substituted or unsubstituted $C_2$ to $C_{24}$ heteroaryl.

According to an embodiment, Ar is selected from substituted or unsubstituted $C_6$ to $C_{18}$ aryl or substituted or unsubstituted $C_2$ to $C_{18}$ heteroaryl.

According to an embodiment, Ar is selected from substituted or unsubstituted $C_6$ to $C_{12}$ aryl or substituted or unsubstituted $C_2$ to $C_{12}$ heteroaryl.

According to an embodiment, Ar is selected from substituted or unsubstituted $C_6$ to $C_{10}$ aryl or substituted or unsubstituted $C_2$ to $C_{10}$ heteroaryl.

According to an embodiment, Ar is selected from substituted or unsubstituted $C_6$ aryl or substituted or unsubstituted $C_3$ to $C_5$ heteroaryl.

According to an embodiment, Ar is selected from substituted or unsubstituted $C_6$ aryl or substituted or unsubstituted $C_3$ to $C_5$ heteroaryl, wherein the heteroaryl is a six-member ring.

According to an embodiment, Ar is selected from the group consisting of substituted or unsubstituted phenyl or substituted or unsubstituted pyridinyl, substituted or unsubstituted pyrazinyl, substituted or unsubstituted pyrimidyl, substituted or unsubstituted triazinyl, and substituted or unsubstituted quinolinyl.

Substituents on Ar of formula (I)

Ar may be unsubstituted or substituted with one or more substituents.

According to an embodiment, the one or more substituents on Ar, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $PX^1(R^8)_2$, halogen, F or CN, wherein the substituents may be linked via a single bond or a heteroatom to form a ring, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O.

According to an embodiment, the one or more substituents on Ar, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $PX^1(R^8)_2$, halogen, F or CN, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O.

According to an embodiment, the one or more substituents on Ar, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, $PX^1(R^8)_2$, halogen, F or CN, wherein the substituents may be linked via a single bond or a heteroatom to form a ring, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O.

According to an embodiment, the one or more substituents on Ar, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{26}$ cyclic alkyl, partially or perfluorinated $C_1$ to $C_{16}$ alkyl, partially or perdeuterated $C_1$ to $C_{16}$ alkyl, $PX^1(R^8)_2$, halogen, F or CN, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O.

According to an embodiment, the one or more substituents on Ar, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, halogen, F or CN, wherein the substituents may be linked via a single bond or a heteroatom to form a ring.

According to an embodiment, the one or more substituents on Ar, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, halogen, F or CN.

According to an embodiment, the one or more substituents on Ar, if present, are independently selected from D, substituted or unsubstituted $C_6$ to $C_{12}$ aryl, substituted or unsubstituted $C_3$ to $C_{14}$ heteroaryl, $C_1$ to $C_{10}$, alkyl, $C_3$ to $C_{10}$, branched alkyl, $C_3$ to $C_{10}$ cyclic alkyl, partially or perfluorinated $C_1$ to $C_{10}$ alkyl, partially or perdeuterated $C_1$ to $C_{10}$ alkyl, halogen, F or CN, wherein the substituents may be linked via a single bond or a heteroatom to form a ring.

According to an embodiment, the one or more substituents on Ar, if present, are independently selected from D, substituted or unsubstituted $C_6$ to $C_{12}$ aryl, substituted or unsubstituted $C_3$ to $C_{14}$ heteroaryl, $C_1$ to $C_{10}$ alkyl, $C_3$ to $C_{10}$ branched alkyl, $C_3$ to $C_{10}$ cyclic alkyl, partially or perfluorinated $C_1$ to $C_{10}$ alkyl, partially or perdeuterated $C_1$ to $C_{10}$ alkyl, halogen, F or CN.

According to an embodiment, the one or more substituents on Ar, if present, are independently selected from D, substituted or unsubstituted $C_6$ to $C_{10}$ aryl, substituted or unsubstituted $C_3$ to $C_{12}$ heteroaryl, $C_1$ to $C_6$ alkyl, $C_3$ to $C_6$ branched alkyl, $C_3$ to $C_6$ cyclic alkyl, partially or perfluorinated $C_1$ to $C_6$ alkyl, partially or perdeuterated $C_1$ to $C_6$ alkyl, halogen, F or CN, wherein the substituents may be linked via a single bond or a heteroatom to form a ring.

According to an embodiment, the one or more substituents on Ar, if present, are independently selected from D, substituted or unsubstituted $C_6$ to $C_{10}$ aryl, substituted or unsubstituted $C_3$ to $C_{12}$ heteroaryl, $C_1$ to $C_6$ alkyl, $C_3$ to $C_6$ branched alkyl, $C_3$ to $C_6$ cyclic alkyl, partially or perfluorinated $C_1$ to $C_6$ alkyl, partially or perdeuterated $C_1$ to $C_6$ alkyl, halogen, F or CN.

According to an embodiment, the one or more substituents on Ar, if present, are independently selected from D, substituted or unsubstituted $C_6$ to $C_{10}$ aryl, substituted or unsubstituted $C_3$ to $C_{10}$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN, wherein the substituents may be linked via a single bond or a heteroatom to form a ring.

According to an embodiment, the one or more substituents on Ar, if present, are independently selected from D, substituted or unsubstituted $C_6$ to $C_{10}$ aryl, substituted or unsubstituted $C_3$ to $C_{10}$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN.

According to an embodiment, the one or more substituents on Ar, if present, are independently selected from D, substituted or unsubstituted $C_6$ aryl, substituted or unsubstituted $C_3$ to $C_5$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN, wherein the substituents may be linked via a single bond or a heteroatom to form a ring.

According to an embodiment, the one or more substituents on Ar, if present, are independently selected from D, substituted or unsubstituted $C_6$ aryl, substituted or unsubstituted $C_3$ to $C_5$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN.

According to an embodiment, Ar is selected from the following D1 to $D_{19}$ shown in Table 1

Table 1:

| | | | | |
|---|---|---|---|---|
| 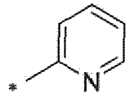 | 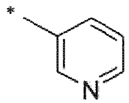 | 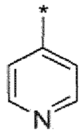 | 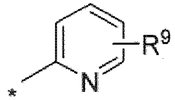 | 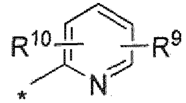 |
| D1 | D2 | D3 | D4 | D5 |
| 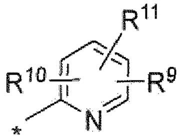 | 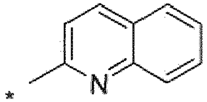 | 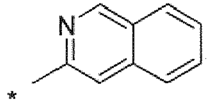 | 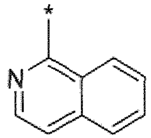 | 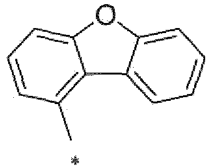 |
| D6 | D7 | D8 | D9 | D10 |
| 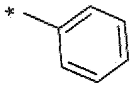 | 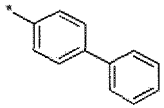 | 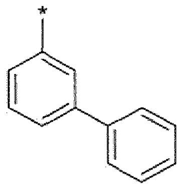 | 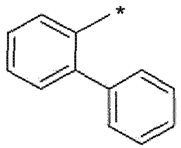 | 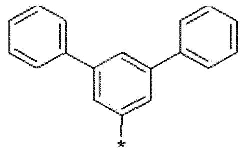 |
| D11 | D12 | D13 | D14 | D15 |
| 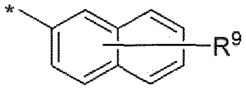 | 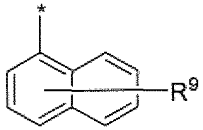 | 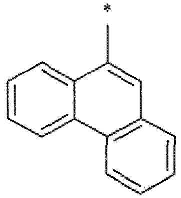 | 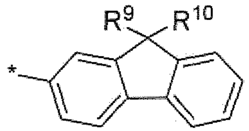 | |
| D16 | D17 | D18 | D19 | |

In $D_1$ to $D_{19}$, $R^9$, $R^{10}$ and $R^{11}$ are independently selected from H, $C_1$ to $C_{16}$ alkyl, $C_1$ to $C_{16}$ alkoxy, $C_6$ to $C_{18}$ aryl, $C_3$ to $C_{20}$ heteroaryl, perfluorinated $C_1$ to $C_{16}$ alkyl, perfluorinated $C_1$ to $C_{16}$ alkoxy, wherein $R^9$ and $R^{10}$ may be linked via a single bond or a heteroatom to form a ring, wherein the asterisk "*" denotes the binding position.

$R^1$ to $R^7$ of formula (I)

According to an embodiment, $R^1$ to $R^7$ are independently selected from H, D, substituted or unsubstituted $C_1$ to $C_{16}$ alkyl, substituted or unsubstituted $C_6$ to $C_{24}$ aryl or substituted or unsubstituted $C_2$ to $C_{24}$ heteroaryl, CN, halogen, F, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, or $PX^1(R^8)_2$ wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O,

According to an embodiment, $R^1$ to $R^7$ are independently selected from H, D, substituted or unsubstituted $C_1$ to $C_{16}$ alkyl, substituted or unsubstituted $C_6$ to $C_{24}$ aryl or substituted or unsubstituted $C_2$ to $C_{24}$ heteroaryl, CN, halogen, F.

According to an embodiment, $R^1$ to $R^7$ are independently selected from H, D, substituted or unsubstituted $C_1$ to $C_{16}$ alkyl, substituted or unsubstituted $C_6$ to $C_{24}$ aryl or substituted or unsubstituted $C_2$ to $C_{24}$ heteroaryl, halogen, F.

According to an embodiment, $R^1$ to $R^7$ are independently selected from H, D, substituted or unsubstituted $C_1$ to $C_{16}$ alkyl, substituted or unsubstituted $C_6$ to $C_{24}$ aryl or substituted or unsubstituted $C_2$ to $C_{24}$ heteroaryl, halogen.

According to an embodiment, $R^1$ to $R^7$ are independently selected from H, D, substituted or unsubstituted $C_1$ to $C_{12}$ alkyl, substituted or unsubstituted $C_6$ to $C_{18}$ aryl or substituted or unsubstituted $C_2$ to $C_{28}$ heteroaryl, CN, halogen, Cl, F.

According to an embodiment, $R^1$ to $R^7$ are independently selected from H, D, substituted or unsubstituted $C_1$ to $C_{12}$ alkyl, substituted or unsubstituted $C_6$ to $C_{18}$ aryl or substituted or unsubstituted $C_2$ to $C_{18}$ heteroaryl, halogen.

According to an embodiment, $R^1$ to $R^7$ are independently selected from H, D, substituted or unsubstituted $C_1$ to $C_6$ alkyl, substituted or unsubstituted $C_6$ to $C_{12}$ aryl or substituted or unsubstituted $C_2$ to $C_{12}$ heteroaryl, CN, halogen, Cl, F.

According to an embodiment, $R^1$ to $R^7$ are independently selected from H, D, substituted or unsubstituted $C_1$ to $C_6$ alkyl, substituted or unsubstituted $C_6$ to $C_{12}$ aryl or substituted or unsubstituted $C_2$ to $C_{12}$ heteroaryl, halogen.

According to an embodiment, $R^1$ to $R^7$ are independently selected from H, D, substituted or unsubstituted $C_1$ to $C_4$ alkyl, substituted or unsubstituted $C_6$ to $C_{10}$ aryl or substituted or unsubstituted $C_2$ to $C_{10}$ heteroaryl, CN, halogen, Cl, F.

According to an embodiment, $R^1$ to $R^7$ are independently selected from H, D, substituted or unsubstituted $C_1$ to $C_4$ alkyl, substituted or unsubstituted $C_6$ to $C_{10}$ aryl or substituted or unsubstituted $C_2$ to $C_{10}$ heteroaryl, halogen.

According to an embodiment, $R^1$ to $R^7$ are independently selected from H, D, substituted or unsubstituted $C_1$ to $C_4$ alkyl, substituted or unsubstituted $C_6$ aryl or substituted or unsubstituted $C_3$ to $C_5$ heteroaryl, CN, halogen, Cl, F.

According to an embodiment, $R^1$ to $R^7$ are independently selected from H, D, substituted or unsubstituted $C_1$ to $C_4$ alkyl, substituted or unsubstituted $C_6$ aryl or substituted or unsubstituted $C_3$ to $C_5$ heteroaryl, halogen.

Substituents on $R^1$ to $R^7$ of formula (I)

$R^1$ to $R^7$ may be independently unsubstituted or substituted with one or more substituents.

According to an embodiment, the one or more substituents on $R^1$ to $R^7$, if present, are independently selected from D, $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, halogen, F, CN or $PX^1(R^8)_2$, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O,

According to an embodiment, the one or more substituents on $R^1$ to $R^7$, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, $PX^1(R^8)_2$, halogen, F or CN, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O.

According to an embodiment, the one or more substituents on $R^1$ to $R^7$, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, halogen, F or CN.

According to an embodiment, the one or more substituents on $R^1$ to $R^7$, if present, are independently selected from D, substituted or unsubstituted $C_6$ to $C_{12}$ aryl, substituted or unsubstituted $C_3$ to $C_{14}$ heteroaryl, $C_1$ to $C_{10}$ alkyl, $C_3$ to

$C_{10}$ branched alkyl, $C_3$ to $C_{10}$, cyclic alkyl, partially or perfluorinated $C_1$ to $C_{10}$ alkyl, partially or perdeuterated $C_1$ to $C_{10}$ alkyl, halogen, F or CN.

According to an embodiment, the one or more substituents on $R^1$ to $R^7$, if present, are independently selected from D, substituted or unsubstituted $C_6$ to $C_{10}$ aryl, substituted or unsubstituted $C_3$ to $C_{10}$ heteroaryl, $C_1$ to $C_6$ alkyl, $C_3$ to $C_6$ branched alkyl, $C_3$ to $C_6$ cyclic alkyl, partially or perfluorinated $C_1$ to $C_6$ alkyl, partially or perdeuterated $C_1$ to $C_6$ alkyl, halogen, F or CN.

According to an embodiment, the one or more substituents on $R^1$ to $R^7$, if present, are independently selected from D, substituted or unsubstituted $C_6$ to $C_{10}$ aryl, substituted or unsubstituted $C_3$ to $C_{10}$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN.

According to an embodiment, the one or more substituents on $R^1$ to $R^7$, if present, are independently selected from D, substituted or unsubstituted $C_6$ aryl, substituted or unsubstituted $C_3$ to $C_5$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN.

L of formula (I)

According to an embodiment, L is selected from substituted or unsubstituted $C_6$ to $C_{18}$ aryl, substituted or unsubstituted $C_2$ to $C_{18}$ heteroaryl.

According to an embodiment, L is selected from substituted or unsubstituted $C_6$ to $C_{12}$ aryl or substituted or unsubstituted $C_2$ to $C_{12}$ heteroaryl.

According to an embodiment, L is selected from substituted or unsubstituted $C_6$ to $C_{10}$ aryl or substituted or unsubstituted $C_2$ to $C_{10}$ heteroaryl.

According to an embodiment, L is selected from substituted or unsubstituted $C_6$ aryl or substituted or unsubstituted $C_3$ to $C_5$ heteroaryl.

According to an embodiment, L is selected from substituted or unsubstituted $C_6$ aryl or substituted or unsubstituted $C_3$ to $C_5$ heteroaryl, wherein heteroaryl is a six-member ring.

According to an embodiment, L is selected from substituted or unsubstituted phenyl or substituted or unsubstituted pyridinyl, pyrazinyl, pyrimidyl, triazinyl.

According to an embodiment, L is selected from substituted or unsubstituted phenyl.

According to an embodiment, L is selected from unsubstituted phenyl.

Substituents on L of formula (I)

L may be independently unsubstituted or substituted with one or more substituents.

According to an embodiment, the one or more substituents on L, if present, are independently selected from D, $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, halogen, F, CN or $PX^1(R^8)_2$, wherein the substituents may be linked via a single bond or a heteroatom to form a ring, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O.

According to an embodiment, the one or more substituents on L, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $PX^1(R^8)_2$, halogen, F or CN, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^2$ is selected from O, S or Se, preferably O.

According to an embodiment, the one or more substituents on L, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, $PX^1(R^8)_2$, halogen, F or CN, wherein the substituents may be linked via a single bond or a heteroatom to form a ring, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O.

According to an embodiment, the one or more substituents on L, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, $PX^1(R^8)_2$, halogen, F or CN, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O.

According to an embodiment, the one or more substituents on L, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, halogen, F or CN, wherein the substituents may be linked via a single bond or a heteroatom to form a ring.

According to an embodiment, the one or more substituents on L, if present, are independently selected from D, substituted or unsubstituted $C_6$ to $C_{18}$ aryl, substituted or unsubstituted $C_3$ to $C_{20}$ heteroaryl, C, to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, halogen, F or CN.

According to an embodiment, the one or more substituents on L, if present, are independently selected from D, substituted or unsubstituted $C_6$ to $C_{12}$ aryl, substituted or unsubstituted $C_3$ to $C_{14}$ heteroaryl, $C_1$ to $C_{10}$ alkyl, $C_3$ to $C_{10}$ branched alkyl, $C_3$ to $C_{10}$ cyclic alkyl, partially or perfluorinated $C_1$ to $C_{10}$ alkyl, partially or perdeuterated $C_1$ to $C_{10}$ alkyl, halogen, F or CN, wherein the substituents may be linked via a single bond or a heteroatom to form a ring.

According to an embodiment, the one or more substituents on L, if present, are independently selected from D, substituted or unsubstituted $C_6$ to $C_{12}$ aryl, substituted or unsubstituted $C_3$ to $C_{14}$ heteroaryl, $C_1$ to $C_{10}$ alkyl, $C_3$ to $C_{10}$ branched alkyl, $C_3$ to $C_{10}$ cyclic alkyl, partially or perfluorinated $C_1$ to $C_{10}$ alkyl, partially or perdeuterated $C_1$ to $C_{10}$ alkyl, halogen, F or CN.

According to an embodiment, the one or more substituents on L, if present, are independently selected from D, substituted or unsubstituted $C_6$ to $C_{30}$ aryl, substituted or unsubstituted $C_3$ to $C_{12}$ heteroaryl, $C_1$ to $C_6$ alkyl, $C_3$ to $C_6$ branched alkyl, $C_3$ to $C_6$ cyclic alkyl, partially or perfluorinated $C_1$ to $C_6$ alkyl, partially or perdeuterated $C_1$ to $C_6$ alkyl, halogen, F or CN, wherein the substituents may be linked via a single bond or a heteroatom to form a ring.

According to an embodiment, the one or more substituents on L, if present, are independently selected from D, substituted or unsubstituted $C_6$ to $C_{10}$ aryl, substituted or unsubstituted $C_3$ to $C_{12}$ heteroaryl, $C_1$ to $C_6$ alkyl, $C_3$ to $C_6$ branched alkyl, $C_3$ to $C_6$ cyclic alkyl, partially or perfluorinated $C_1$ to $C_6$ alkyl, partially or perdeuterated $C_1$ to $C_6$ alkyl, halogen, F or CN.

According to an embodiment, the one or more substituents on L, if present, are independently selected from D, substituted or unsubstituted $C_6$ to $C_{10}$ aryl, substituted or unsubstituted $C_3$ to $C_{10}$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN, wherein the substituents may be linked via a single bond or a heteroatom to form a ring.

According to an embodiment, the one or more substituents on L, if present, are independently selected from D, substituted or unsubstituted $C_6$ to $C_{10}$ aryl, substituted or unsubstituted $C_3$ to $C_{10}$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN.

According to an embodiment, the one or more substituents on L, if present, are independently selected from D, substituted or unsubstituted $C_6$ aryl, substituted or unsubstituted $C_3$ to $C_5$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN, wherein the substituents may be linked via a single bond or a heteroatom to form a ring.

According to an embodiment, the one or more substituents on L, if present, are independently selected from D, substituted or unsubstituted $C_6$ aryl, substituted or unsubstituted $C_3$ to $C_5$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN.

According to an embodiment, L is selected from the following moieties E1 to E33 shown in Table 2.

Table 2:

| | | | |
|---|---|---|---|
| 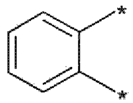 | 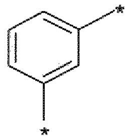 | 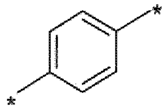 | 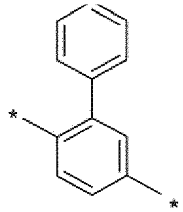 |
| E1, | E2, | E3, | E4, |
| 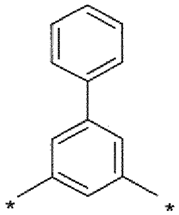 | 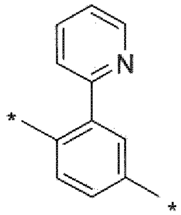 | 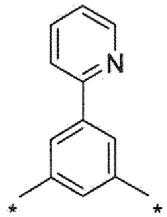 | 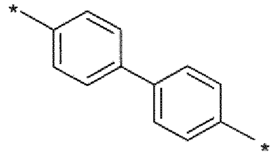 |
| E5, | E6, | E7, | E8, |
| 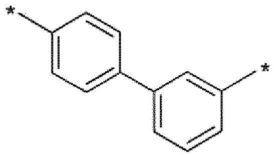 | 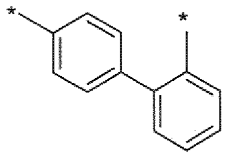 | 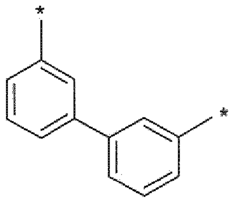 | 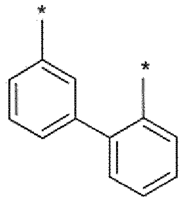 |
| E9, | E10, | E11, | E12, |
| 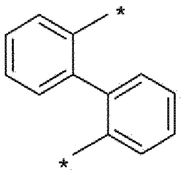 | 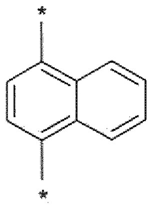 | 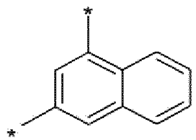 | 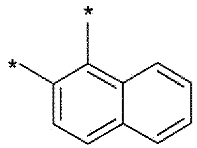 |
| E13, | E14, | E15, | E16, |

(continued)

| | | | |
|---|---|---|---|
| 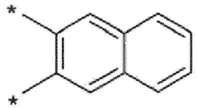 | 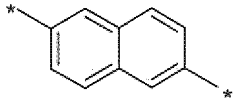 | 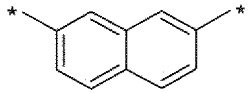 | 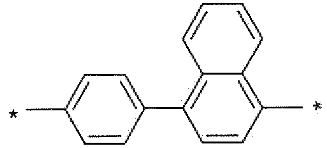 |
| E17, | E18, | E19, | E20, |
| 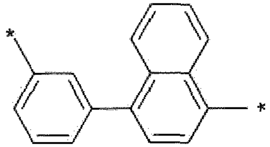 | 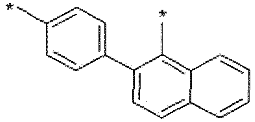 | 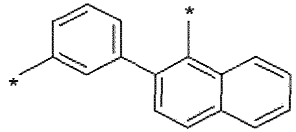 | 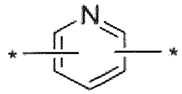 |
| E21, | E22, | E23, | E24, |
| 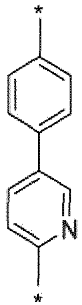 | 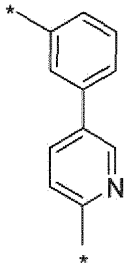 | 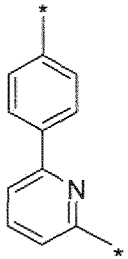 | 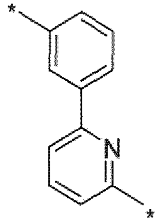 |
| E25, | E26, | E27, | E28, |
| 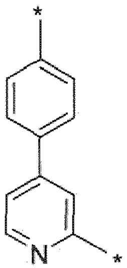 | 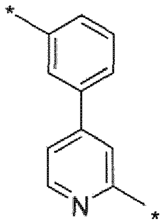 | 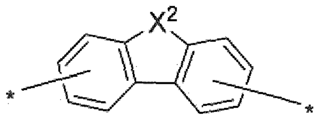 | 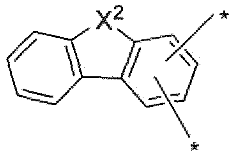 |
| E29, | E30, | E31, | E32, |

(continued)

| | | | |
|---|---|---|---|
| 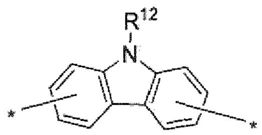 | | | |
| E33. | | | |

wherein in E1 to E33

$X^2$ is selected from O or S, preferably O;
$R^{12}$ and $R^{13}$ are independently selected from H, $C_1$ to $C_{16}$ alkyl, $C_1$ to $C_{16}$ alkoxy, $C_6$ to $C_{18}$ aryl, $C_3$ to $C_{20}$ heteroaryl, perfluorinated $C_1$ to $C_{16}$ alkyl, perfluorinated $C_1$ to $C_{16}$ alkoxy; and
the asterisk "*" denotes the binding position.

According to an embodiment, L is selected from the moieties E1 to E30.
According to an embodiment, L is selected from the moieties E1 to E24.
According to an embodiment, L is selected from the moieties E1 to E23.
According to an embodiment, L is selected from the moieties E1 to E5, and E8 to E23.
According to an embodiment, L is selected from the moieties E1 to E5, and E8 to E13.
According to an embodiment, L is selected from the moieties E1 to E5, and E11.
According to an embodiment, L is selected from the moieties E1 to E5.
According to an embodiment, L is selected from the moieties E1 to E3, and E11.
According to an embodiment, L is selected from the moieties E2 to E3, and E11.
According to an embodiment, L is selected from the moieties E2 to E3.

Properties of the compound of formula (I)

According to an embodiment, the compound of formula (I) has a LUMO energy level when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase ≤ -1.3 eV, preferably ≤ -1.35 eV, and more preferably of ≤ -1.4 eV..
According to an embodiment, the compound of formula (I) has a LUMO energy level when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase of ≥ -2.5 eV, preferably of ≥ -2.0 eV, more preferably ≥ -1.9 eV, even more preferably≥ -1.85 eV, and most preferably ≥ -1.8 eV.
According to an embodiment, the compound of formula (I) has a LUMO energy level when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase in the range of ≥ -2.5 eV and ≤ -1.3 eV, preferably of ≥ -2.0 eV to ≤ -1.35 eV, more preferably ≥ -1.9 eV to ≤ -1.4 eV, even more preferably ≥ -1.85 eV to ≤ -1.4 eV and most preferably ≥ -1.8 eV to ≤ -1.4 eV.
According to an embodiment, the compound of formula (I) has a molecular weight in the range of ≥400 g/mol to ≤2000 g/mol, preferably ≥415 g/mol to ≤1500 g/mol, more preferably ≥430 g/mol to ≤1000 g/mol, and most preferably ≥440 g/mol to ≤900 g/mol.

Specific embodiments of the compound of formula (I)

According to an embodiment, in formula (I) $R^1$ to $R^7$ are independently selected from H, or D; L is selected from substituted or unsubstituted $C_6$ to $C_{18}$ aryl, substituted or unsubstituted $C_2$ to $C_{18}$ heteroaryl.
According to an embodiment, in formula (I) $R^1$ to $R^7$ are independently selected from H, or D; L is selected from substituted or unsubstituted $C_6$ to $C_{18}$ aryl, substituted or unsubstituted $C_2$ to $C_{18}$ heteroaryl; Ar is selected from a substituted or unsubstituted pyridine group or a substituted or unsubstituted quinoline group or substituted or unsubstituted phenyl.
According to an embodiment, in formula (I) $R^1$ to $R^7$ are independently selected from H, or D; L is selected from substituted or unsubstituted $C_6$ to $C_{18}$ aryl, substituted or unsubstituted $C_2$ to $C_{18}$ heteroaryl; Ar is selected from a substituted or unsubstituted 2-pyridine group or a substituted or unsubstituted 2-quinoline group, or substituted or unsubstituted phenyl.
According to an embodiment, in formula (I) $R^1$ to $R^7$ are independently selected from H, or D; L is selected from substituted or unsubstituted $C_6$ to $C_{18}$ aryl, substituted or unsubstituted $C_2$ to $C_{18}$ heteroaryl; Ar is selected from an unsubstituted 2-pyridine group or an unsubstituted 2-quinoline group or an unsubstituted phenyl.
According to an embodiment, in formula (I) $R^1$ to $R^7$ are independently selected from H, or D; L is selected from substituted or unsubstituted $C_6$ to $C_{12}$ aryl or substituted or unsubstituted $C_2$ to $C_{12}$ heteroaryl.
According to an embodiment, in formula (I) $R^1$ to $R^7$ are independently selected from H, or D; L is selected from substituted or unsubstituted $C_6$ to $C_{12}$ aryl or substituted or unsubstituted $C_2$ to $C_{12}$ heteroaryl; Ar is selected from a substituted or unsubstituted pyridine group or a substituted or unsubstituted quinoline group or substituted or unsubstituted phenyl.

According to an embodiment, in formula (I) $R^1$ to $R^7$ are independently selected from H, or D; L is selected from substituted or unsubstituted $C_6$ to $C_{12}$ aryl or substituted or unsubstituted $C_2$ to $C_{12}$ heteroaryl; Ar is selected from a substituted or unsubstituted 2-pyridine group or a substituted or unsubstituted 2-quinoline group, or substituted or unsubstituted phenyl.

According to an embodiment, in formula (I) $R^1$ to $R^7$ are independently selected from H, or D; L is selected from substituted or unsubstituted $C_6$ to $C_{12}$ aryl or substituted or unsubstituted $C_2$ to $C_{12}$ heteroaryl; Ar is selected from an unsubstituted 2-pyridine group or an unsubstituted 2-quinoline group or an unsubstituted phenyl.

According to an embodiment, in formula (I) $R^1$ to $R^7$ are independently selected from H, or D; L is selected from substituted or unsubstituted $C_6$ to $C_{10}$ aryl or substituted or unsubstituted $C_2$ to $C_{10}$ heteroaryl.

According to an embodiment, in formula (I) $R^1$ to $R^7$ are independently selected from H, or D; L is selected from substituted or unsubstituted $C_6$ to $C_{10}$ aryl or substituted or unsubstituted $C_2$ to $C_{10}$ heteroaryl; Ar is selected from a substituted or unsubstituted pyridine group or a substituted or unsubstituted quinoline group or substituted or unsubstituted phenyl.

According to an embodiment, in formula (I) $R^1$ to $R^7$ are independently selected from H, or D; L is selected from substituted or unsubstituted $C_6$ to $C_{10}$ aryl or substituted or unsubstituted $C_2$ to $C_{10}$ heteroaryl; Ar is selected from a substituted or unsubstituted 2-pyridine group or a substituted or unsubstituted 2-quinoline group, or substituted or unsubstituted phenyl.

According to an embodiment, in formula (I) $R^1$ to $R^7$ are independently selected from H, or D; L is selected from substituted or unsubstituted $C_6$ to $C_{10}$ aryl or substituted or unsubstituted $C_2$ to $C_{10}$ heteroaryl; Ar is selected from an unsubstituted 2-pyridine group or an unsubstituted 2-quinoline group or an unsubstituted phenyl.

According to an embodiment, in formula (I) $R^1$ to $R^7$ are independently selected from H, or D; L is selected from substituted or unsubstituted $C_6$ aryl or substituted or unsubstituted $C_3$ to $C_5$ heteroaryl.

According to an embodiment, in formula (I) $R^1$ to $R^7$ are independently selected from H, or D; L is selected from substituted or unsubstituted $C_6$ aryl or substituted or unsubstituted $C_3$ to $C_5$ heteroaryl; Ar is selected from a substituted or unsubstituted pyridine group or a substituted or unsubstituted quinoline group or substituted or unsubstituted phenyl.

According to an embodiment, in formula (I) $R^1$ to $R^7$ are independently selected from H, or D; L is selected from substituted or unsubstituted $C_6$ aryl or substituted or unsubstituted $C_3$ to $C_5$ heteroaryl; Ar is selected from a substituted or unsubstituted 2-pyridine group or a substituted or unsubstituted 2-quinoline group, or substituted or unsubstituted phenyl.

According to an embodiment, in formula (I) $R^1$ to $R^7$ are independently selected from H, or D; L is selected from substituted or unsubstituted $C_6$ aryl or substituted or unsubstituted $C_3$ to $C_5$ heteroaryl; Ar is selected from an unsubstituted 2-pyridine group or an unsubstituted 2-quinoline group or an unsubstituted phenyl.

According to an embodiment, in formula (I) $R^1$ to $R^7$ are independently selected from H, or D; L is selected from substituted or unsubstituted $C_6$ aryl or substituted or unsubstituted $C_3$ to $C_5$ heteroaryl, wherein heteroaryl is a six-member ring.

According to an embodiment, in formula (I) $R^1$ to $R^7$ are independently selected from H, or D; L is selected from substituted or unsubstituted $C_6$ aryl or substituted or unsubstituted $C_3$ to $C_5$ heteroaryl, wherein heteroaryl is a six-member ring; Ar is selected from a substituted or unsubstituted pyridine group or a substituted or unsubstituted quinoline group or substituted or unsubstituted phenyl.

According to an embodiment, in formula (I) $R^1$ to $R^7$ are independently selected from H, or D; L is selected from substituted or unsubstituted $C_6$ aryl or substituted or unsubstituted $C_3$ to $C_5$ heteroaryl, wherein heteroaryl is a six-member ring; Ar is selected from a substituted or unsubstituted 2-pyridine group or a substituted or unsubstituted 2-quinoline group, or substituted or unsubstituted phenyl.

According to an embodiment, in formula (I) $R^1$ to $R^7$ are independently selected from H, or D; L is selected from substituted or unsubstituted $C_6$ aryl or substituted or unsubstituted $C_3$ to $C_5$ heteroaryl, wherein heteroaryl is a six-member ring; Ar is selected from an unsubstituted 2-pyridine group or an unsubstituted 2-quinoline group or an unsubstituted phenyl.

According to an embodiment, in formula (I) $R^1$ to $R^7$ are independently selected from H, or D; L is selected from substituted or unsubstituted phenyl or substituted or unsubstituted pyridinyl, pyrazinyl, pyrimidyl, triazinyl.

According to an embodiment, in formula (I) $R^1$ to $R^7$ are independently selected from H, or D; L is selected from substituted or unsubstituted phenyl or substituted or unsubstituted pyridinyl, pyrazinyl, pyrimidyl, triazinyl; Ar is selected from a substituted or unsubstituted pyridine group or a substituted or unsubstituted quinoline group or substituted or unsubstituted phenyl.

According to an embodiment, in formula (I) $R^1$ to $R^7$ are independently selected from H, or D; L is selected from substituted or unsubstituted phenyl or substituted or unsubstituted pyridinyl, pyrazinyl, pyrimidyl, triazinyl; Ar is selected from a substituted or unsubstituted 2-pyridine group or a substituted or unsubstituted 2-quinoline group, or substituted or unsubstituted phenyl.

According to an embodiment, in formula (I) $R^1$ to $R^7$ are independently selected from H, or D; L is selected from substituted or unsubstituted phenyl or substituted or unsubstituted pyridinyl, pyrazinyl, pyrimidyl, triazinyl; Ar is selected

from an unsubstituted 2-pyridine group or an unsubstituted 2-quinoline group or an unsubstituted phenyl.

According to an embodiment, in formula (I) $R^1$ to $R^7$ are independently selected from H, or D; L is selected from substituted or unsubstituted phenyl.

According to an embodiment, in formula (I) $R^1$ to $R^7$ are independently selected from H, or D; L is selected from substituted or unsubstituted phenyl; Ar is selected from a substituted or unsubstituted pyridine group or a substituted or unsubstituted quinoline group or substituted or unsubstituted phenyl.

According to an embodiment, in formula (I) $R^1$ to $R^7$ are independently selected from H, or D; L is selected from substituted or unsubstituted phenyl; Ar is selected from a substituted or unsubstituted 2-pyridine group or a substituted or unsubstituted 2-quinoline group, or substituted or unsubstituted phenyl.

According to an embodiment, in formula (I) $R^1$ to $R^7$ are independently selected from H, or D; L is selected from substituted or unsubstituted phenyl; Ar is selected from an unsubstituted 2-pyridine group or an unsubstituted 2-quinoline group or an unsubstituted phenyl.

According to an embodiment, in formula (I) $R^1$ to $R^7$ are independently selected from H, or D; L is selected from E1 to E5, and E8 to E23.

According to an embodiment, in formula (I) $R^1$ to $R^7$ are independently selected from H, or D; L is selected from E1 to E5, and E8 to E23; Ar is selected from a substituted or unsubstituted pyridine group or a substituted or unsubstituted quinoline group or substituted or unsubstituted phenyl.

According to an embodiment, in formula (I) $R^1$ to $R^7$ are independently selected from H, or D; L is selected from E1 to E5, and E8 to E23; Ar is selected from a substituted or unsubstituted 2-pyridine group or a substituted or unsubstituted 2-quinoline group, or substituted or unsubstituted phenyl.

According to an embodiment, in formula (I) $R^1$ to $R^7$ are independently selected from H, or D; L is selected from E1 to E5, and E8 to E23; Ar is selected from an unsubstituted 2-pyridine group or an unsubstituted 2-quinoline group or an unsubstituted phenyl.

According to an embodiment, in formula (I) $R^1$ to $R^7$ are independently selected from H, or D; L is selected from E1 to E5, and E8 to E23.

According to an embodiment, in formula (I) $R^1$ to $R^7$ are independently selected from H, or D; L is selected from E2, E3, E8, E9, E11, E14 and E15; Ar is selected from a substituted or unsubstituted pyridine group or a substituted or unsubstituted quinoline group or substituted or unsubstituted phenyl.

According to an embodiment, in formula (I) $R^1$ to $R^7$ are independently selected from H, or D; L is selected from E2, E3, E8, E9, E11, E14 and E15; Ar is selected from a substituted or unsubstituted 2-pyridine group or a substituted or unsubstituted 2-quinoline group, or substituted or unsubstituted phenyl.

According to an embodiment, in formula (I) $R^1$ to $R^7$ are independently selected from H, or D; L is selected from E2, E3, E8, E9, E11, E14 and E153; Ar is selected from an unsubstituted 2-pyridine group or an unsubstituted 2-quinoline group or an unsubstituted phenyl.

According to an embodiment, in formula (I) $R^1$ to $R^7$ are independently selected from H, or D; L is selected from E2 to E3, and E11.

According to an embodiment, in formula (I) $R^1$ to $R^7$ are independently selected from H, or D; L is selected from E2 to E3, and E11; Ar is selected from a substituted or unsubstituted pyridine group or a substituted or unsubstituted quinoline group or substituted or unsubstituted phenyl.

According to an embodiment, in formula (I) $R^1$ to $R^7$ are independently selected from H, or D; L is selected from E2 to E3, and E11; Ar is selected from a substituted or unsubstituted 2-pyridine group or a substituted or unsubstituted 2-quinoline group, or substituted or unsubstituted phenyl.

According to an embodiment, in formula (I) $R^1$ to $R^7$ are independently selected from H, or D; L is selected from E2 to E3, and E11; Ar is selected from an unsubstituted 2-pyridine group or an unsubstituted 2-quinoline group or an unsubstituted phenyl.

According to an embodiment, the compound of formula (I) is selected from the compound of formula (II)

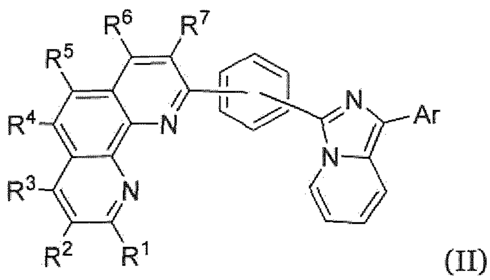

(II)

wherein

Ar is selected from substituted or unsubstituted $C_6$ to $C_{24}$ aryl or substituted or unsubstituted $C_2$ to $C_{24}$ heteroaryl,

$R^1$ to $R^7$ are independently selected from H, D, substituted or unsubstituted $C_1$ to $C_{16}$ alkyl, substituted or unsubstituted $C_6$ to $C_{24}$ aryl or substituted or unsubstituted $C_2$ to $C_{24}$ heteroaryl, CN, halogen, F, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, or $PX^1(R^8)_2$ wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O;

wherein formula (II) can be undeuterated, partially deuterated or fully deuterated.

According to an embodiment, the compound of formula (I) is selected from the compound of formula (III)

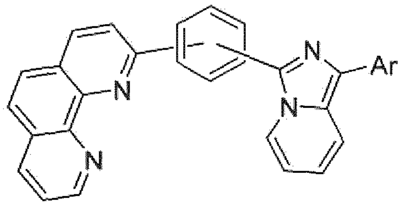

(III)

wherein

Ar is selected from substituted or unsubstituted $C_6$ to $C_{24}$ aryl or substituted or unsubstituted $C_2$ to $C_{24}$ heteroaryl;

wherein formula (III) can be undeuterated, partially deuterated or fully deuterated.

According to an embodiment, the compound of formula (I) is selected from the compound of formula (IVa) or (IVb)

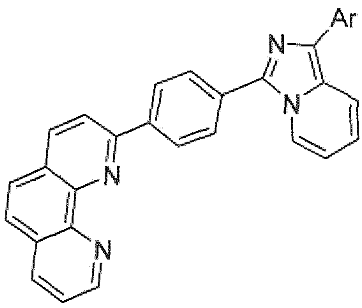

(IVa) or

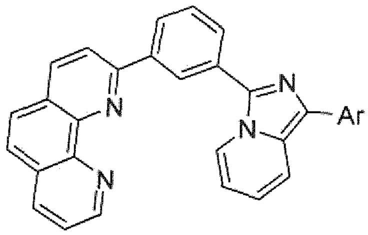

(IVb)

Ar is selected from substituted or unsubstituted $C_6$ to $C_{24}$ aryl or substituted or unsubstituted $C_2$ to $C_{24}$ heteroaryl;

wherein formula (IVa) or (IVb) can be undeuterated, partially deuterated or fully deuterated.

According to an embodiment, the compound of formula (I) is selected from the compound of formula (Va), (Vb), (Vc) or (Vd)

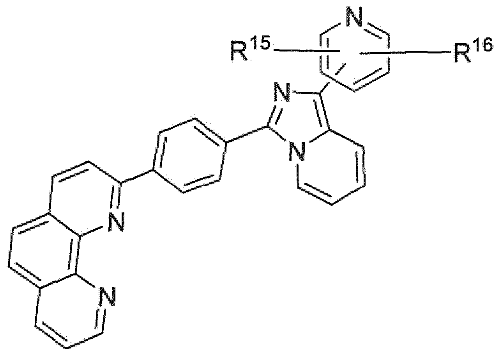 (Va), 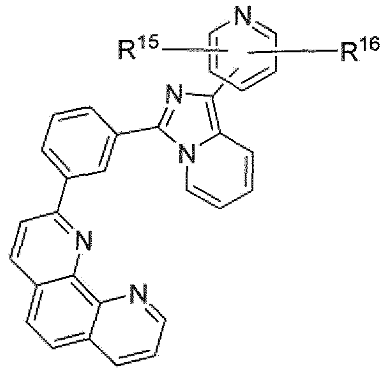 (Vb),

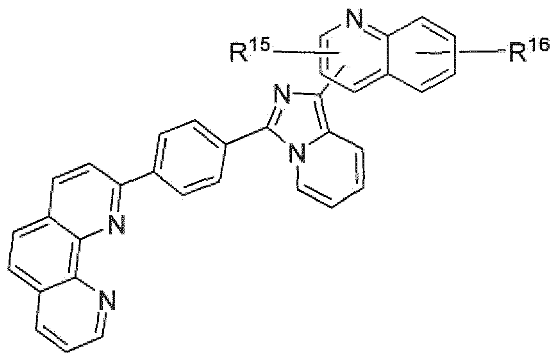 (Vc), or 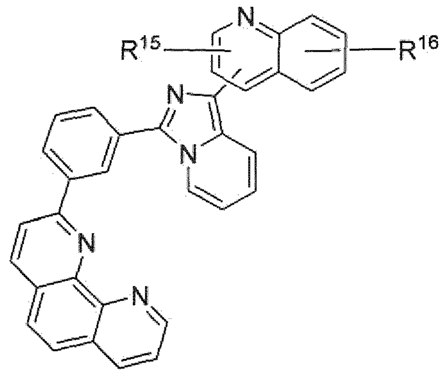 (Vd)

wherien

$R^{15}$ and $R^{16}$ are independently of each other selected from H, D, substituted or unsubstituted $C_1$ to $C_{16}$ alkyl, substituted or unsubstituted $C_6$ to $C_{24}$ aryl or substituted or unsubstituted $C_2$ to $C_{24}$ heteroaryl, CN, halogen, F, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, or $PX^1(R^8)_2$ wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O;

wherein formula (Va) to (Vd) can be undeuterated, partially deuterated or fully deuterated.

According to an embodiment, the one more substituents on $R^{15}$ and $R^{16}$, if present, are independently selected from D, $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, halogen, F, CN or $PX^1(R^8)_2$, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^2$ is selected from O, S or Se, preferably O,

According to an embodiment, wherein one or more substituents on $R^{15}$ to $R^{16}$, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, $PX^1(R^8)_2$, halogen, F or CN, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O.

According to an embodiment, wherein one or more substituents on $R^{15}$ to $R^{16}$, if present, are independently selected from D, substituted or unsubstituted $C_6$ to $C_{18}$ aryl, substituted or unsubstituted $C_3$ to $C_{20}$ heteroaryl, $C_1$ to $C_{16}$ alkyl, $C_3$ to $C_{16}$ branched alkyl, $C_3$ to $C_{16}$ cyclic alkyl, partially or perfluorinated $C_1$ to $C_{16}$ alkyl, partially or perdeuterated $C_1$ to $C_{16}$ alkyl, halogen, F or CN.

According to an embodiment, wherein one or more substituents on $R^{15}$ to $R^{16}$, if present, are independently selected from D, substituted or unsubstituted $C_6$ to $C_{12}$ aryl, substituted or unsubstituted $C_3$ to $C_{14}$ heteroaryl, $C_1$ to $C_{10}$ alkyl, $C_3$ to $C_{10}$ branched alkyl, $C_3$ to $C_{10}$ cyclic alkyl, partially or perfluorinated $C_1$ to $C_{10}$ alkyl, partially or perdeuterated $C_1$ to $C_{10}$ alkyl, halogen, F or CN.

According to an embodiment, wherein one or more substituents on $R^{15}$ to $R^{16}$, if present, are independently selected from D, substituted or unsubstituted $C_6$ to $C_{10}$ aryl, substituted or unsubstituted $C_3$ to $C_{12}$ heteroaryl, $C_1$ to $C_6$ alkyl, $C_3$ to $C_6$ branched alkyl, $C_3$ to $C_6$ cyclic alkyl, partially or perfluorinated $C_1$ to $C_6$ alkyl, partially or perdeuterated $C_1$ to $C_6$ alkyl, halogen, F or CN.

According to an embodiment, wherein one or more substituents on $R^{15}$ to $R^{16}$, if present, are independently selected from D, substituted or unsubstituted $C_6$ to $C_{10}$ aryl, substituted or unsubstituted $C_3$ to $C_{10}$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN.

According to an embodiment, wherein one or more substituents on $R^{15}$ to $R^{16}$, if present, are independently selected from D, substituted or unsubstituted $C_6$ aryl, substituted or unsubstituted $C_3$ to $C_5$ heteroaryl, $C_1$ to $C_4$ alkyl, $C_3$ to $C_4$ branched alkyl, $C_3$ to $C_6$ cyclic alkyl, partially or perfluorinated $C_1$ to $C_4$ alkyl, partially or perdeuterated $C_1$ to $C_4$ alkyl, halogen, F or CN.

According to an embodiment, the compound of formula (I) is selected from the compound of formula (VIa), (VIb), (VIc) or (VId):

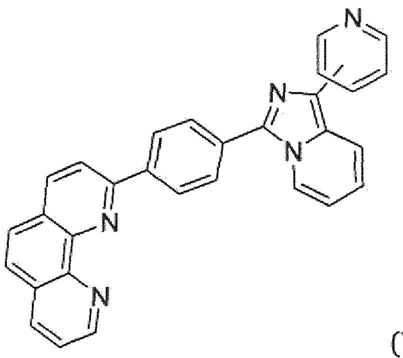
(VIa),
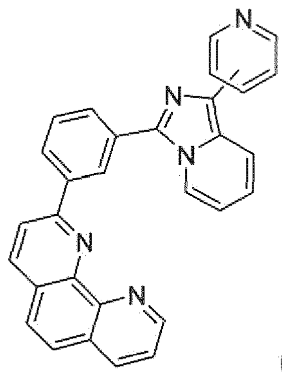
(VIb),

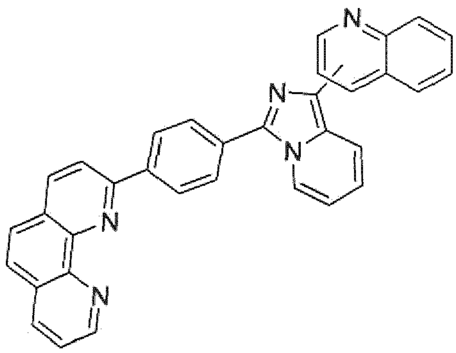
(VIc), or
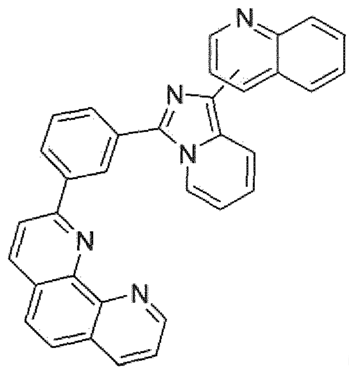
(VId),

$R^{15}$ and $R^{16}$ are independently of each other selected from

wherein formula (VIa) or (VId) can be undeuterated, partially deuterated or fully deuterated.

The compound of formula (I) may be selected from the following compounds I-1 to I-150 shwon in Table 3.

Table 3:

| | |
|---|---|
| 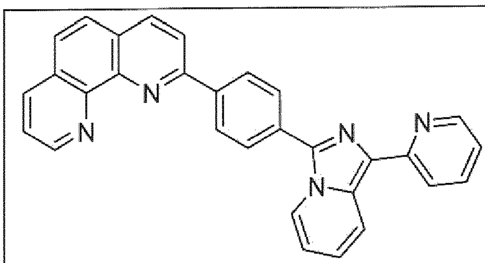 I-1 | 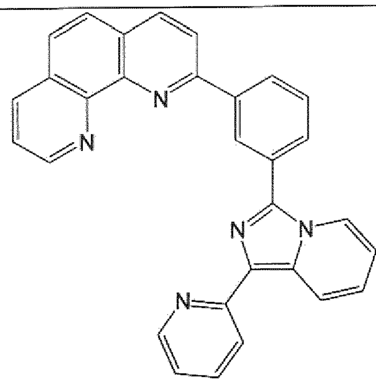 I-2 |
| 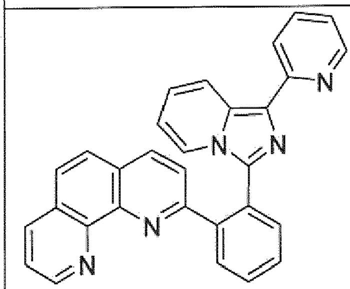 I-3 | 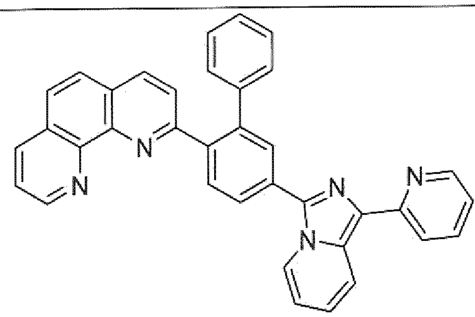 I-4 |
| 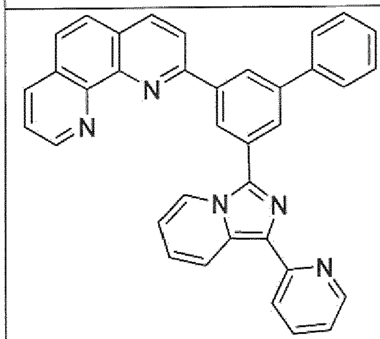 I-5 | 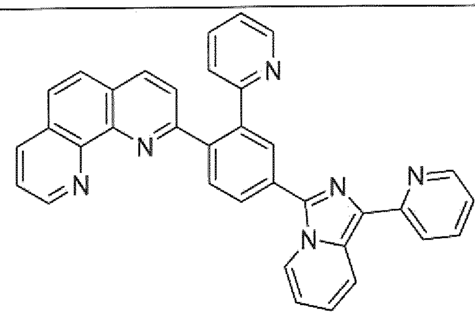 I-6 |
| 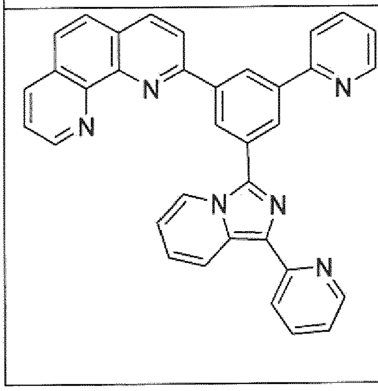 I-7 | 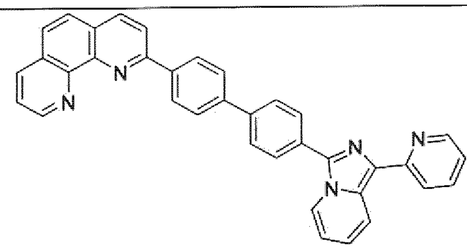 I-8 |

| | |
|---|---|
| 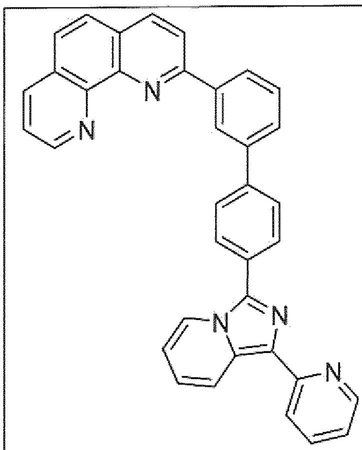 I-9 | 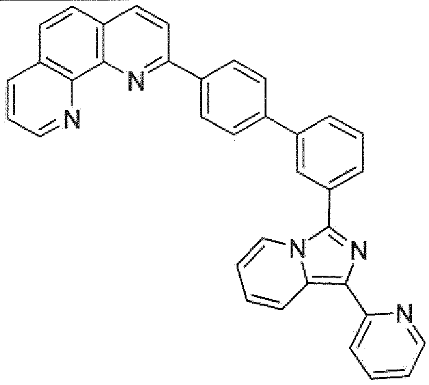 I-10 |
| 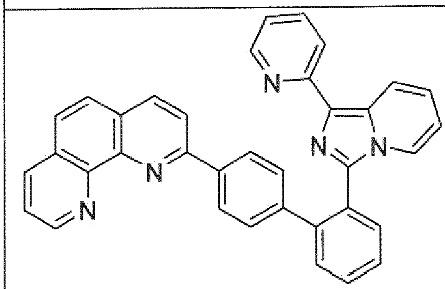 I-11 | 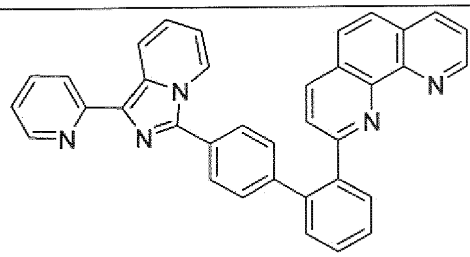 I-12 |
| 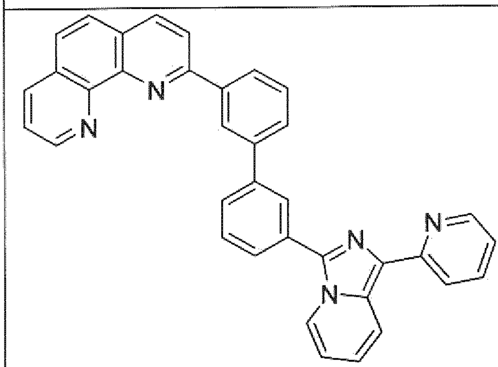 I-13 | 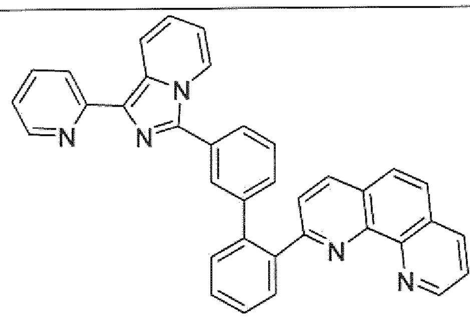 I-14 |
| 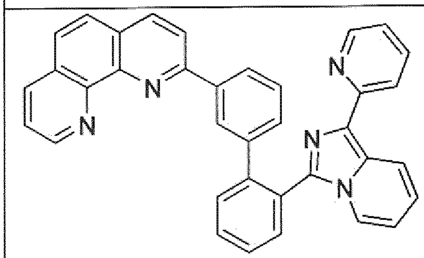 I-15 | 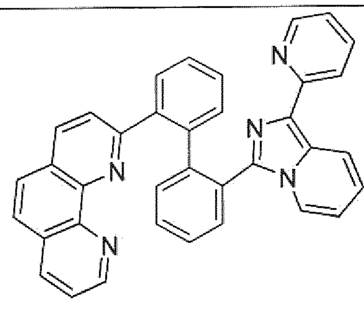 I-16 |

| | |
|---|---|
| 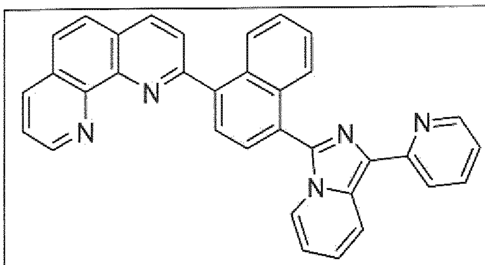 I-17 | 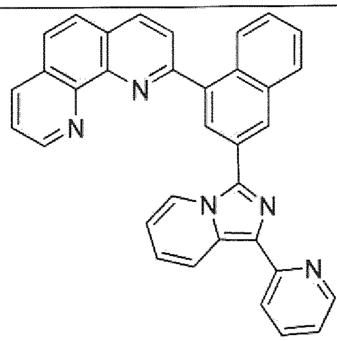 I-18 |
| 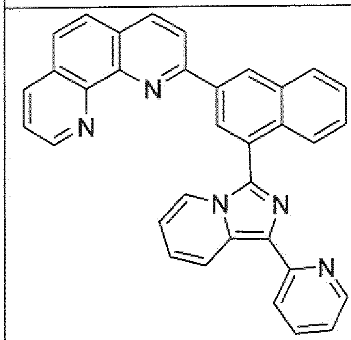 I-19 | 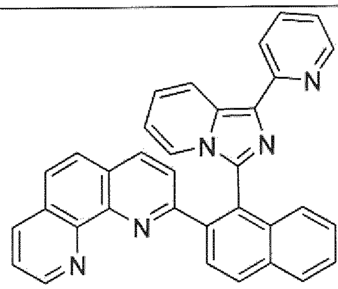 I-20 |
| 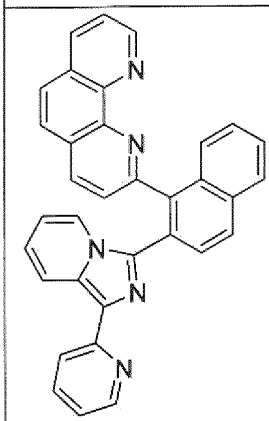 I-21 | 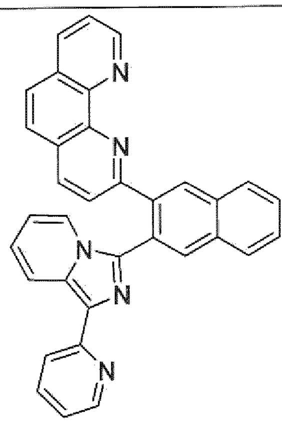 I-22 |
| 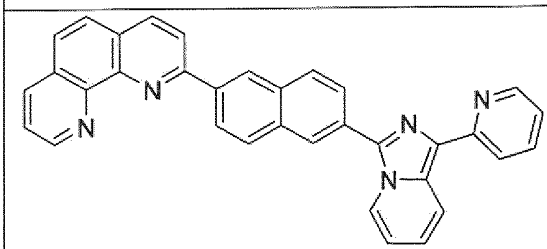 I-23 | 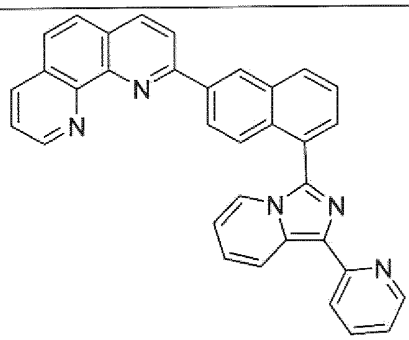 I-24 |

| | |
|---|---|
| 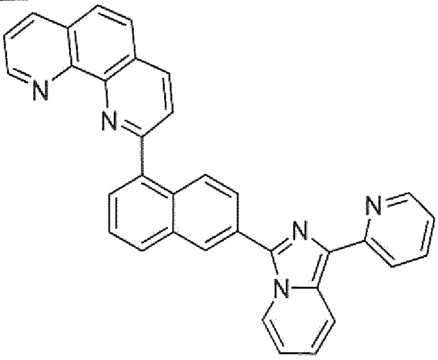 I-25 | 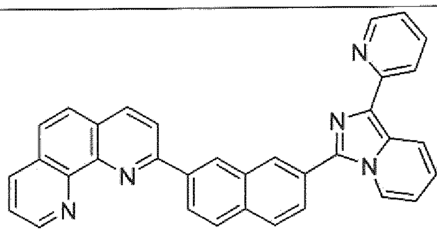 I-26 |
| 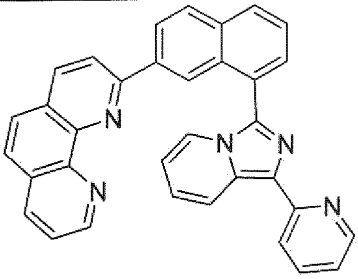 I-27 | 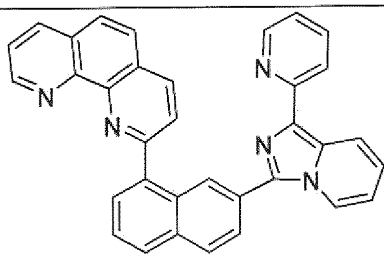 I-28 |
| 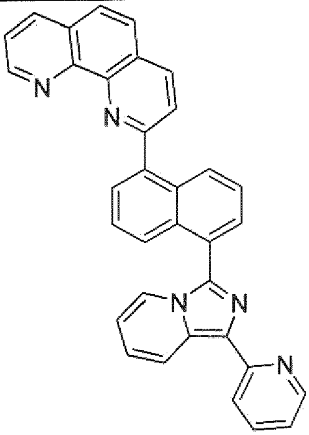 I-29 | 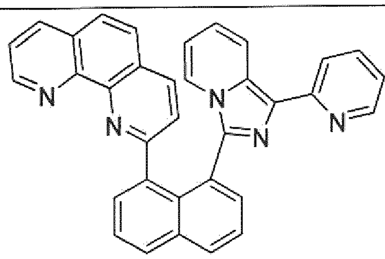 I-30 |
| 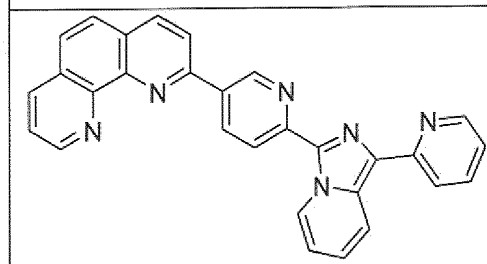 I-31 | 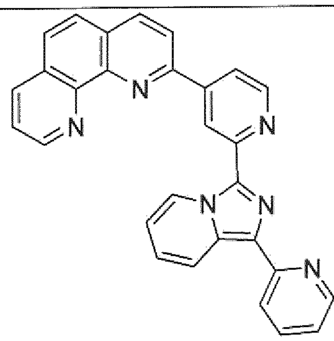 I-32 |

| | |
|---|---|
| 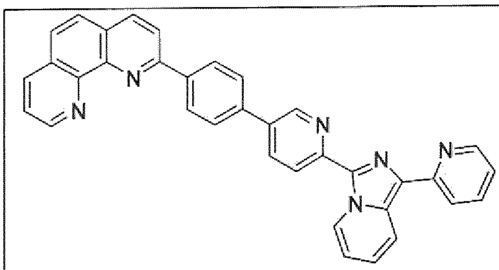 I-33 | 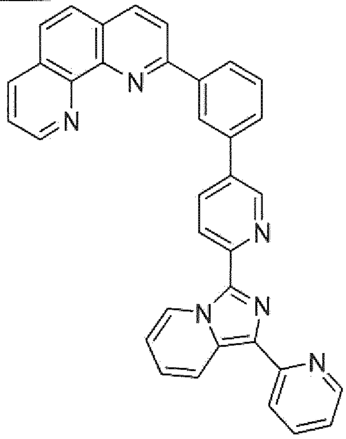 I-34 |
| 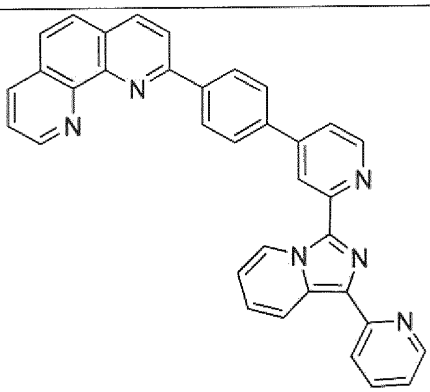 I-35 | 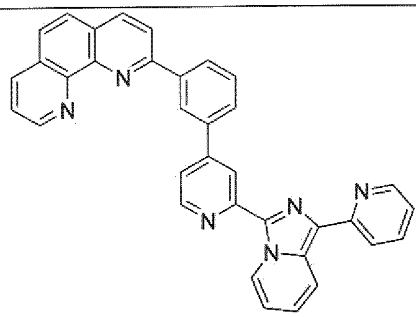 I-36 |
| 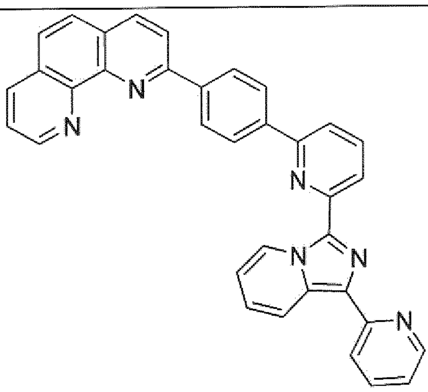 I-37 | 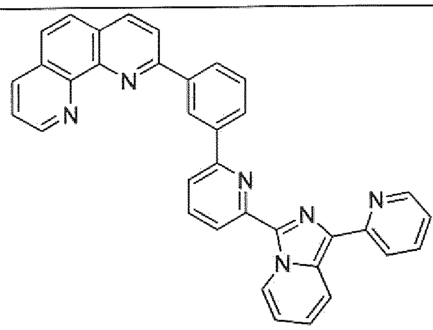 I-38 |
| 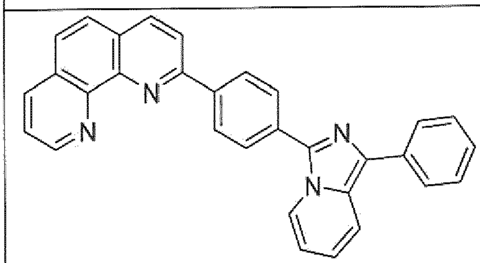 I-39 | 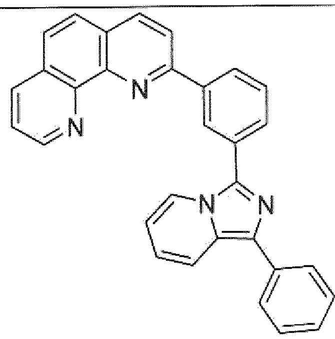 I-40 |

| | |
|---|---|
| 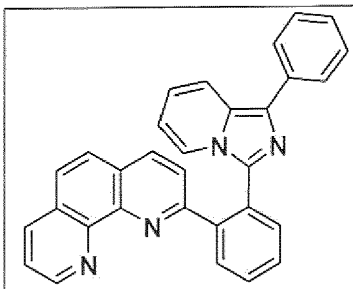 I-41 | 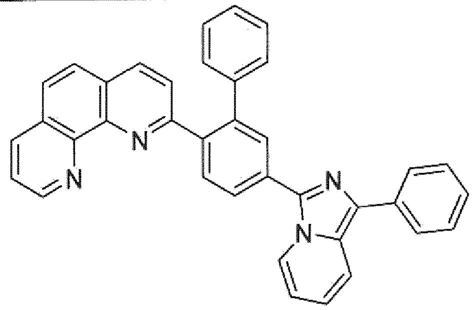 I-42 |
| 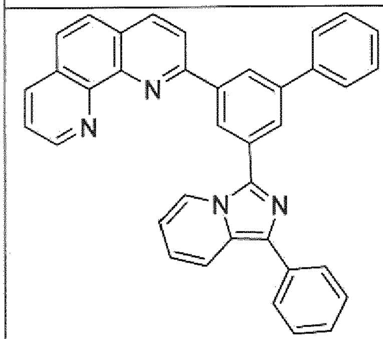 I-43 | 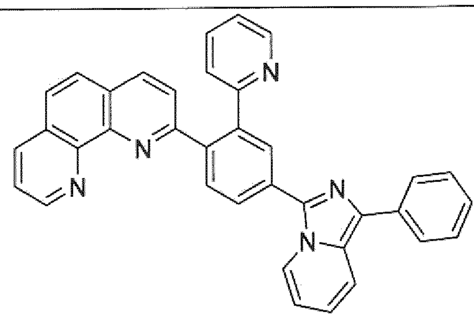 I-44 |
| 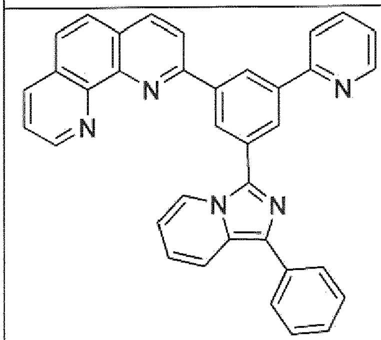 I-45 | 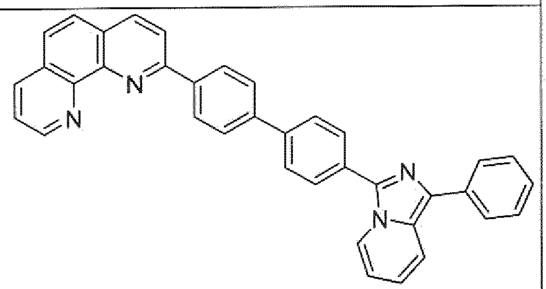 I-46 |
| 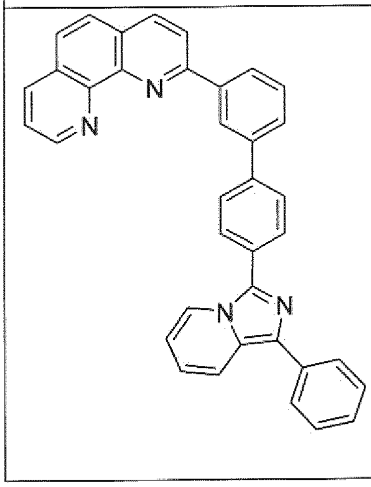 I-47 | 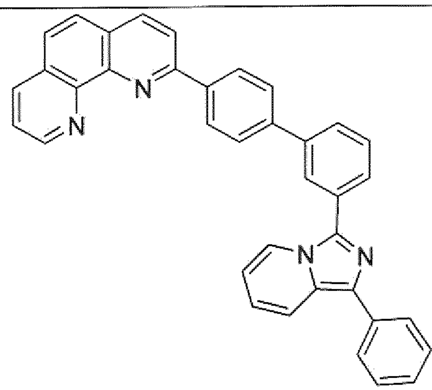 I-48 |

| | |
|---|---|
| 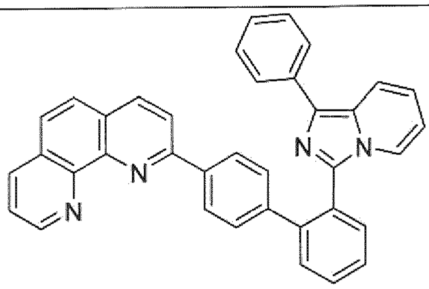 I-49 | 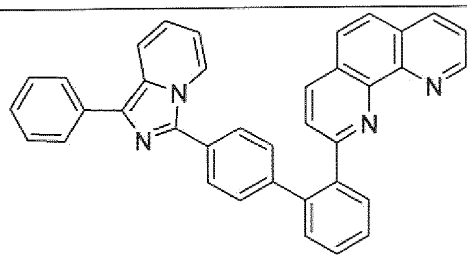 I-50 |
| 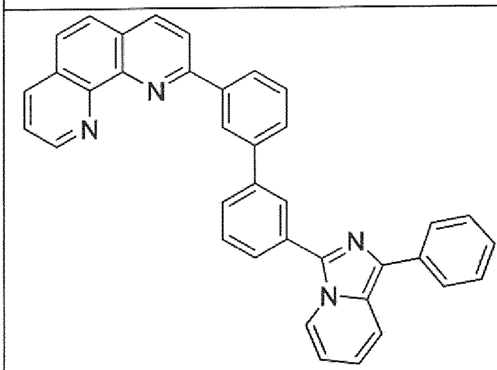 I-51 | 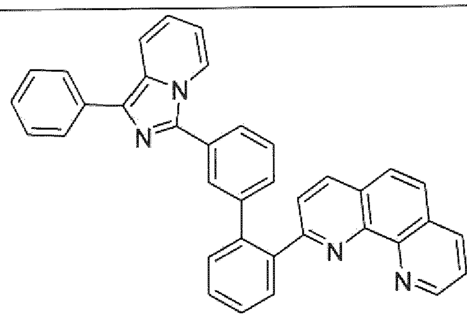 I-52 |
| 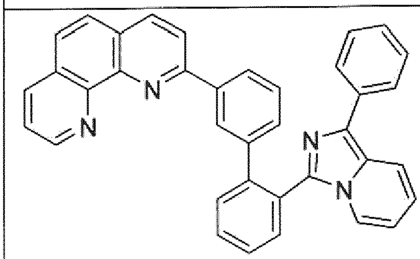 I-53 | 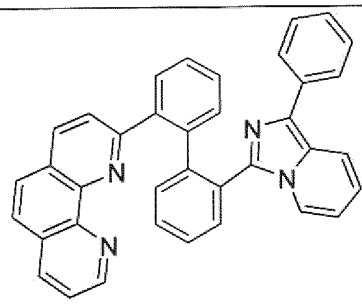 I-54 |
| 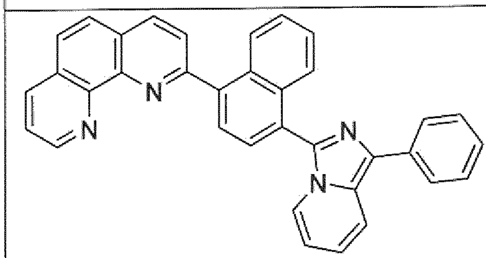 I-55 | 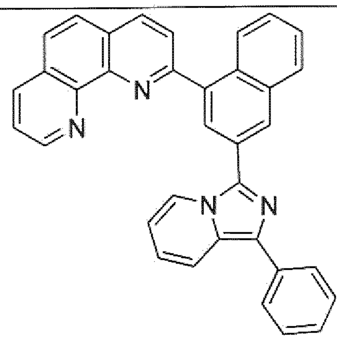 I-56 |

| | |
|---|---|
| 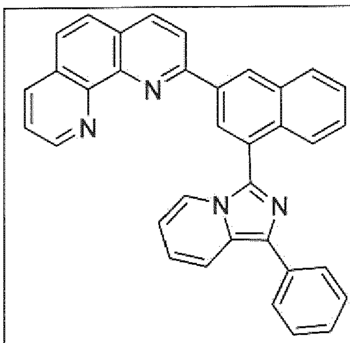 I-57 | 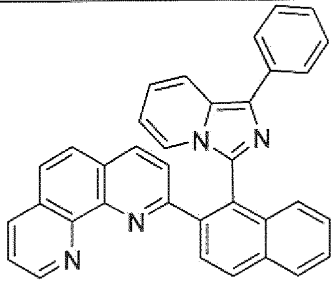 I-58 |
| 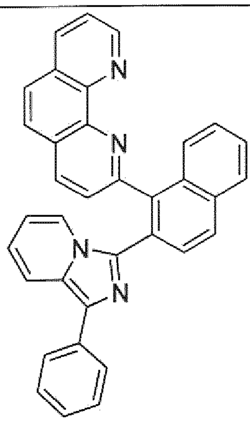 I-59 | 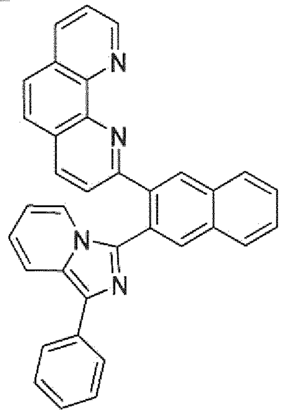 I-60 |
| 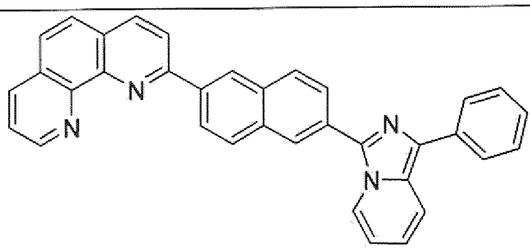 I-61 | 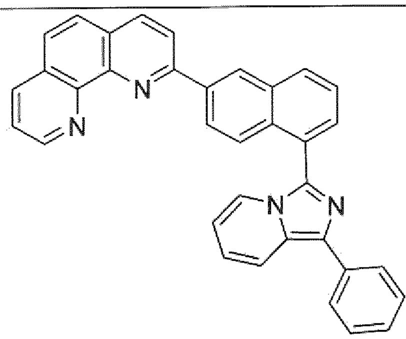 I-62 |
| 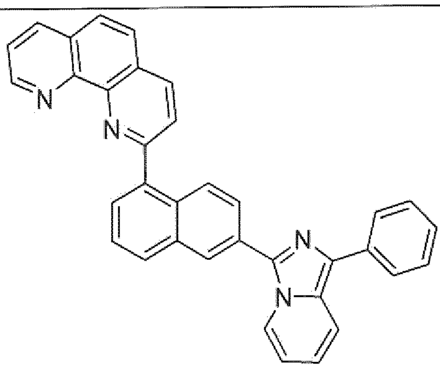 I-63 | 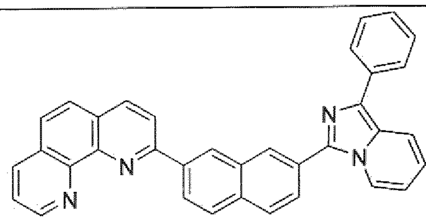 I-64 |

| | |
|---|---|
| 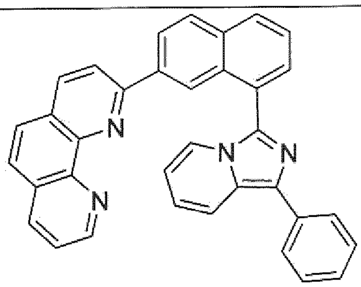 I-65 | 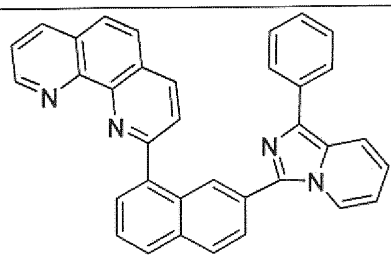 I-66 |
| 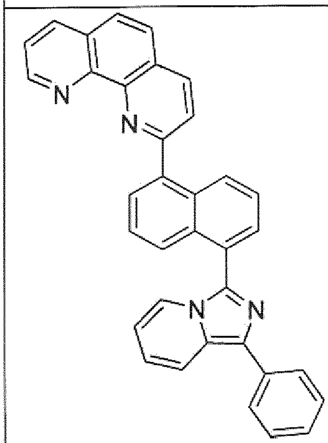 I-67 | 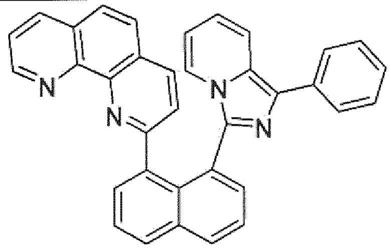 I-68 |
| 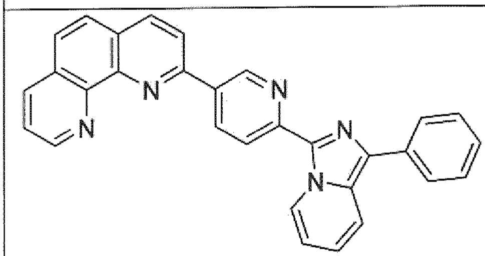 I-69 | 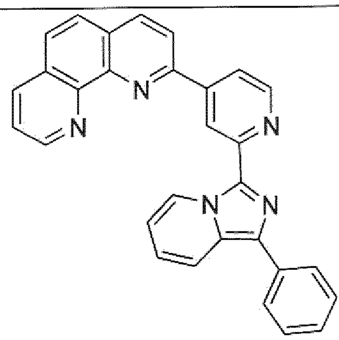 I-70 |
| 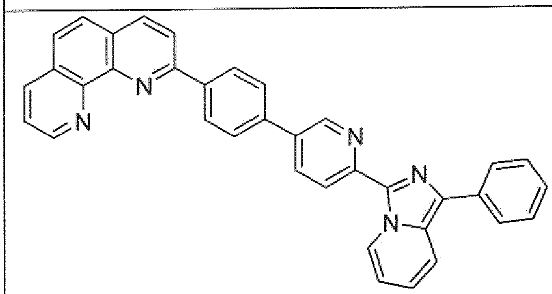 I-71 | 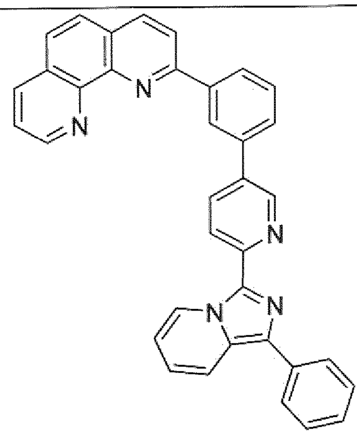 I-72 |

| | |
|---|---|
| 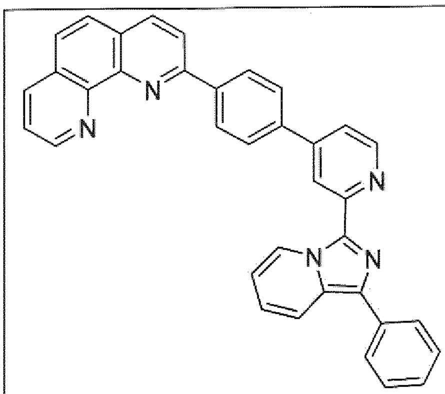 I-73 | 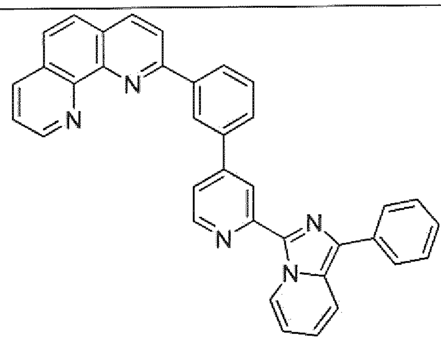 I-74 |
| 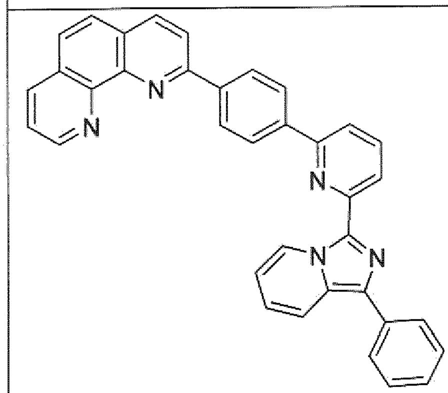 I-75 | 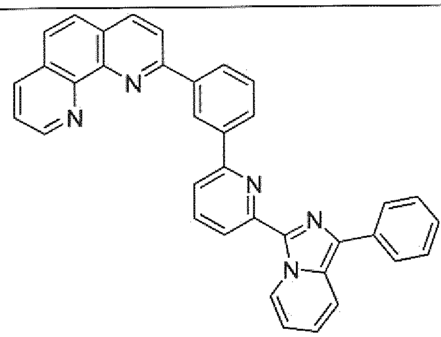 I-76 |
| 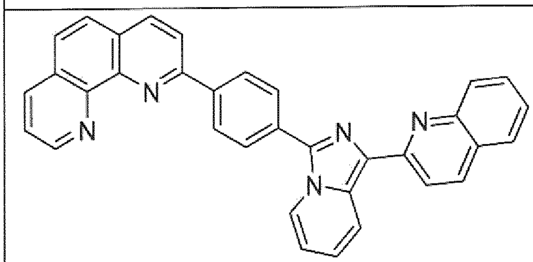 I-77 | 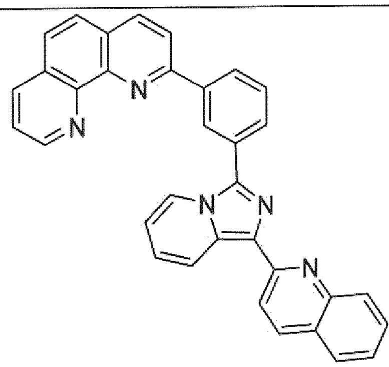 I-78 |
| 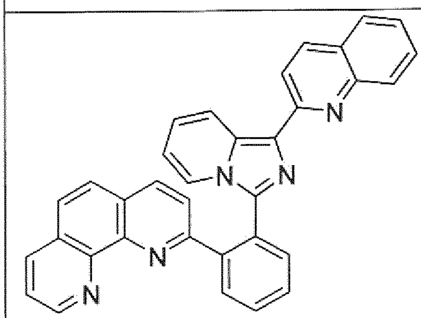 I-79 | 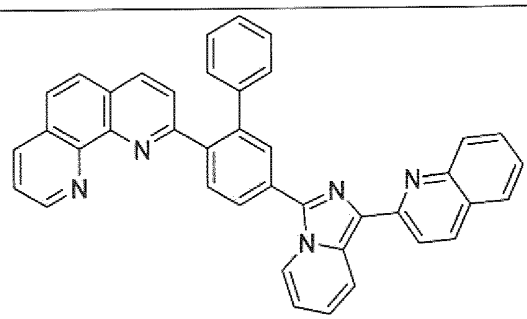 I-80 |

| | |
|---|---|
| 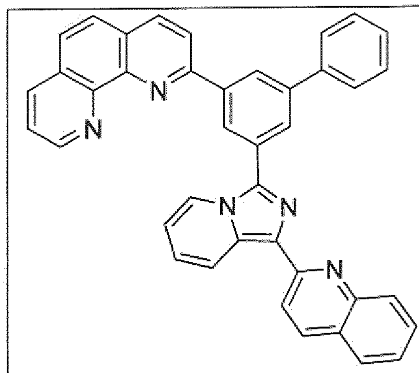 I-81 | 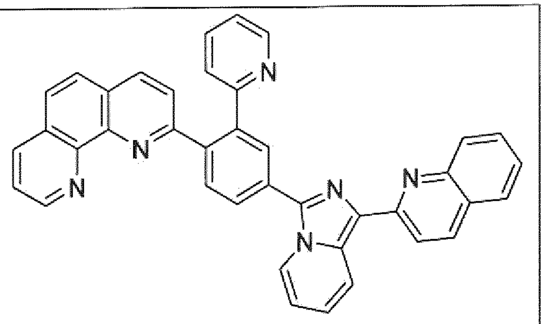 I-82 |
| 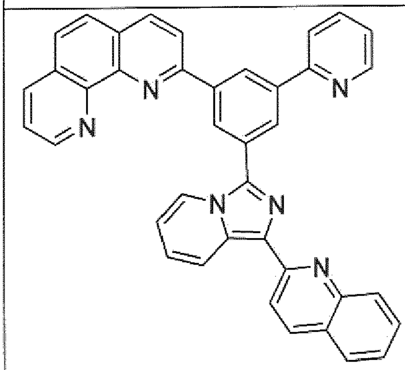 I-83 | 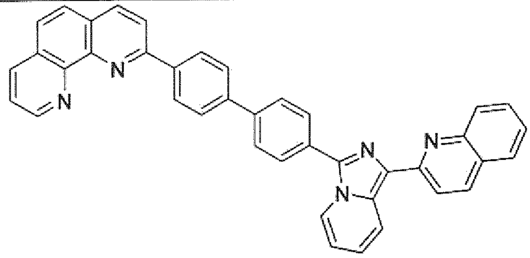 I-84 |
| 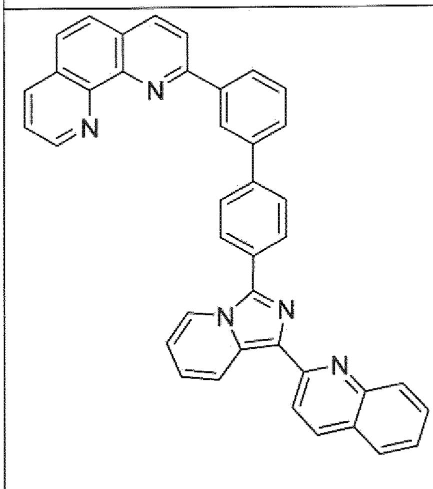 I-85 | 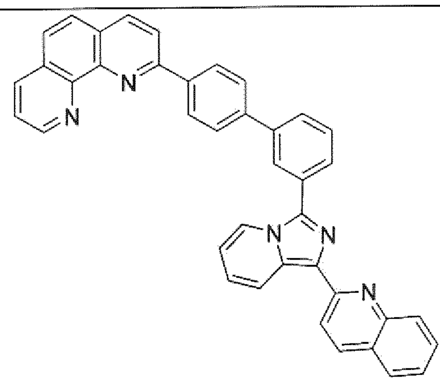 I-86 |
| 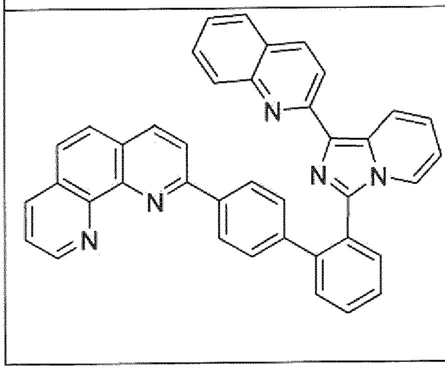 I-87 | 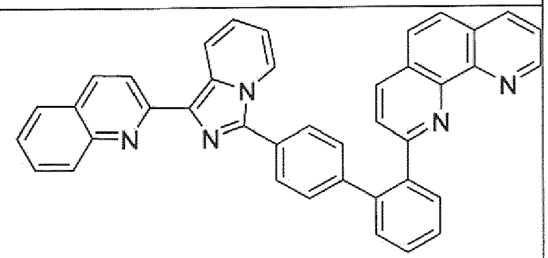 I-88 |

| | |
|---|---|
| 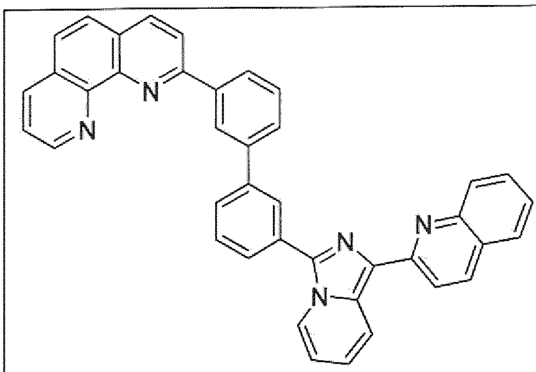 I-89 | 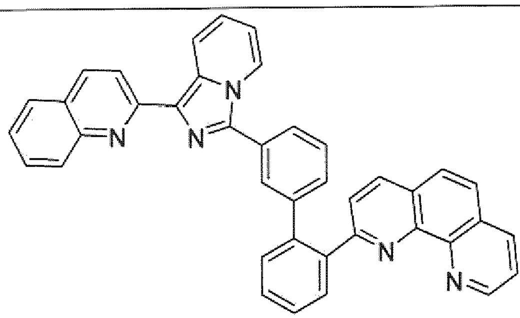 I-90 |
| 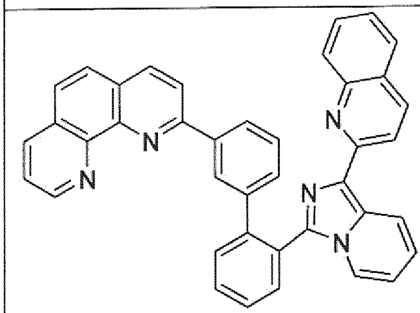 I-91 | 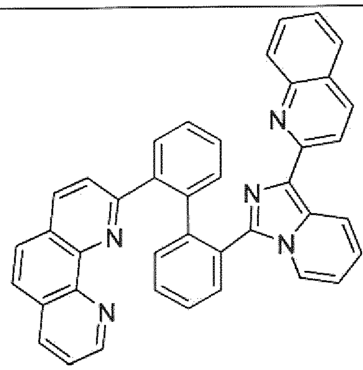 I-92 |
| 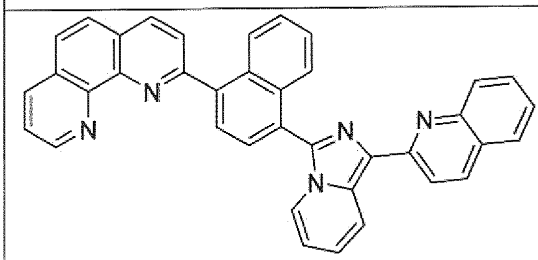 I-93 | 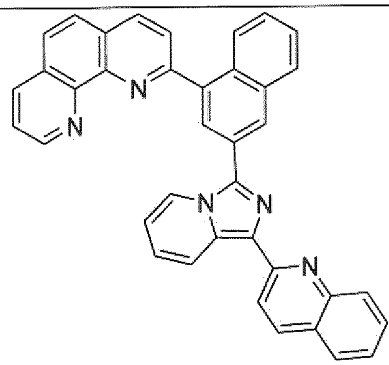 I-94 |
| 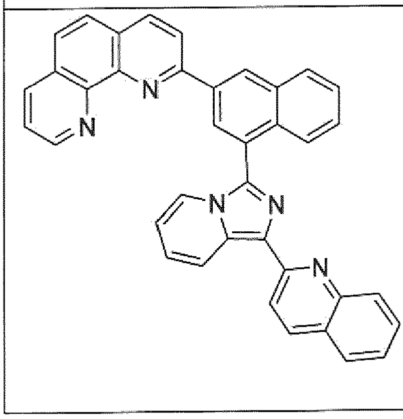 I-95 | 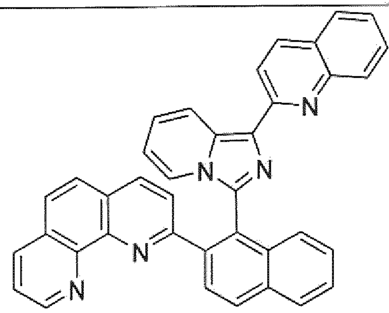 I-96 |

| | |
|---|---|
| 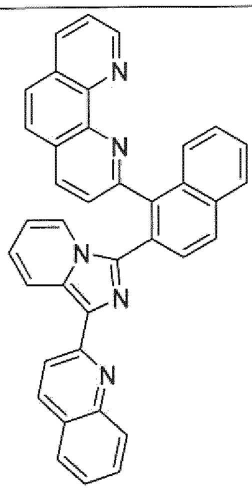 I-97 | 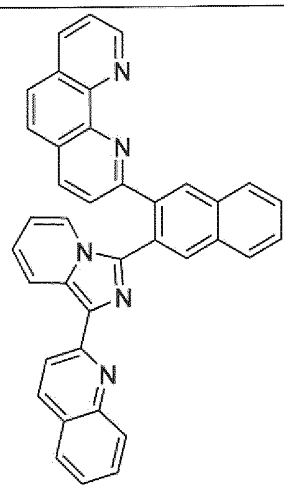 I-98 |
| 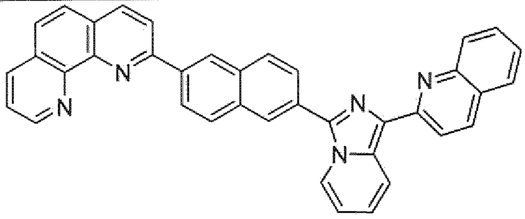 I-99 | 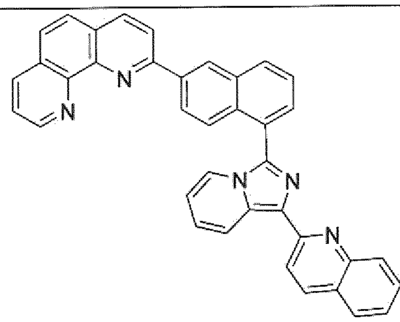 I-100 |
| 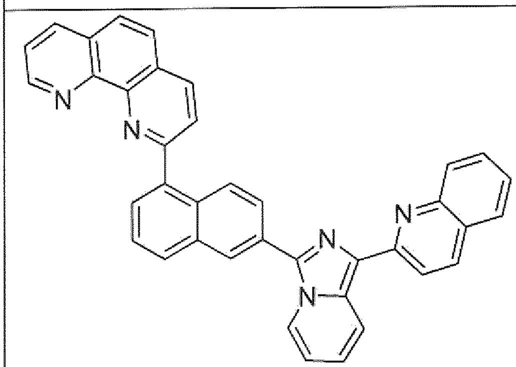 I-101 | 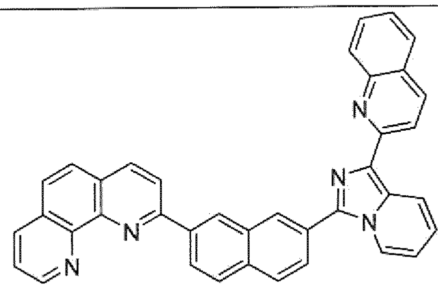 I-102 |
| 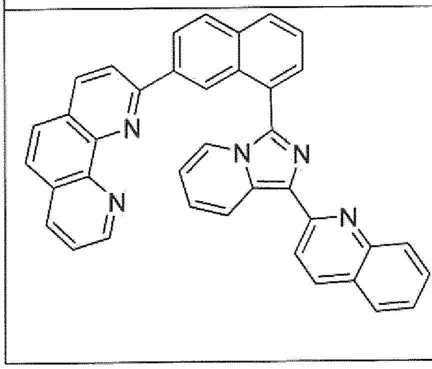 I-103 | 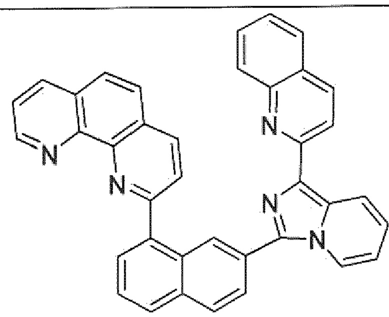 I-104 |

| | |
|---|---|
| 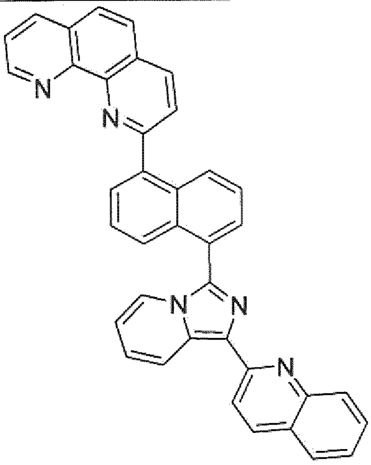 I-105 | 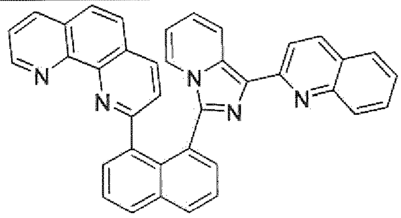 I-106 |
| 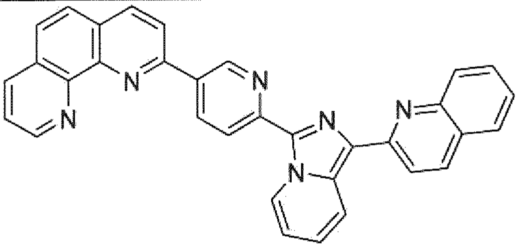 I-107 | 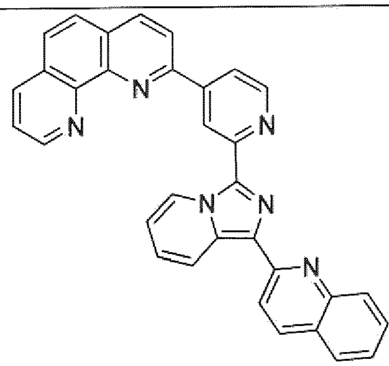 I-108 |
| 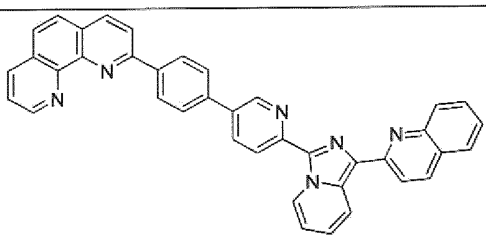 I-109 | 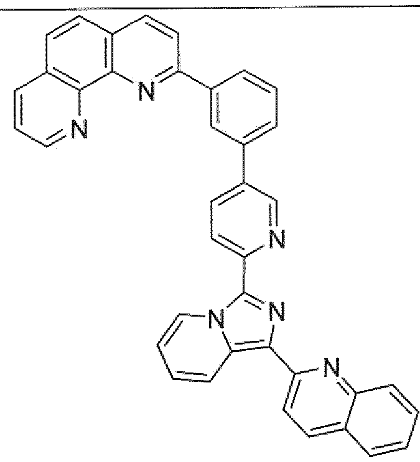 I-110 |

| | |
|---|---|
| 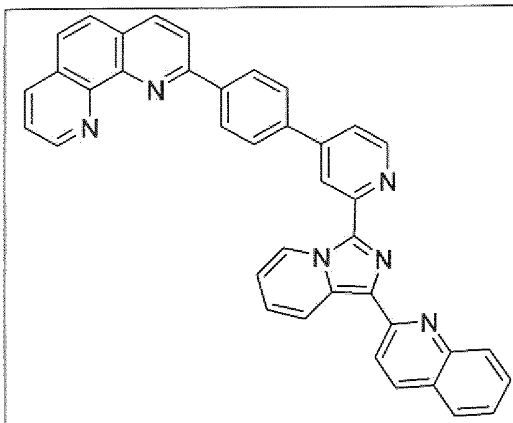 I-111 | 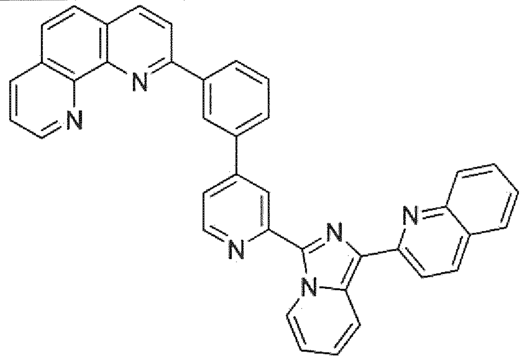 I-112 |
| 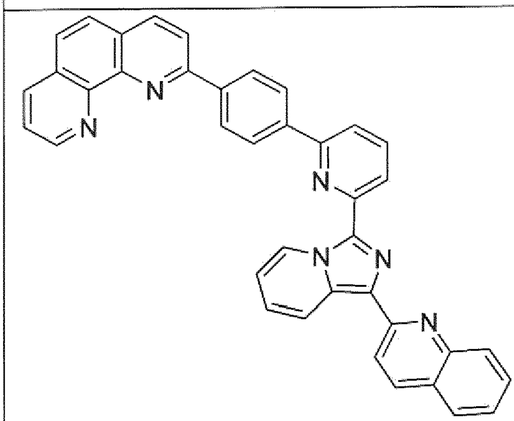 I-113 | 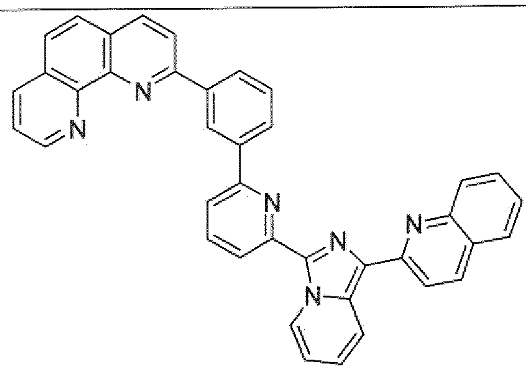 I-114 |
| 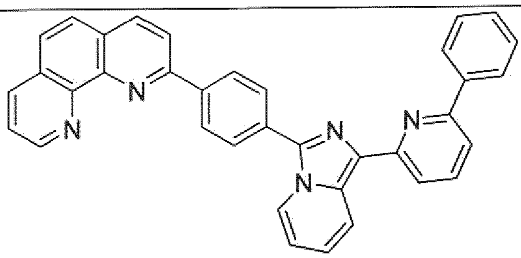 I-115 | 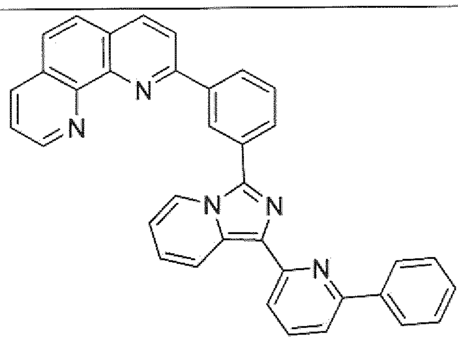 I-116 |
| 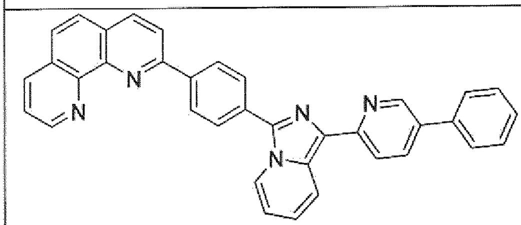 I-117 | 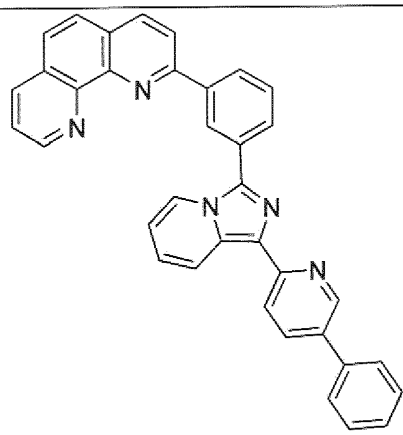 I-118 |

| | |
|---|---|
| 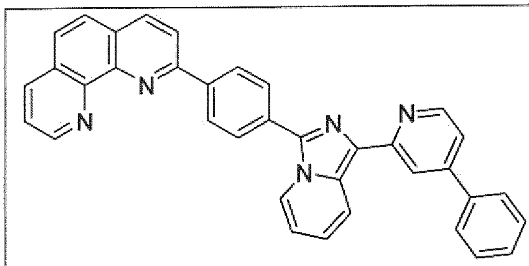 I-119 | 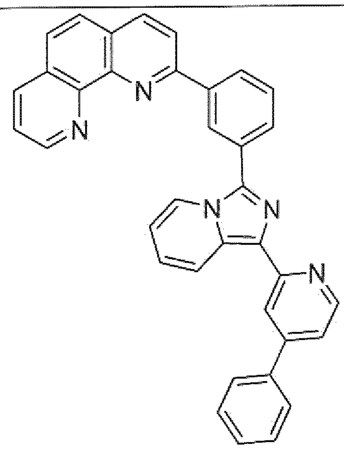 I-120 |
| 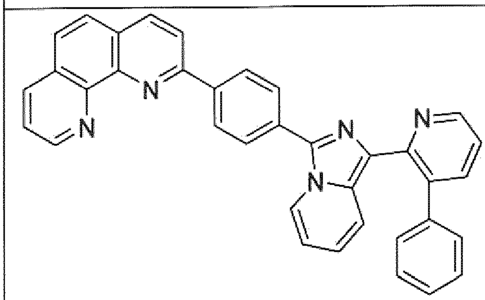 I-121 | 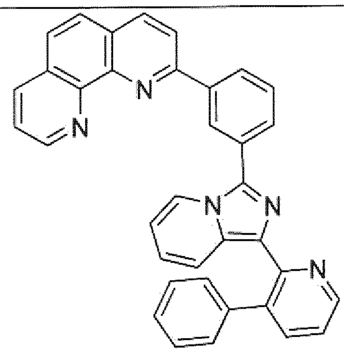 I-122 |
| 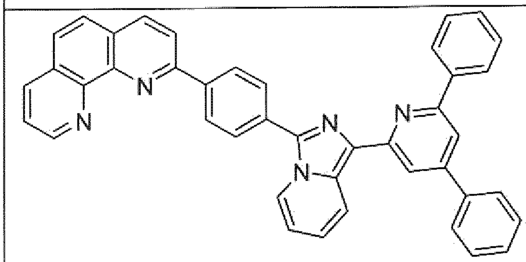 I-123 | 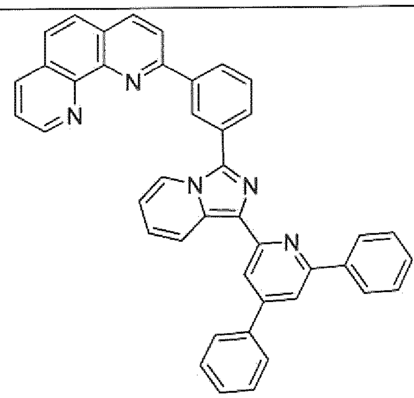 I-124 |
| 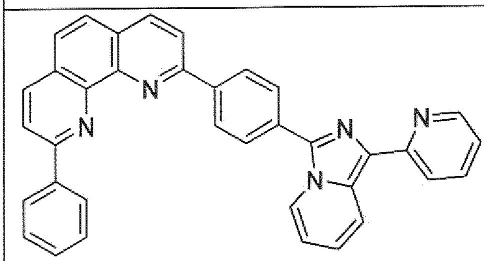 I-125 | 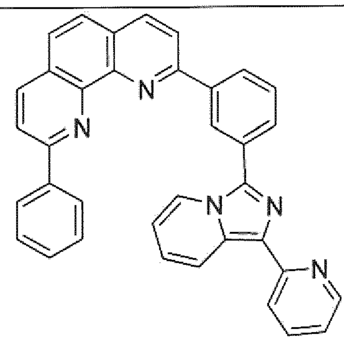 I-126 |

| | |
|---|---|
| 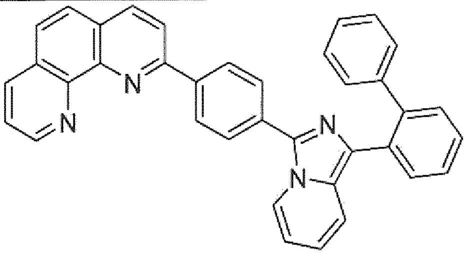 I-127 | 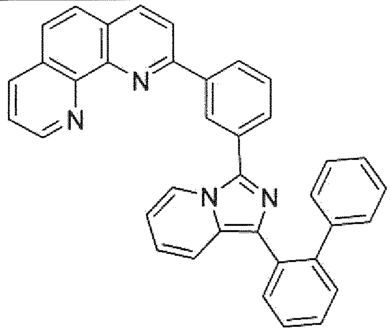 I-128 |
| 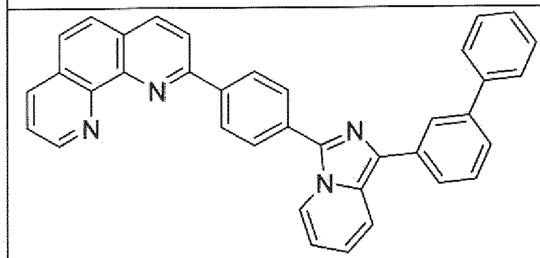 I-129 | 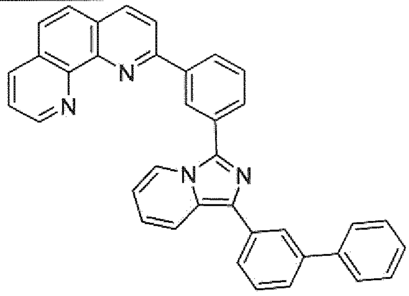 I-130 |
| 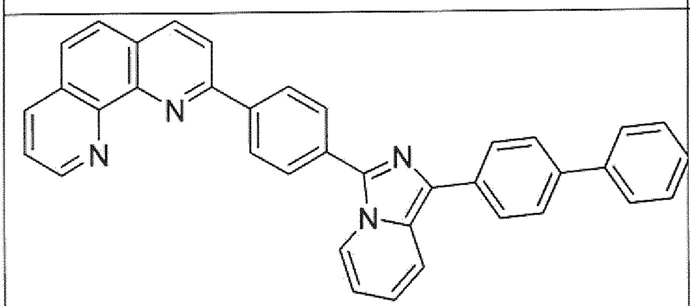 I-131 | 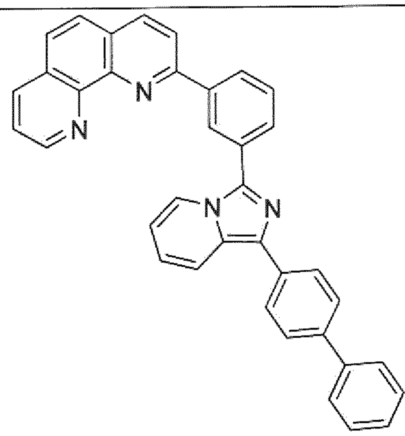 I-132 |
| 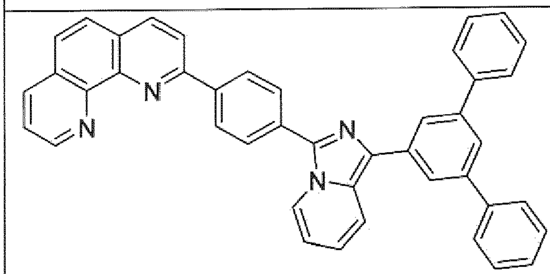 I-133 | 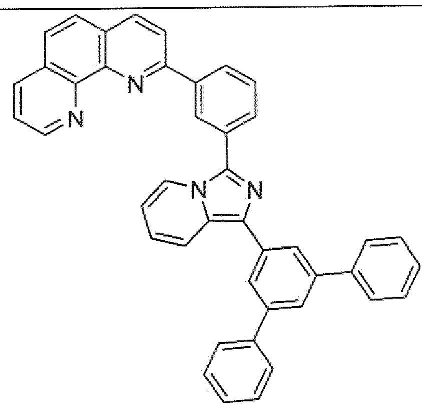 I-134 |

| | |
|---|---|
| 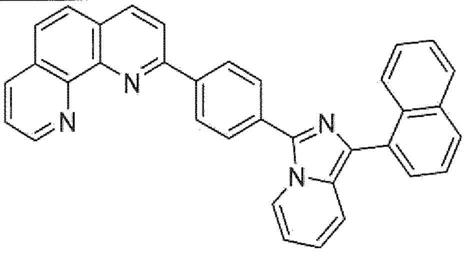 I-135 | 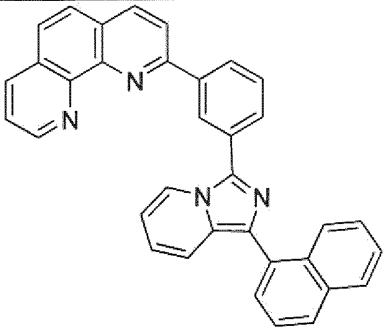 I-136 |
| 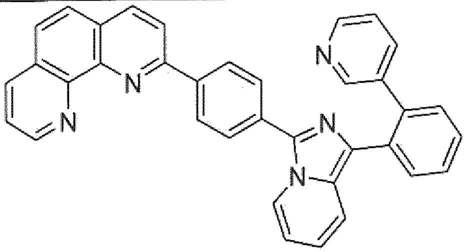 I-137 | 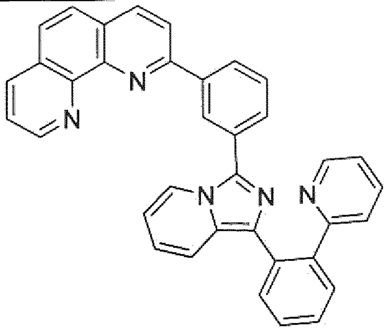 I-138 |
| 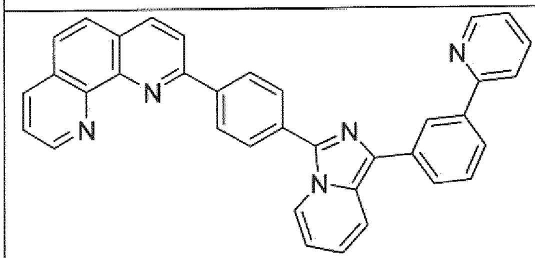 I-139 | 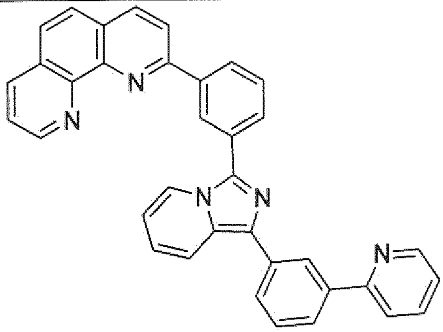 I-140 |
| 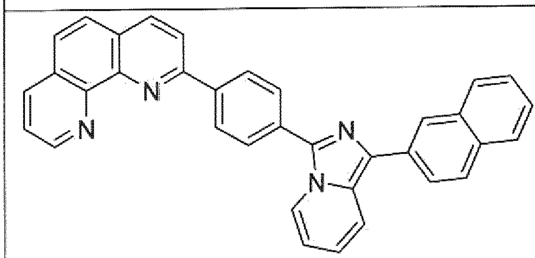 I-141 | 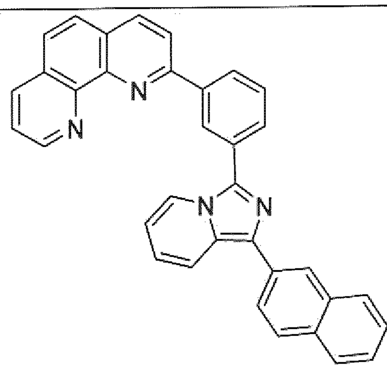 I-142 |

| | |
|---|---|
| 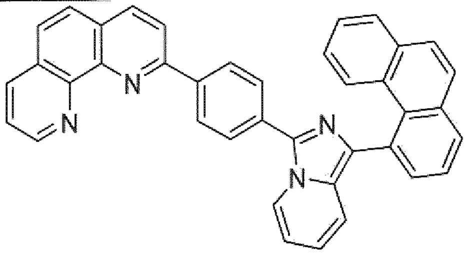 I-143 | 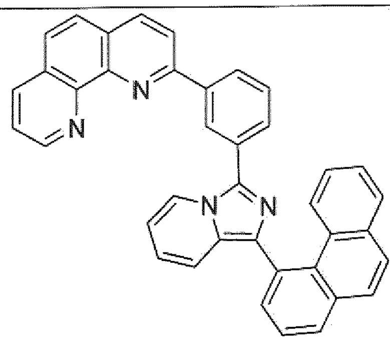 I-144 |
| 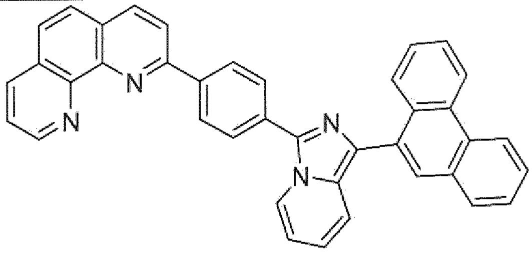 I-145 | 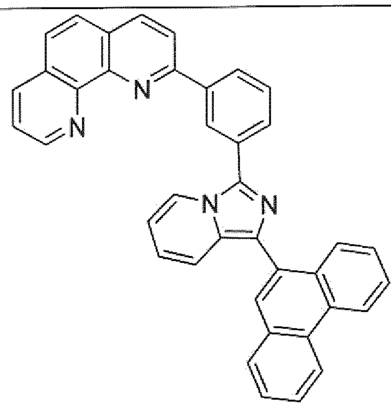 I-146 |
| 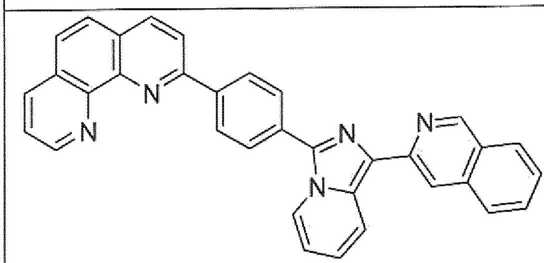 I-147 | 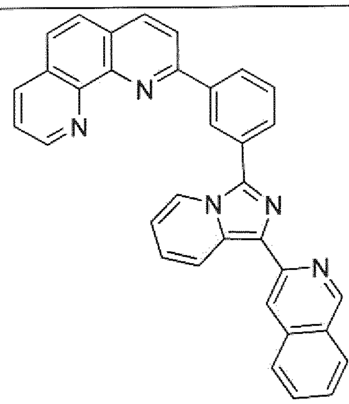 I-148 |
| 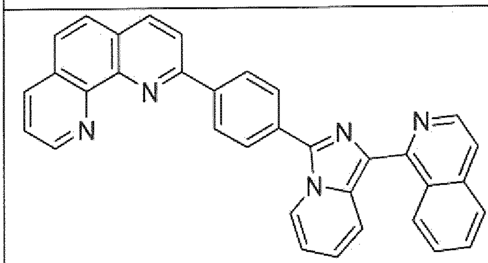 I-149 | 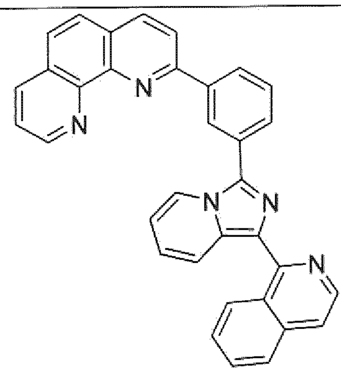 I-150 |

wherein the respective compound can be undeuterated, partially deuterated or fully deuterated.

According to an embodiment, the compound of formula (I) is selected from the compounds shown in Table 4.

Table 4:

| | |
|---|---|
| 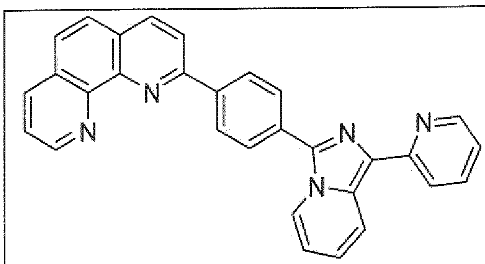 | 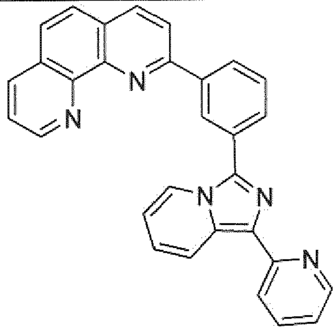 |
| 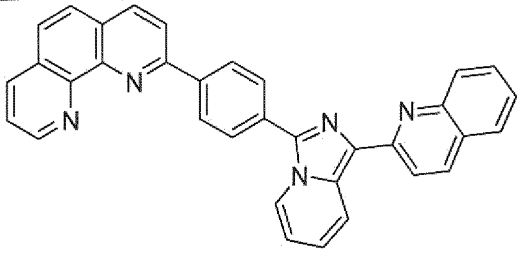 I-77 | 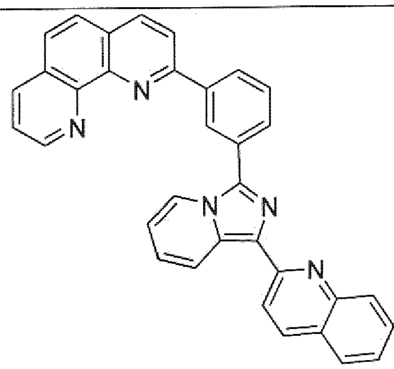 I-78 |
| 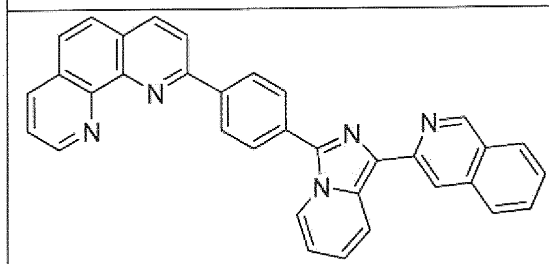 I-147 | 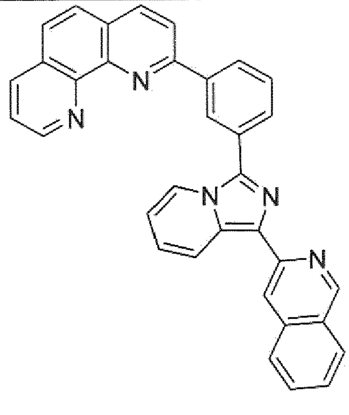 I-148 |
| 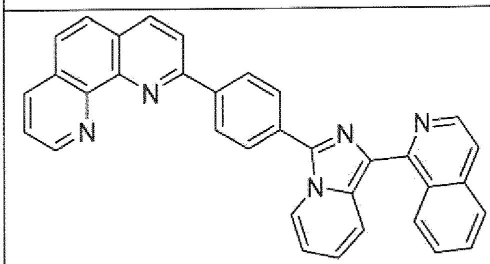 I-149 | 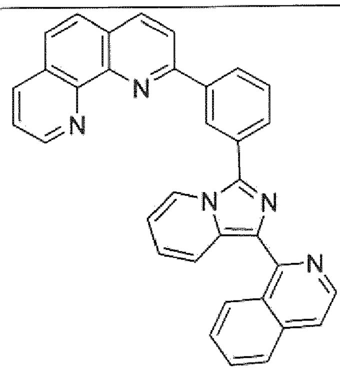 I-150 |

| | |
|---|---|
| 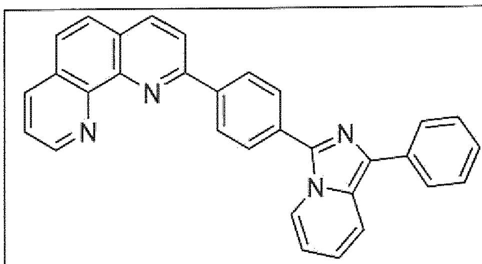 I-39 | 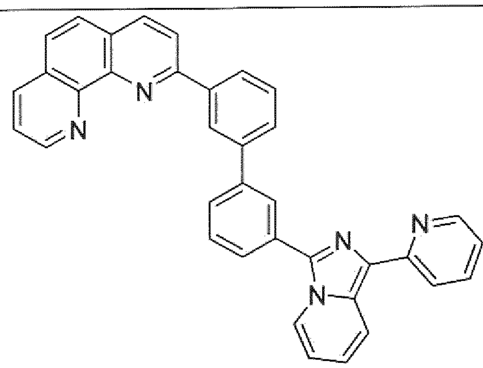 I-13 |
| 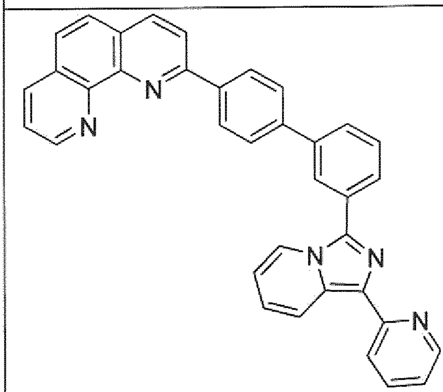 I-10 | 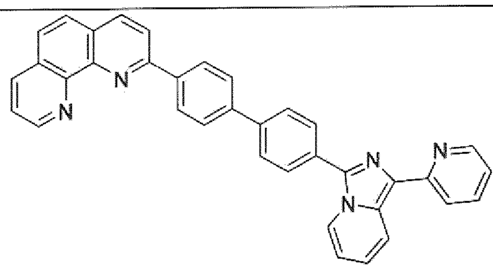 I-8 |
| 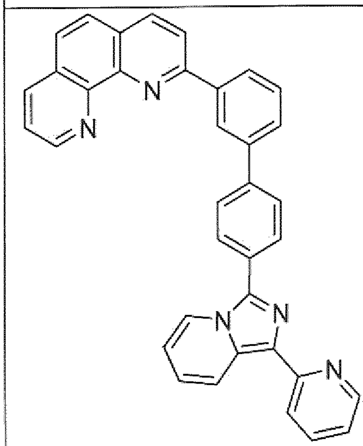 I-9 | 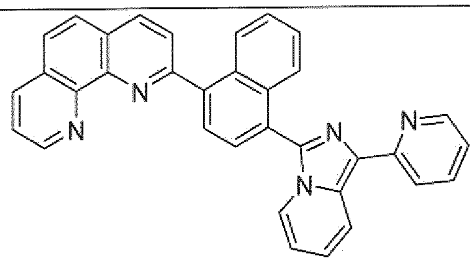 I-17 |
| 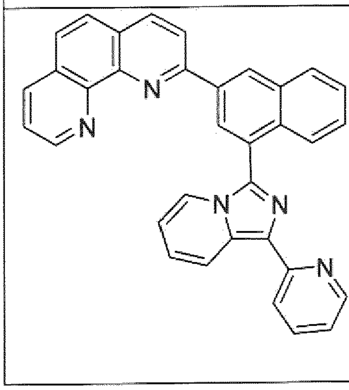 I-19 | |

wherein the respective compound can be undeuterated, partially deuterated or fully deuterated.

According to an embodiment, the compound of formula (I) is selected from the compounds shown in Table 5.

Table 5:

| | |
|---|---|
| 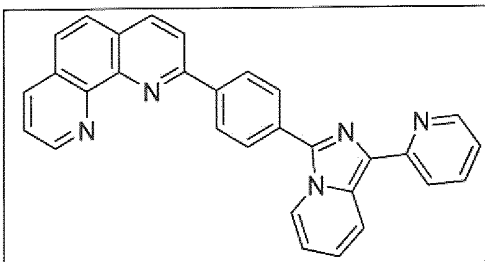 I-1 | 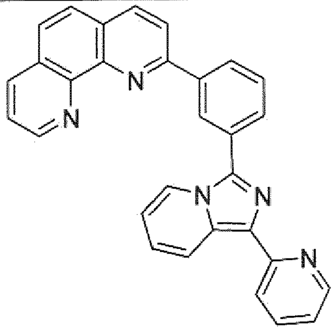 I-2 |
| 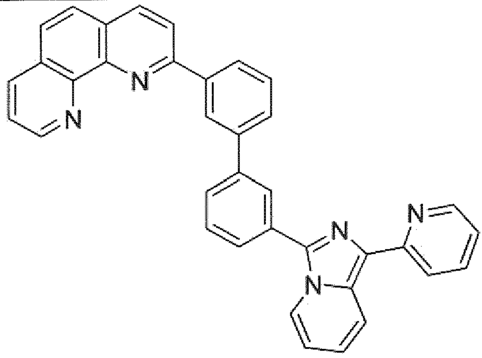 I-13 | 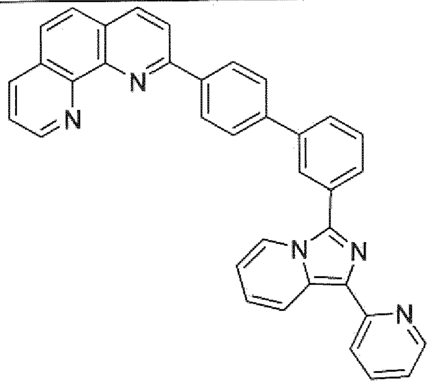 I-10 |
| 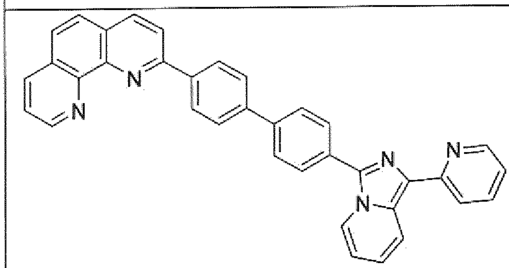 I-8 | 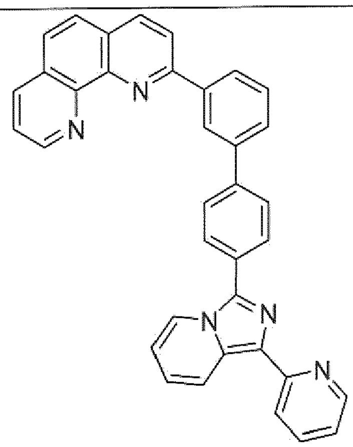 I-9 |

| | |
|---|---|
| 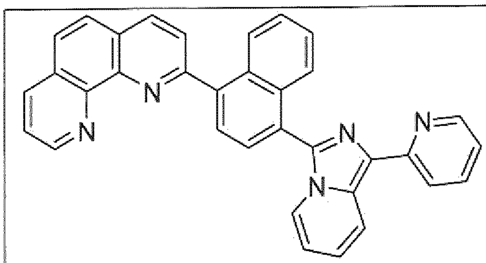 I-17 | 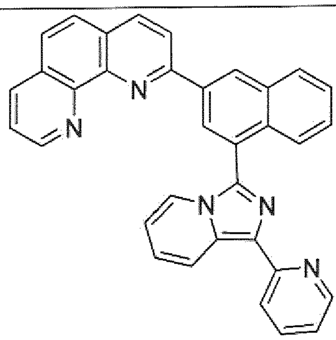 I-19 |
| 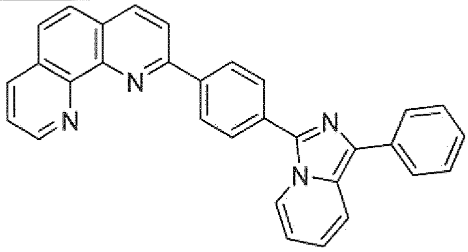 I-39 | |

wherein the respective compound can be undeuterated, partially deuterated or fully deuterated.

According to an embodiment, the compound of formula (I) is selected from the compounds shown in Table 6.

Table 6:

| | |
|---|---|
| 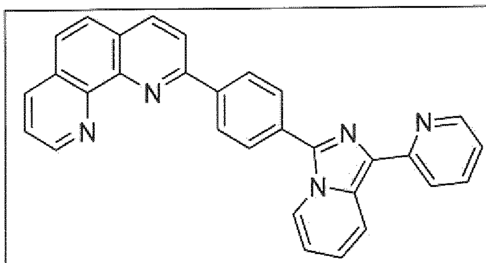 I-1 | 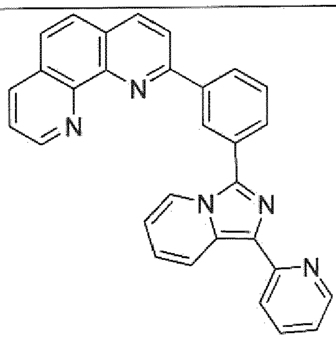 I-2 |
| 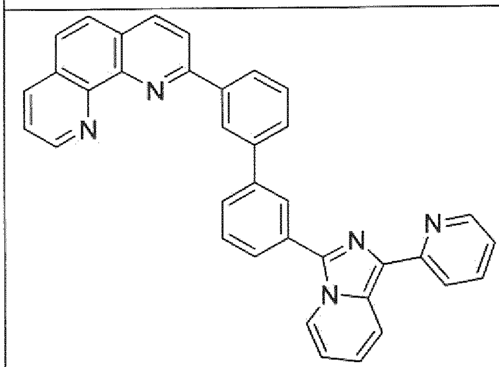 I-13 | 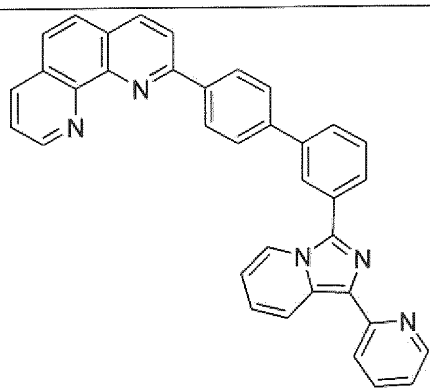 I-10 |

| | |
|---|---|
| 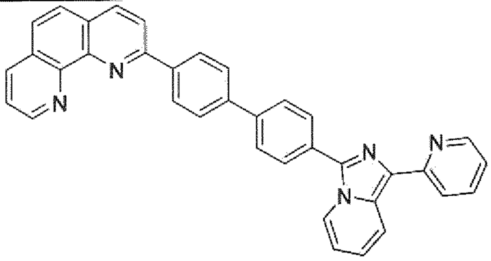 I-8 | 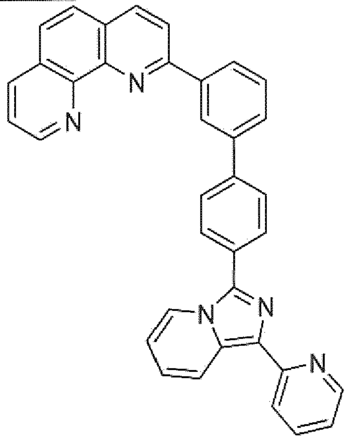 I-9 |
| 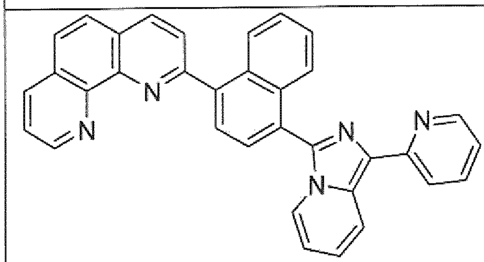 I-17 | 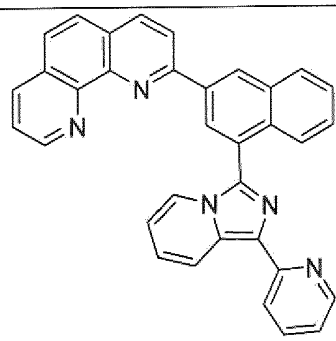 I-19 |

wherein the respective compound can be undeuterated, partially deuterated or fully deuterated.

According to an embodiment, the compound of formula (I) is selected from the compounds shown in Table 7.

Table 7:

| | |
|---|---|
| 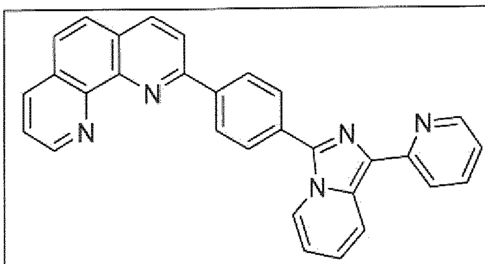 I-1 | 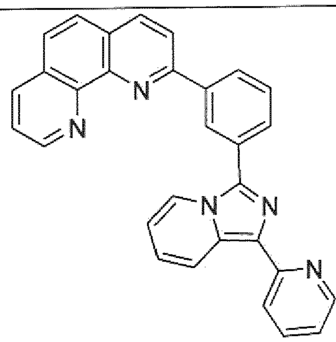 I-2 |

| | |
|---|---|
| 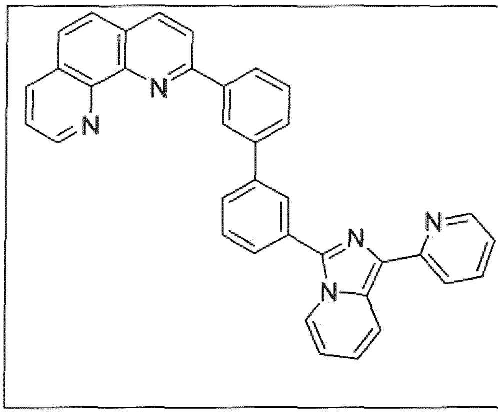 I-13 | 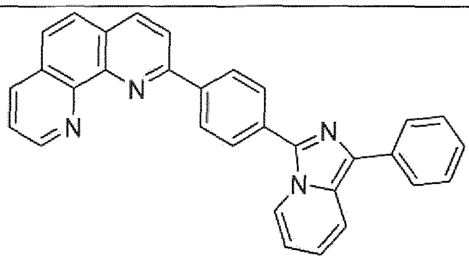 I-39 |

wherein the respective compound can be undeuterated, partially deuterated or fully deuterated.

According to an embodiment, the compound of formula (I) is selected from the compounds shown in Table 8.

Table 8:

| | |
|---|---|
| 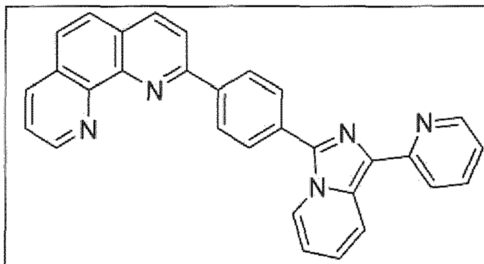 I-1 | 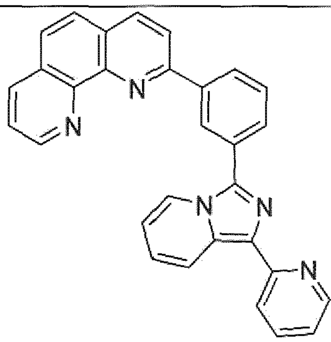 I-2 |

wherein the respective compound can be undeuterated, partially deuterated or fully deuterated.

First electron transport layer

According to an embodiment, the first electron transport layer is in direct contact with the first emission layer.

According to an embodiment, the first electron transport layer is closer to the anode layer than any other electron transport layer within the organic electroluminescent device.

According to an embodiment, the first electron transport layer which is in direct contact with the first n-type charge generation layer is the electron transport layer closest to the anode layer.

According to an embodiment, the first electron transport layer is arranged between the first emission layer and the first charge generation layer.

The thickness of the first electron transport layer may be in the range from 0.5 nm to 50 nm; alternatively in the range from 1 nm to about 40 nm; or alternatively in the range of 2 nm to 30 nm.

According to one embodiment of the present invention the first electron transport layer is non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

The first electron transport layer is free of 8-hydroxyquinolinolato-lithium.

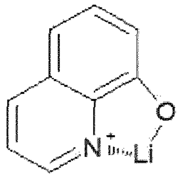

8-hydroxyquinolinolato-lithium

According to an embodiment, the first electron transport layer is free of a lithium metal complex.

According to an embodiment, the first electron transport layer is free of a metal complex.

According to one embodiment, the first electron transport layer comprises an electron transport compound.

The electron transport compound may have a molecular weight in the range of ≥400 g/mol to ≤2000 g/mol, preferably ≥415 g/mol to ≤1500 g/mol, more preferably ≥430 g/mol to ≤1000 g/mol, and most preferably ≥440 g/mol to ≤900 g/mol.

According to an embodiment, the first electron transport layer comprises an electron transport compound, wherein the electron transport compound comprises 8 to 13 aromatic or heteroaromatic rings, optionally 8 to 11 aromatic or heteroaromatic rings, optionally 9 to 11 aromatic or heteroaromatic rings, and optionally 9 aromatic or heteroaromatic rings, wherein one or more of the aromatic or heteroaromatic rings may be substituted with C1 to C4 alkyl. In this regard, an aromatic, respectively heteroaromatic ring is a single aromatic ring, for example a 6-membered aromatic ring such as phenyl, a 6-membered heteroaromatic ring such as pyridyl, a 5-membered heteroaromatic ring such as pyrrolyl etc. In a system of condensed (hetero)aromatic rings, each ring is considered as a single ring in this regard. For example, naphthalene comprises two aromatic rings.

The electron transport compound may comprise at least one heteroaromatic ring, optionally 1 to 5 heteroaromatic rings, optionally 1 to 4 heteroaromatic rings, optionally 1 to 3 heteroaromatic rings, and optionally 1 or 2 heteroaromatic rings.

The aromatic or heteroaromatic rings of the electron transport compound may be 6-membered rings.

The heteroaromatic rings of the electron transport compound may be a N-containing heteroaromatic ring, optionally all of the heteroaromatic rings are N-containing heteroaromatic rings, optionally all of the heteroaromatic rings heteroaromatic rings contain N as the only type of heteroatom.

The electron transport compound may comprise at least one six-member heteroaromatic ring containing one to three N-atoms in each heteroaromatic ring, optionally one to three 6-membered heteroaromatic rings containing one to three N-atoms in each heteroaromatic ring, respectively.

The at least one 6-membered heteroaromatic ring comprised in the electron transport compound may be an azine. The at least one 6-membered heteroaromatic ring comprised in the electron transport compound may be triazine, diazine, pyrazine, pyrimidine pyridine preferably triazine.

If the electron transport compound comprises two or more heteroaromatic rings, the heteroaromatic rings may be separated from each other by at least on aromatic ring which is free of a heteroatom.

In an embodiment, the heteroatoms in the heteroaromatic rings of the electron transport compound are bound into the molecular structure of the electron transport compound by at least one double bond.

According to an embodiment, wherein the compound comprising at least one nitrogen atom in a six-member aromatic ring in the electron transport layer is selected from formula (xxa) or formula (xxb) shown in Table 9.

Table 9:

| | |
|---|---|
| 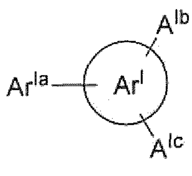 (xxa) | 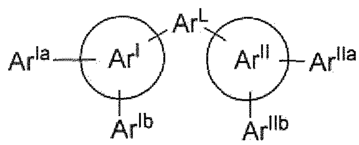 (xxb) |

wherein $Ar^{I}$ is a substituted or unsubstituted $C_3$ to $C_{40}$ heteroaromatic ring system comprising at least one nitrogen atom,

wherein $Ar^{II}$ is substituted or unsubstituted $C_3$ to $C_{40}$ heteroaromatic ring system comprising at least one nitrogen atom,

wherein the substituents on $Ar^{I}$ and $Ar^{II}$ are, identically or differently on each occurrence, D, a monovalent aromatic or heteroaromatic ring system having 5 to 60 aromatic ring atoms, which may in each case be substituted by one or more radicals $R^a$;

wherein $Ar^{Ia}$, $Ar^{Ib}$, $Ar^{Ic}$, $Ar^{IIa}$, and $Ar^{IIb}$ are, identically or differently on each occurrence, H, D, a monovalent aromatic or heteroaromatic ring system having 5 to 60 aromatic ring atoms, which may in each case be substituted by one or more radicals $R^a$;

wherein at least one of $Ar^{Ia}$, $Ar^{Ib}$, $Ar^{Ic}$ in formula (xxa) and in case of formula (xxb) at least one of

$Ar^{Ia}$, $Ar^{Ib}$, $Ar^{IIa}$, and $Ar^{IIb}$ is independently selected from a monovalent aromatic or heteroaromatic ring system having 5 to 60 aromatic ring atoms, which may in each case be substituted by one or more radicals $R^a$;

wherein $Ar^{L}$ is a divalent aromatic or heteroaromatic ring system having 5 to 60 aromatic ring atoms, which is optionally substituted by one or more radicals $R^a$;

wherein $R^a$ is on each occurrence, identically or differently, H, D, F, Cl, Br, I, CHO, $N(R^b)_2$, $N(Ar^{1s})_2$, $B(Ar^{1s})_2$, $C(=O)Ar^{1s}$, $P(=Y)(R^c)_2$, $S(=O)Ar^{1s}$, $S(=O)_2Ar^{1s}$, $CR^b=CR^bAr^{1s}$, CN, $NO_2$, $Si(R^b)_3$, $B(OR^b)_2$, $B(R^b)_2$, $B(N(R^b)_2)_2$, $OSO_2R^b$, a substituted or unsubstituted straight-chain $C_1$ to $C_{20}$ alkyl, a substituted or unsubstitutedstraight-chain $C_1$ to $C_{20}$ alkenyl, a substituted or unsubstituted straight-chain $C_1$ to $C_{20}$ alkynyl, a substituted or unsubstituted straight-chain $C_1$ to $C_{20}$ alkoxy, a substituted or unsubstituted straight-chain $C_1$ to $C_{20}$ thioalkoxy, substituted or unsubstituted branched $C_3$ to $C_{20}$ alkyl, a substituted or unsubstituted branched $C_3$ to $C_{20}$ alkenyl, a substituted or unsubstituted branched $C_3$ to $C_{20}$ a substituted or unsubstituted branched $C_3$ to $C_{20}$ alkynyl, a substituted or unsubstituted branched $C_3$ to $C_{20}$ alkoxy or a substituted or unsubstituted $C_3$ to $C_{20}$ branched thioalkoxy,substituted or unsubstituted cyclic $C_3$ to $C_{40}$ alkyl, substituted or unsubstituted cyclic $C_3$ to $C_{40}$ alkenyl, substituted or unsubstituted cyclic $C_3$ to $C_{40}$ alkinyl, substituted or unsubstituted cyclic $C_3$ to $C_{40}$ alkoxy or substituted or unsubstituted cyclic $C_3$ to $C_{40}$ thioalkoxy;substituted or unsubstituted heterocyclic $C_3$ to $C_{40}$ alkyl, substituted or unsubstituted heterocyclic $C_3$ to $C_{40}$ alkenyl, substituted or unsubstituted heterocyclic $C_3$ to $C_{40}$ alkynyl, substituted or unsubstituted heterocyclic $C_3$ to $C_{40}$ alkoxy or substituted or unsubstituted heterocyclic $C_3$ to $C_{40}$ thioalkoxy; wherein the one or more substituents, if present, is selected from $R^b$, where one or more non-adjacent $CH_2$ groups is optionally replaced by $R^bC=CR^b$, C=C, $Si(R^b)_2$, $Ge(R^b)_2$, $Sn(R^b)_2$, C=O, C=S, C=Se, $C=NR^b$, $P(=O)(R^b)$, SO, $SO_2$, $NR^b$, O, S or $CONR^b$ and where one or more H atoms is optionally replaced by D, F, Cl, Br, I, CN or $NO_2$, or an aromatic or heteroaromatic ring system having 5 to 60 aromatic ring atoms, which may in each case be substituted by one or more radicals $R^b$, or an aryloxy or heteroaryloxy group having 5 to 60 aromatic ring atoms, which is optionally substituted by one or more radicals $R^b$, or a combination of these systems; two or more adjacent substituents $R^a$ here optionally forms a mono- or polycyclic, aliphatic or aromatic ring system with one another;

wherein $Ar^{1s}$ is on each occurrence, identically or differently, an aromatic or heteroaromatic ring system having 5 to 30 aromatic ring atoms, which is optionally substituted by one or more radicals $R^b$; two radicals $Ar^{1s}$ here which are bonded to the same nitrogen, phosphorus or boron atom may also be linked to one another by a single bond or a bridge selected from $B(R^b)$, $C(R^b)_2$, $Si(R^b)_2$, C=O, C=NRb, $C=C(R^b)_2$, O, S, S=O, $SO_2$, $N(R^b)$, $P(R^b)$ and $P(=Y)R^c$;

wherein $R^b$ is on each occurrence, identically or differently, H, D or a $C_1$ to $C_{20}$ aliphatic hydrocarbyl, a $C_1$ to $C_{20}$ aryl and/or $C_1$ to $C_{20}$ heteroaryl, in which, in addition, H atoms is optionally replaced by D or F; two or more adjacent substituents $R^b$ here may also form a mono- or polycyclic, aliphatic or aromatic ring system with one another;

wherein Y is selected from O, S or Se, preferably O, and $R^c$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated C, to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

For the purposes of this invention, an aromatic or heteroaromatic ring system is intended to be taken to mean a system which does not necessarily contain only one aryl or one heteroaryl group or only aryl or heteroaryl groups, but instead in which a plurality of aryl or heteroaryl groups may also be interrupted by a short non-aromatic unit (preferably less than 10 percent of the atoms other than H), such as, for example, an $sp^3$-hybridised C, N or O atom. Thus, for example, systems such as 9,9'-spirobifluorene, 9,9-diarylfluorene, triarylamine, diaryl ether, stilbene, benzophenone, etc., are also intended to be taken to mean aromatic ring systems for the purposes of this invention. Likewise, an aromatic or heteroaromatic ring system is taken to mean systems in which a plurality of aryl or heteroaryl groups are linked to one another by single bonds, for example biphenyl, terphenyl or bipyridine.

According to one embodiment, $Ar^{I}$ is selected from pyrazine, pyridine, pyrimidine, or triazine, phenanthroline.
According to one embodiment, $Ar^{II}$ is selected from pyrazine, pyridine, pyrimidine, or triazine, phenanthroline.
According to one embodiment, $Ar^{I}$ in formula (xxa) or (xxxa) is selected from pyrazine, pyridine, pyrimidine, triazine, phenanthroline; or $Ar^{I}$ in formula (xxa) or (xxxa) and $Ar^{II}$ in formula (xxb) or (xxxb) are independently of each other selected from pyrazine, pyridine, pyrimidine, triazine, or phenanthroline.
According to an embodiment, wherein the compound comprising at least one nitrogen atom in a six-member aromatic ring in the electron transport layer is selected from formula (xxxa) or formula (xxxb):

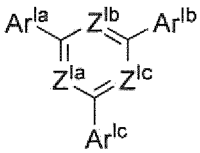 (xxxa)

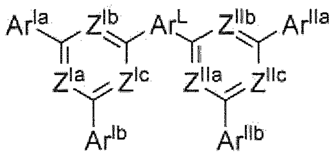 (xxxb)

wherein

$Z^{Ia}$ is selected from N or CH,

$Z^{Ib}$ is selected from N or CH,

$Z^{Ic}$ is selected from N or CH,

$Z^{IIa}$ is selected from N or CH,

$Z^{IIb}$ is selected from N or CH, and

$Z^{IIc}$ is selected from N or CH,

wherein in formula (XXxa) at least one of $Z^{Ia}$, $Z^{Ib}$ and $Z^{Ic}$ is selected from N,

wherein in formula (XXxb) at least one of $Z^{Ia}$, $Z^{ib}$, $Z^{Ic}$, $Z^{IIa}$, $Z^{IIb}$, and $Z^{IIc}$ is selected from N,

wherein $Ar^{Ia}$, $Ar^{Ib}$, $Ar^{Ic}$, $Ar^{IIa}$ and $Ar^{IIb}$ are identically or differently on each occurrence, a monovalent aromatic or heteroaromatic ring system having 5 to 60 aromatic ring atoms, which may in each case be substituted by one or more radicals $R^{a}$;

wherein $Ar^{L}$ is a divalent aromatic or heteroaromatic ring system having 5 to 60 aromatic ring atoms, which is optionally substituted by one or more radicals $R^{a}$,

wherein $R^{a}$ is on each occurrence, identically or differently, H, D, F, Cl, Br, I, CHO, $N(R^{b})_2$, $N(Ar^{1s})_2$, $B(Ar^{1s})_2$, $C(=O)Ar^{1s}$, $P(=Y)(R^{c})_2$, $S(=O)Ar^{1s}$, $S(=O)_2Ar^{1s}$, $CR^{b}=CR^{b}Ar^{1s}$, CN, $NO_2$, $Si(R^{b})_3$, $B(OR^{b})_2$, $B(R^{b})_2$, $B(N(R^{b})_2)_2$, $OSO_2R^{b}$, a substituted or unsubstituted straight-chain $C_1$ to $C_{20}$ alkyl, a substituted or unsubstitutedstraight-chain $C_1$ to $C_{20}$ alkenyl, a substituted or unsubstituted straight-chain $C_1$ to $C_{20}$ alkynyl, a substituted or unsubstituted straight-chain $C_1$ to $C_{20}$ alkoxy, a substituted or unsubstituted straight-chain $C_1$ to $C_{20}$ thioalkoxy, substituted or unsubstituted branched $C_3$ to $C_{20}$ alkyl, a substituted or unsubstituted branched $C_3$ to $C_{20}$ alkenyl, a substituted or unsubstituted branched $C_3$ to $C_{20}$ a substituted or unsubstituted branched $C_3$ to $C_{20}$ alkynyl, a substituted or unsubstituted branched $C_3$ to $C_{20}$ alkoxy or a substituted or unsubstituted branched $C_3$ to $C_{20}$ thioalkoxy,substituted or unsubstituted cyclic $C_3$ to $C_{40}$ alkyl, substituted or unsubstituted cyclic $C_3$ to $C_{40}$ alkenyl, substituted or unsubstituted cyclic $C_3$ to $C_{40}$ alkinyl, substituted or unsubstituted cyclic $C_3$ to $C_{40}$ alkoxy or substituted or unsubstituted cyclic $C_3$ to $C_{40}$ thioalkoxy;substituted or unsubstituted heterocyclic $C_3$ to $C_{40}$ alkyl, substituted or unsubstituted heterocyclic $C_3$ to $C_{40}$ alkenyl, substituted or unsubstituted heterocyclic $C_3$ to $C_{40}$ alkynyl, substituted or unsubstituted heterocyclic $C_3$ to $C_{40}$ alkoxy or substituted or unsubstituted heterocyclic $C_3$ to $C_{40}$ thioalkoxy;

wherein the one or more substituents, if present, is selected from $R^{b}$, where one or more non-adjacent $CH_2$ groups is optionally replaced by $R^{b}C=CR^{b}$, C=C, $Si(R^{b})_2$, $Ge(R^{b})_2$, $Sn(R^{b})_2$, C=O, C=S, C=Se, $C=NR^{b}$, $P(=O)(R^{b})$, SO, $SO_2$, $NR^{b}$, O, S or $CONR^{b}$ and where one or more H atoms is optionally replaced by D, F, Cl, Br, I, CN or $NO_2$, or an aromatic or heteroaromatic ring system having 5 to 60 aromatic ring atoms, which may in each case be substituted by one or

more radicals $R^b$, or an aryloxy or heteroaryloxy group having 5 to 60 aromatic ring atoms, which is optionally substituted by one or more radicals $R^b$, or a combination of these systems; two or more adjacent substituents $R^a$ here optionally forms a mono- or polycyclic, aliphatic or aromatic ring system with one another;

wherein $Ar^{1s}$ is on each occurrence, identically or differently, an aromatic or heteroaromatic ring system having 5 to 30 aromatic ring atoms, which is optionally substituted by one or more radicals $R^b$; two radicals $Ar^{1s}$ here which are bonded to the same nitrogen, phosphorus or boron atom may also be linked to one another by a single bond or a bridge selected from $B(R^b)$, $C(R^b)_2$, $Si(R^b)_2$, C=O, $C=NR^b$, $C=C(R^b)_2$, O, S, S=O, $SO_2$, $N(R^b)$, $P(R^b)$ and $P(=Y)R^c$;

wherein $R^b$ is on each occurrence, identically or differently, H, D or a $C_2$ to $C_{20}$ aliphatic hydrocarbyl, a $C_1$ to $C_{20}$ aryl and/or $C_1$ to $C_{20}$ heteroaryl, in which, in addition, H atoms is optionally replaced by D or F; two or more adjacent substituents $R^b$ here may also form a mono- or polycyclic, aliphatic or aromatic ring system with one another;

wherein Y is selected from O, S or Se, preferably O, and $R^c$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy.

For the purposes of this invention, an aromatic or heteroaromatic ring system is intended to be taken to mean a system which does not necessarily contain only aryl or heteroaryl groups, but instead in which a plurality of aryl or heteroaryl groups may also be interrupted by a short nonaromatic unit (preferably less than 10 percent of the atoms other than H), such as, for example, an $sp^3$-hybridised C, N or 0 atom. Thus, for example, systems such as 9,9'-spirobifluorene, 9,9-diarylfluorene, triarylamine, diaryl ether, stilbene, benzophenone, etc., are also intended to be taken to mean aromatic ring systems for the purposes of this invention. Likewise, an aromatic or heteroaromatic ring system is taken to mean systems in which a plurality of aryl or heteroaryl groups are linked to one another by single bonds, for example biphenyl, terphenyl or bipyridine.

According to an embodiment, the first electron transport layer comprises an electron transport compound, wherein the electron transport compound is selected from 2-([1,1'-biphenyl]-3-yl)-4-(2',6'-diphenyl-[1,1':4',1"-terphenyl]-4-yl)-6-phenyl-1,3,5-triazine, 2,2'-(1,3-Phenylene)bis[9-phenyl-1,10-phenanthroline], 2-(4-(1-(pyridin-2-yl)imidazo[1,5-a]pyridin-3-yl)phenyl)-1,10-phenanthroline, 3-(3-(9,10-diphenylanthracen-2-yl)phenyl)-1-(pyridin-2-yl)imidazo[1,5-a]pyridine, 2,4-diphenyl-6-(3"-(3,5,6-triphenylpyrazin-2-yl)-[1,1':3',1"-terphenyl]-3-yl)-1,3,5-triazine, 2-(3'-(9,9-dimethyl-9H-fluoren-2-yl)-[1,1'-biphenyl]-3-yl)-4,6-diphenyl-1,3,5-triazine, 2,4-diphenyl-6-(4',5',6'-triphenyl-[1,1':2',1":3",1‴:3‴,1⁗-quinquephenyl]-3⁗-yl)-1,3,5-triazine; 2-phenyl-4-(3-(pyridin-4-yl)phenyl)-6-(3-(3,5,6-triphenylpyrazin-2-yl)phenyl)pyrimidine; (3-(10-(3-(2,6-diphenylpyrimidin-4-yl)phenyl)anthracen-9-yl)phenyl)dimethylphosphine oxide.

According to one embodiment, wherein the electron transport compound has a LUMO energy level when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase of ≤ -1.50 eV, preferably ≤ -1.55 eV, preferably ≤ -1.60 eV, and most preferably ≤ -1.65 eV.

According to an embodiment, wherein the electron transport compound has a LUMO energy level when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase in the range of = -2.5 eV to ≤ -1.5 eV, preferably ≥ -2.0 eV to ≤ - 1.5 eV, more preferably ≥ -1.95 eV to ≤ -1.55 eV, even more preferably ≥ -1.90 eV to ≤ -1.6 eV, even more preferably ≥ -1.90 eV to ≤ -1.65 eV, and most preferably ≥ -1.87 eV to ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 4.0 D; alternatively ≥ o D to ≤ 3.7 D; alternatively ≥ 0 D to ≤ 3.5 D; alternatively ≥ 0 D to ≤ 3.0 D; alternatively ≥ 0 D to ≤ 2.7 D; alternatively ≥ 0 D to ≤ 2.5 D; alternatively ≥ o D to ≤ 2.0 D; alternatively ≥ 0 D to ≤ 1.5 D; alternatively ≥ 0 D to ≤ 1.0 D.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 3.7 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.90 eV and ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 3.7 D; and the LUMO energy level of the electron transport compound of the first electron transport layer when calculated with the program package TURBOMOLE V6.5 (TURBOMOLE GmbH,

Litzenhardtstrasse 19, 76135 Karlsruhe, Germany) by applying the hybrid functional B3LYP with a 6-31G* basis set in the gas phase is in the range of ≥ -1.87 eV and ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 3.5 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.90 eV and ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 3.5 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.87 eV to ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 3.0 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.90 eV and ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 3.0 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.87 eV to ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 2.7 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.90 eV and ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 2.7 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.87 eV to ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 2.5 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.90 eV and ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 2.5 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.87 eV to ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 2.0 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.90 eV and ≤ -1.65 eV.

According to an embodiment, wherein the electron transport compound has a molecular dipole moment calculated by the TURBOMOLE V6.5 program package using hybrid functional B3LYP and Gaussian 6-31G* basis

set in the gas phase in the range of ≥ 0 D to ≤ 2.0 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.87 eV to ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 1.5 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.90 eV and ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 1.5 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.87 eV to ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 1.0 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.90 eV and ≤ -1.65 eV.

According to an embodiment, wherein the electron transport compound has a molecular dipole moment calculated by the TURBOMOLE V6.5 program package using hybrid functional B3LYP and Gaussian 6-31G* basis set in the gas phase in the range of ≥ 0 D to ≤ 1.0 D; and the LUMO energy level of the electron transport compound of the first electron transport layer when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is in the range of ≥ -1.87 eV to ≤ -1.65 eV.

The unit for the dipole moment "Debye" is abbreviated with the symbol "D". In this regard, the dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule. The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

<u>Further electron transport layers (not the first electron transport layer)</u>

The organic electroluminescent device according to the invention may comprise besides the first electron transport layer one or more further electron transport layer. Such a further electron transport layer does not have to be free of 8-Hydroxyquinolinolato-lithium. In particular, a further electron transport layer which is closest to the cathode preferably contains 8-Hydroxyquinolinolato-lithium, i.e. the electron transport layer which is not interrelated to a charge generation layer.

In accordance with one preferred embodiment of the invention, the electron transport layer may be the inventive organic semiconductor layer comprising the inventive compound represented by the general Formula (I) as defined herein.

According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The one or more further electron transport layer(s) of the organic electroluminescent device may comprise the compound represented by general formula (xxxa) or formula (xxxb) as defined above as the organic electron transport matrix (ETM) material. The electron transport layer may comprise, besides or instead of the compound represented by the general Formula (I), further ETM materials known in the art. Likewise, the electron transport layer may comprise as the only electron transport matrix material the compound represented by general Formula (I). In case that the inventive organic electronic device comprises more than one electron transport layers, the compound represented by the general Formula (I) may be comprised in only one of the electron transport layers, in more than one of the electron transport layers or in all of the electron transport layers. In accordance with the invention, the electron transport layer may comprise, besides the ETM material, at least one additive as defined below.

Further, the electron transport layer may comprise one or more n-type dopants. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (I) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

<u>Specific embodiments of the organic electroluminescent device according to the invention</u>

According to an embodiment, wherein an organic electroluminescent device is provide, comprising an anode layer, a cathode layer, a first emission layer and a second emission layer, a first charge generation layer, a first electron transport layer,

wherein the first charge generation layer is arranged between the first emission layer and the second emission layer,

wherein the first charge generation layer comprises a first n-type charge generation layer and a firstp-type charge generation layer,

wherein the first n-type charge generation layer is closer to the anode layer than the first p-type charge generation layer,

wherein the first p-type charge generation layer is closer to the cathode layer than the first n-type charge generation layer,

wherein the first electron transport layer is arranged in direct contact with the first n-type charge generation layer, and

wherein the first n-type charge generation layer comprises a metal dopant and a compound of formula (I)

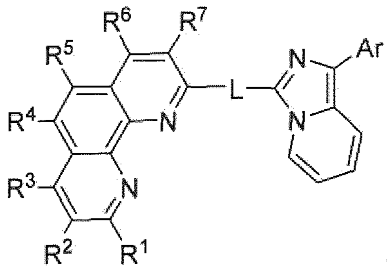

(I),

Ar is selected from substituted or unsubstituted $C_6$ to $C_{24}$ aryl or substituted or unsubstituted $C_2$ to $C_{24}$ heteroaryl,

wherein one or more substituents on Ar, if present, are independently selected from D, substituted or unsubstituted $C_6$

to $C_{18}$ aryl, substituted or unsubstituted $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $PX^1(R^8)_2$, halogen, F or CN, wherein the substituents may be linked via a single bond or a heteroatom to form a ring, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O,

$R^1$ to $R^7$ are independently selected from H, D, substituted or unsubstituted $C_1$ to $C_{16}$ alkyl, substituted or unsubstituted $C_6$ to $C_{24}$ aryl or substituted or unsubstituted $C_2$ to $C_{24}$ heteroaryl, CN, halogen, F $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, or $PX^1(R^8)_2$ wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O,

wherein one or more substituents on $R^2$ to $R^7$, if present, are independently selected from D, $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, halogen, F, CN or $PX^1(R^8)_2$, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy;

and $X^1$ is selected from O, S or Se, preferably O,

wherein L is selected from substituted or unsubstituted $C_6$ to $C_{24}$ aryl, substituted or unsubstituted $C_2$ to $C_{24}$ heteroaryl,

wherein one or more substituents on L, if present, are independently selected from D, $C_6$ to $C_{18}$ aryl, $C_3$ to $C_{20}$ heteroaryl, $C_1$ to $C_{16}$ alkyl, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, halogen, F, CN or $PX^1(R^8)_2$, wherein the substituents may be linked via a single bond or a heteroatom to form a ring, wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O,

wherein formula (I) can be undeuterated, partially deuterated or fully deuterated,

wherein the metal dopant is selected from Ytterbium;

wherein the first electron transport layer is free of 8-Hydroxyquinolinolato-lithium.

Further layers

In accordance with the invention, the organic electronic device may comprise, besides the layers already mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

*Anode electrode*

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.
When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.
The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).
The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about - 5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.
The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.
The HTL may be formed of any compound that is commonly used to form a HTL. Compounds that can be suitably used are disclosed for example in Yasuhiko Shirota and Hiroshi Kageyama, Chem. Rev. 2007, 107, 953-1010. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.
The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.
When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The EML may be formed on the HTL by vacuum deposition, spin coating, slot-die coat-ing, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp) 3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the inventive organic semiconductor layer comprising or consisting of the inventive compound represented by the general Formula (1) as defined above.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

The hole blocking layer may also be described as a-ETL or auxiliary ETL.

*Electron injection layer (EIL)*

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.
The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

*Cathode electrode*

The cathode electrode is formed on the EIL if present. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.
The thickness of the cathode electrode may be in the range from about 5 nm to about 1000 nm, for example, in the range from about 10 nm to about 100 nm. When the thickness of the cathode electrode is in the range from about 5 nm to about 5o nm, the cathode electrode may be transparent or semitransparent even if formed from a metal or metal alloy.
It is to be understood that the cathode electrode is not part of an electron injection layer or the electron transport layer.

*Charge generation layer/hole generating layer*

The first charge generation layer (CGL) comprises a p- type and an n-type charge generation layer. An interlayer may be arranged between the p-type charge generation layer and the n-type charge generation layer.
Typically, the charge generation layer is a pn junction joining an n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.
Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode, while the hole generating layer provides holes to the second light emission layer arranged between the first emission layer and the cathode.
Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, FeCl3, FeF3, and SbCl5. Also, the host can be one selected from a group consisting of N,N'-di(naphthalen-1-yl)-N,N-diphenylbenzidine (NPB), N,N'-diphenyl-N,N'-bis(3-methylphenyl)-1,1-biphenyl-4,4'-diamine (TPD) and N,N',N'-tetranaphthylbenzidine (TNB). The p-type charge generation layer may consist of CNHAT.

<u>Organic electroluminescent device</u>

According to an embodiment, the organic electroluminescent device further comprises a layer selected from hole injection layer, hole transport layer, electron blocking layer, a hole blocking layer, an electron transport layer, and an electron injection layer.
According to an embodiment, the organic electroluminescent device further comprises a hole injection layer, a first hole transport layer, a second hole transport layer, first electron blocking layer, second electron blocking layer, optionally a first hole blocking layer, optionally a second hole blocking layer, a second electron transport layer, and an electron injection layer.
According to an embodiment , the organic electroluminescent device comprises an anode layer (120), a hole injection layer (130), a first hole transport layer (141), a first electron blocking layer (142), a first emission layer (145), a first optional hole blocking layer(147), and a first electron transport layer (149), wherein the first electron transport layer (149) is free of 8-Hydroxyquinolinolato-lithium, preferably free of a lithium organic metal complex, and more preferably free of a

lithium metal complex, wherein the first electron transport layer (149) is preferably in direct contact with the first emission layer (145), a first charge generation layer (160) disposed over the first electron transport layer (149), wherein the first charge generation layer (160) comprises a first n-type charge generation layer (161), and a first p-type charge generation layer (162), wherein the first n-type charge generation layer (161) comprises a compound of formula (I) and a metal dopant, wherein the metal dopant is selected from Ytterbium, and wherein the first electron transport layer (149) is in direct contact with the first n-type charge generation layer (161), a second hole transport layer (241), a second electron blocking layer (242), a second emission layer (245), a second optional hole blocking layer (247), an electron transport layer (148), an electron injection layer (180), and a cathode layer (190).

According to an embodiment , the organic electroluminescent device further comprises a third emission layer, a second electron transport layer, and a second charge generation layer,

wherein the third emission layer is arranged between the second charge generation and the cathode layer,

wherein the second charge generation layer is arranged between the first charge generation layer and the cathode layer, and between the second emission layer and the third emission layer

wherein the second charge generation layer comprises a second n-type charge generation layer and second p-type charge generation layer,

wherein the second n-type charge generation layer is arranged closer to the anode layer than the second p-type charge generation layer,

wherein the second p-type charge generation layer is arranged closer to the cathode layer than the second n-type charge generation layer,

wherein the second electron transport layer is arranged between the second emission layer and the third emission layer,

wherein the second electron transport layer is in direct contact with the second n-type charge generation layer,

wherein

wherein the second n-type charge generation layer comprises a metal dopant and a compound of formula (I),

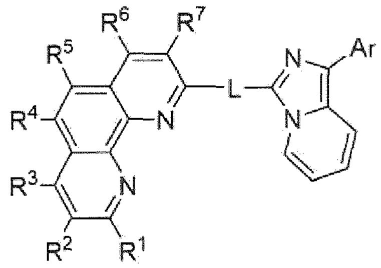

(I);

wherein $R^1$ to $R^7$; L and Ar may be selected as defined above;

wherein the compound of formula (I) in the first and second n-type charge generation layer and the compound of formula (I) is selected the same or different preferably the same,

wherein the metal dopant is Ytterbium; and

wherein the second electron transport layer is free of 8-Hydroxyquinolinolato-lithium.

According to an embodiment , the organic electroluminescent device comprises an anode layer (120), a hole injection layer (130), a hole transport layer (141), a first electron blocking layer (142), a first emission layer (145), a first optional hole blocking layer (147), a first electron transport layer (149), wherein the first electron transport layer (149) is free of 8-Hydroxyquinolinolato-lithium, preferably free of a lithium organic metal complex, and more preferably free of a lithium

metal complex, wherein the first electron transport layer (149) is preferably in direct contact with the first emission layer (145), a first charge generation layer (160) disposed over the first electron transport layer (149), wherein the first charge generation layer (160) comprises a first n-type charge generation layer (161), and a first p-type charge generation layer (162), wherein the first n-type charge generation layer (161) comprises a compound of formula (I) and a metal dopant, wherein the metal dopant is selected from Ytterbium, and wherein the first electron transport layer (149) is in direct contact with the first n-type charge generation layer (261), a second hole transport layer (241), and a second electron blocking layer (242), a second emission layer (245), a second optional hole blocking layer (247), a second electron transport layer (249), wherein the second electron transport layer (ETL2) 249 is free of 8-Hydroxyquinolinolato-lithium, preferably free of a lithium organic metal complex, and more preferably free of a lithium metal complex, wherein the second electron transport layer (249) is preferably in direct contact with the second emission layer (245), a second charge generation layer (260) disposed over the second electron transport layer (249), wherein the second charge generation layer (CGL2) 260 comprises a second n-type charge generation layer (n-CGL2) 261, and a first p-type charge generation layer (p-CGL2) 262, wherein the second n-type charge generation layer (n-CGL2) 261 comprises a compound of formula (I) and a metal dopant, wherein the metal dopant is selected from Ytterbium, and wherein the second electron transport layer (ETL1) 249 is in direct contact with the second n-type charge generation layer (n-CGL2) 261, wherein the second n-type charge generation layer (n-CGL2) 261 can be selected the same or different than the first n-type charge generation layer (n-CGL1) 161, and wherein the second p-type charge generation layer (p-CGL2) 262 can be selected the same or different than the first p-type charge generation layer (p-CGL1) 161, a third hole transport layer (341), and a third electron blocking layer (342), a third emission layer (345). a third optional hole blocking layer (347), an electron transport layer (148), an electron injection layer (180), and a cathode layer (190).

Preparation of the organic electroluminescent device

According to another aspect of the present invention, there is provided a method of manufacturing an organic electroluminescent device (OLED), the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of Formula (I) according to the invention, and
- a second deposition source to release Yb;

the method comprising the steps of forming the organic semiconductor layer; whereby for an organic light-emitting diode (OLED):

- the organic semiconductor layer is formed by releasing the compound of Formula (I) according to the invention from the first deposition source and Yb from the second deposition source.

According to various embodiments of the present invention, the method may further include forming on the anode electrode, an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,

- on the first anode electrode an emission layer is formed,
- on the emission layer an electron transport layer stack is formed, optionally a hole blocking layer is formed on the emission layer and an organic semiconductor layer is formed,
- and finally a cathode electrode is formed,
- optional a hole injection layer, a hole transport layer, and a hole blocking layer, formed in that order between the first anode electrode and the emission layer,
- optional an electron injection layer is formed between the organic semiconductor layer and the cathode electrode.

According to various embodiments of the present invention, the method may further comprise forming an electron injection layer on the organic semiconductor layer. However, according to various embodiments of the OLED of the present invention, the OLED may not comprise an electron injection layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

In one embodiment, the organic electronic device according to the invention comprising an organic semiconductor layer comprising a compound according to Formula (I) may further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

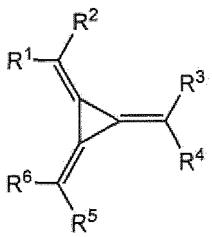 (XX) 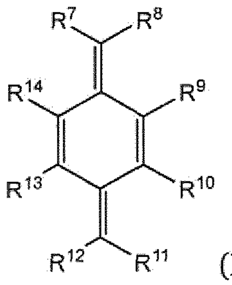 (XXIa) 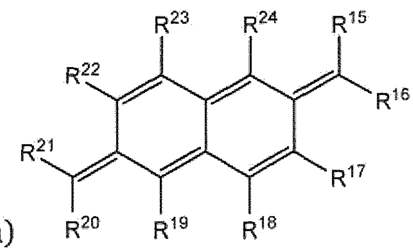 (XXIb),

wherein (as an exception different to the description above) $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

According to one embodiment of the present invention, the organic semiconductor layer comprising compound of formula (I) is adjacent to a layer comprising a compound of formula (XX), (XXIa) or (XXIb).

According to one embodiment of the present invention, the organic semiconductor layer comprising compound of formula (I) is in direct contact to a layer comprising a compound of formula (XX), (XXIa) or (XXIb).

Hereinafter, the embodiments are illustrated in more detail with reference to examples. However, the present disclosure is not limited to the following examples. Reference will now be made in detail to the exemplary aspects.

## Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a schematic sectional view of an organic electroluminescent device according to an exemplary embodiment of the present invention.

FIG. 2 is a schematic sectional view of an organic electroluminescent device according to an exemplary embodiment of the present invention.

FIG. 3 is a schematic sectional view of an organic electroluminescent device according to an exemplary embodiment of the present invention.

FIG. 4 is a schematic sectional view of an organic electroluminescent device according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

Fig. 1 is a schematic sectional view of an organic electroluminescent device 100, according to one exemplary embodiment of the present invention.

Referring to Fig. 1 the organic electroluminescent device 100 includes an anode layer (ANO) 120, a first emission layer (EML1) 145, and a first electron transport layer (ETL1) 149, wherein the first electron transport layer (ETL1) 149 is free of 8-Hydroxyquinolinolato-lithium, preferably free of a lithium organic metal complex, and more preferably free of a lithium metal complex.

The organic electroluminescent device further comprises a first charge generation layer (CGL1) 160 disposed over the first electron transport layer (ETL1) 149, wherein the first charge generation layer (CGL1) 160 comprises a first n-type charge generation layer (n-CGL1) 161, and a first p-type charge generation layer (p-CGL1) 162, wherein the first n-type charge generation layer (n-CGL1) 161 comprises a compound of formula (I) and a metal dopant, wherein the metal dopant is selected from Ytterbium, and wherein the first electron transport layer (ETL1) 149 is in direct contact with the first n-type charge generation layer (n-CGL1) 161.

The organic electroluminescent device 100 further comprises a second emission layer (EML2) 245, and a cathode layer (CAT) 190.

Fig. 2 is a schematic sectional view of an organic electroluminescent device 100, according to one exemplary embodiment of the present invention.

Referring to Fig. 2 the organic electroluminescent device 100 includes an anode layer (ANO) 120, a hole injection layer (HIL) 130, a first hole transport layer (HTL1) 141, a first electron blocking layer (EBL1) 142, a first emission layer (EML1) 145, a first optional hole blocking layer (HBL1) 147, and a first electron transport layer (ETL1) 149, wherein the first electron transport layer (ETL1) 149 is free of 8-Hydroxyquinolinolato-lithium, preferably free of a lithium organic metal complex, and more preferably free of a lithium metal complex.

The organic electroluminescent device further comprises a first charge generation layer (CGL1) 160 disposed over the first electron transport layer (ETL1) 149, wherein the first charge generation layer (CGL1) 160 comprises a first n-type charge generation layer (n-CGL1) 161, and a first p-type charge generation layer (p-CGL1) 162, wherein the first n-type charge generation layer (n-CGL1) 161 comprises a compound of formula (I) and a metal dopant, wherein the metal dopant is selected from Ytterbium, and wherein the first electron transport layer (ETL1) 149 is in direct contact with the first n-type charge generation layer (n-CGL1) 161.

The organic electroluminescent device 100 further comprises a second hole transport layer (HTL2) 241, a second electron blocking layer (EBL2) 242.

The organic electroluminescent device 100 further comprises a second emission layer (EML2) 245.

The organic electroluminescent device 100 further comprises a second optional hole blocking layer (HBL2) 247, an electron transport layer (ETL) 148, an electron injection layer (EIL) 180, and a cathode layer (CAT) 190.

Fig. 3 is a schematic sectional view of an organic electroluminescent device 100, according to one exemplary embodiment of the present invention.

Referring to Fig. 3 the organic electroluminescent device 100 includes an anode layer (ANO) 120, a hole injection layer (HIL) 130, a hole transport layer (HTL1) 141, a first electron blocking layer (EBL1) 142, and a first emission layer (EML1) 145.

The organic electroluminescent device 100 further comprises a first optional hole blocking layer (HBL1) 147.

The organic electroluminescent device 100 further comprises a first electron transport layer (ETL1) 149, wherein the first electron transport layer (ETL1) 149 is free of 8-Hydroxyquinolinolato-lithium, preferably free of a lithium organic metal complex, and more preferably free of a lithium metal complex.

The organic electroluminescent device further comprises a first charge generation layer (CGL1) 160 disposed over the first electron transport layer (ETL1) 149, wherein the first charge generation layer (CGL1) 160 comprises a first n-type charge generation layer (n-CGL1) 161, and a first p-type charge generation layer (p-CGL1) 162, wherein the first n-type charge generation layer (n-CGL1) 161 comprises a compound of formula (I) and a metal dopant, wherein the metal dopant is selected from Ytterbium, and wherein the first electron transport layer (ETL1) 149 is in direct contact with the first n-type charge generation layer (n-CGL1) 161.

The organic electroluminescent device 100 further comprises a second hole transport layer (HTL2) 241, and a second electron blocking layer (EBL2) 242.

The organic electroluminescent device 100 further comprises a second emission layer (EML2) 245.

The organic electroluminescent device 100 further comprises a second optional hole blocking layer (HBL2) 247.

The organic electroluminescent device 100 further comprises a second electron transport layer (ETL2) 249, wherein the second electron transport layer (ETL2) 249 is free of 8-Hydroxyquinolinolato-lithium, preferably free of a lithium organic metal complex, and more preferably free of a lithium metal complex.

The organic electroluminescent device further comprises a second charge generation layer (CGL2) 260 disposed over the second electron transport layer (ETL2) 249, wherein the second charge generation layer (CGL2) 260 comprises a second n-type charge generation layer (n-CGL2) 261, and a first p-type charge generation layer (p-CGL2) 262, wherein the second n-type charge generation layer (n-CGL2) 261 comprises a compound of formula (I) and a metal dopant, wherein the metal dopant is selected from Ytterbium, and wherein the second electron transport layer (ETL1) 249 is in direct contact with the second n-type charge generation layer (n-CGL2) 261, wherein the second n-type charge generation layer (n-CGL2) 261 can be selected the same or different than the first n-type charge generation layer (n-CGL1) 161, and wherein the second p-type charge generation layer (p-CGL2) 262 can be selected the same or different than the first p-type charge generation layer (p-CGL1) 161.

The organic electroluminescent device 100 further comprises a third hole transport layer (HTL3) 341, and a third electron blocking layer (EBL3) 342.

The organic electroluminescent device 100 further comprises a third emission layer (EML3) 345.

The organic electroluminescent device 100 further comprises a third optional hole blocking layer (HBL3) 347, an electron transport layer (ETL)148, an electron injection layer (EIL) 180, and a cathode layer (CAT) 190.

Fig. 4 is a schematic sectional view of an organic electroluminescent device 100, according to one exemplary embodiment of the present invention.

Referring to Fig. 4 the organic electroluminescent device 100 includes an anode layer (ANO) 120, a hole injection layer (HIL) 130, a hole transport layer (HTL1) 141, a first electron blocking layer (EBL1) 142, a first emission layer (EML1) 145.

The organic electroluminescent device 100 further comprises a first optional hole blocking layer (HBL1) 147.

The organic electroluminescent device 100 further comprises a first electron transport layer (ETL1) 149, wherein the first electron transport layer (ETL1) 149 is free of 8-Hydroxyquinolinolato-lithium, preferably free of a lithium organic metal complex, and more preferably free of a lithium metal complex.

The organic electroluminescent device further comprises a first charge generation layer (CGL) 160 disposed over the first electron transport layer (ETL1) 149, wherein the first charge generation layer (CGL1) 160 comprises a first n-type charge generation layer (n-CGL) 161, and a first p-type charge generation layer (p-CGL) 162, wherein the first n-type charge generation layer (n-CGL) 161 comprises a compound of formula (I) and a metal dopant, wherein the metal dopant is selected from Ytterbium, and wherein the first electron transport layer (ETL1) 149 is in direct contact with the first n-type charge generation layer (n-CGL) 161.

The organic electroluminescent device 100 further comprises a second hole transport layer (HTL2) 241, and a second electron blocking layer (EBL2) 242.

The organic electroluminescent device 100 further comprises a second emission layer (EML2) 245.

The organic electroluminescent device 100 further comprises a second optional hole blocking layer (HBL2) 247.

The organic electroluminescent device 100 further comprises a second electron transport layer (ETL2) 249, wherein the second electron transport layer (ETL2) 249 is free of 8-Hydroxyquinolinolato-lithium, preferably free of a lithium organic metal complex, and more preferably free of a lithium metal complex.

The organic electroluminescent device further comprises a charge generation layer (CGL) 260 disposed over the second electron transport layer (ETL2) 249, wherein the second charge generation layer (CGL2) 260 comprising a second n-type charge generation layer (n-CGL) 261, and a second p-type charge generation layer (p-CGL) 262, wherein the second n-type charge generation layer (n-CGL) 261 comprises a compound of formula (I) and a metal dopant, wherein the metal dopant is selected from Ytterbium, and wherein the second electron transport layer (ETL2) 249 is in direct contact with the second n-type charge generation layer (n-CGL2) 261, wherein the second n-type charge generation layer (n-CGL2) 261 can be selected the same or different than the first n-type charge generation layer (n-CGL1) 161, and wherein the second p-type charge generation layer (p-CGL2) 262 can be selected the same or different than the first p-type charge generation layer (p-CGL1) 161.

The organic electroluminescent device 100 further comprises a third hole transport layer (HTL3) 341, and a third electron blocking layer (EBL3) 342.

The organic electroluminescent device 100 further comprises a third emission layer (EML3) 345.

The organic electroluminescent device 100 further comprises a third optional hole blocking layer (HBL3) 347.

The organic electroluminescent device 100 further comprises a third electron transport layer (ETL3) 349, wherein the third electron transport layer (ETL3) 349 is free of 8-Hydroxyquinolinolato-lithium, preferably free of a lithium organic metal complex, and more preferably free of a lithium metal complex.

The organic electroluminescent device further comprises a charge generation layer (CGL3) 360 disposed over the third electron transport layer (ETL3) 349, wherein the third charge generation layer (CGL3) 360 comprising a third n-type charge generation layer (n-CGL3) 361, and a third p-type charge generation layer (p-CGL3) 362, wherein the third n-type charge generation layer (n-CGL3) 361 comprises a compound of formula (I) and a metal dopant, wherein the metal dopant is selected from Ytterbium, and wherein the third transport layer (ETL3) 349 is in direct contact with the third n-type charge generation layer (n-CGL3) 361, wherein the third n-type charge generation layer (n-CGL3) 361 can be selected the same or different than the first n-type charge generation layer (n-CGL1) 161, and wherein the third p-type charge generation layer (p-CGL3) 362 can be selected the same or different than the first p-type charge generation layer (p-CGL1) 161.

The organic electroluminescent device 100 further comprises a fourth hole transport layer (HTL4) 441, and a fourth electron blocking layer (EBL4) 442.

The organic electroluminescent device 100 further comprises a fourth emission layer (EML4) 345.

The organic electroluminescent device 100 further comprises a fourth optional hole blocking layer (HB14) 447, an electron transport layer (ETL) 148, an electron injection layer (EIL) 180, and a cathode layer (CAT) 190.

While not shown in Fig. 1, Fig. 2, Fig. 3 and Fig. 4, a sealing layer may further be formed on the cathode electrodes 190, in order to seal the OLEDs 100. In addition, various other modifications may be applied thereto.

Hereinafter, the embodiments are illustrated in more detail with reference to examples. However, the present disclosure is not limited to the following examples.

## Detailed description

The invention is furthermore illustrated by the following examples which are illustrative only and non-binding.

Calculated LUMO and dipole moment

The LUMO energy level and the dipole moment are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

*Materials for first n-type charge generation layer are shown in Table 10:*

Table 10:

| **Type** | **Abbreviation** | **Structure** | LUMO [eV] |
|---|---|---|---|
| For comparative examples | C-1 | 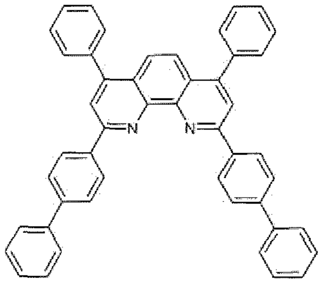 | -1.77 |
| For comparative examples | C-2 | 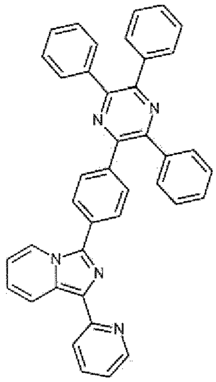 | -1.61 |
| For comparative examples | C-3 | 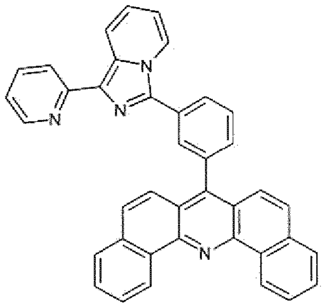 | -1.81 |
| For comparative example | C-4 | 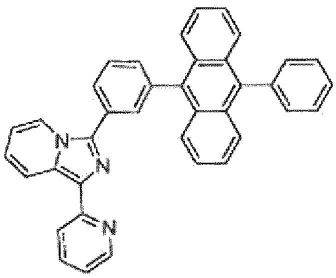 | -1.61 |
| For inventive example | I-1 | 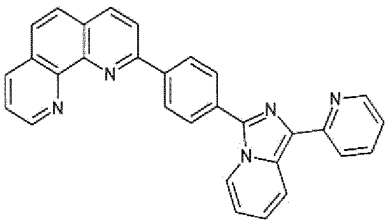 | -1.73 |

(continued)

| Type | Abbreviation | Structure | LUMO [eV] |
|---|---|---|---|
| For inventive example | I-2 | 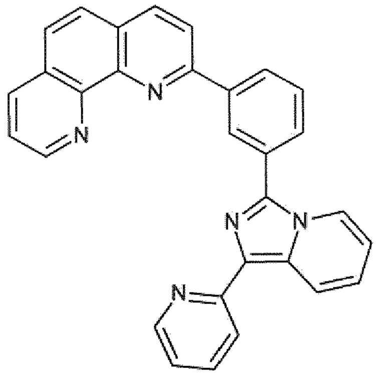 | -1.54 |

*Materials for first electron transport layer are Table 11.*

Table 11:

| Abbreviation | Structure | LUMO [eV] | Dipole moment [debye] |
|---|---|---|---|
| ET-1 | 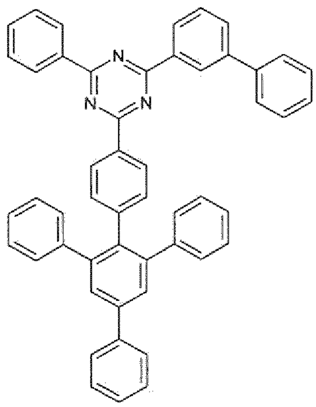 | -1.83 | 0.37 |
| ET-2 | 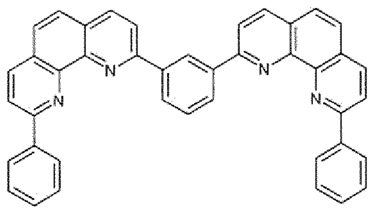 | -1.75 | 2.66 |
| ET-3 | 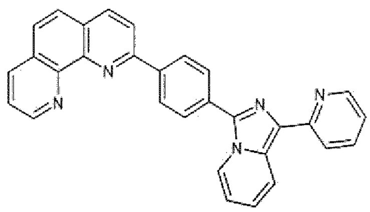 | -1.73 | 3.39 |

(continued)

| Abbreviation | Structure | LUMO [eV] | Dipole moment [debye] |
|---|---|---|---|
| ET-4 | 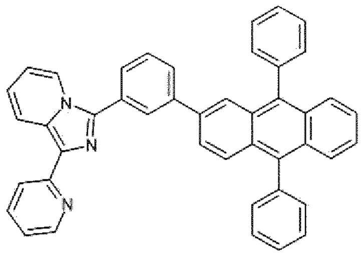 | -1.74 | 3.68 |
| ET-5 | 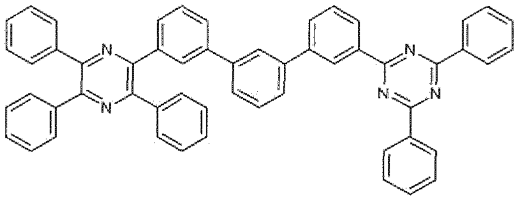 | -1.87 | 0.42 |
| ET-6 | 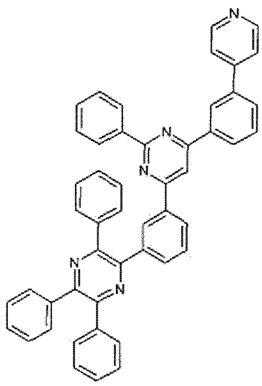 | -1.87 | 0.98 |
| ET-7 | 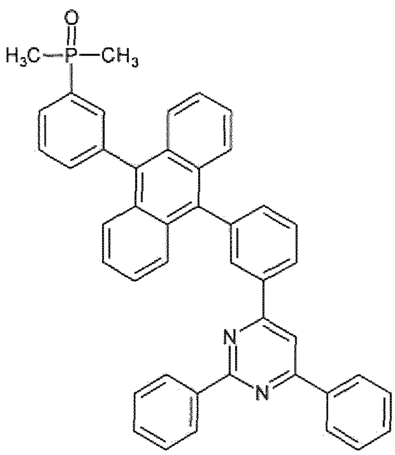 | -1.86 | 2.59 |

General procedure for fabrication of OLEDs

For the examples according to the invention and comparative examples in Table 12, a glass substrate with an anode layer comprising a first anode sub-layer of 10 nm ITO, a second anode sub-layer of 120 nm Ag and a third anode sub-layer of 8 nm ITO was cut to a size of 100 mm x 100 mm x 0.7 mm, ultrasonically washed with water for 60 minutes and then with isopropanol for 20 minutes. The liquid film was removed in a nitrogen stream, followed by plasma treatment, see Table 12, to prepare the anode layer. The plasma treatment was performed in an atmosphere comprising 97.6 vol.-% nitrogen and 2.4 vol.-% oxygen.

Then N-([1,1'-biphenyl]-4-yl)-9,9-dimethyl-N-(4-(9-phenyl-9H-carbazol-3-yl)phenyl)-9H-fluoren-2-amine was vacuum deposited with $\geq$ wt.-% 2,2',2"-(cyclopropane-1,2,3-triylidene)tris(2-(p-cyanotetrafluorophenyl)acetonitrile)to

form a hole injection layer having a thickness 10 nm.

Then N-([1,1'-biphenyl]-4-yl)-9,9-dimethyl-N-(4-(9-phenyl-9H-carbazol-3-yl)phenyl)-9H-fluoren-2-amine was vacuum deposited, to form a first hole transport layer having a thickness of 29 nm

Then N-{[1,1'-biphenyl]-4-yl)-9,9-diphenyl-N-(4-(triphenylsilyl)phenyl)-9H-fluoren-2-amine was vacuum deposited on the HTL, to form an electron blocking layer (EBL) having a thickness of 5 nm.

Then 97 wt.-% H09 (Sun Fine Chemicals, Korea) as EML host and 3 wt.-% BD200 (Sun Fine Chemicals, Korea) as fluorescent blue dopant were deposited on the EBL, to form a first blue-emitting emission layer (EML) with a thickness of 20 nm.

Then, the first electron transporting layer (ETL1) having a thickness of 15 nm is formed on first emission layer by depositing a compound according Table 12. The first electron transporting layer is free of 8-Hydroxyquinolinolato-lithium.

Then an n-type CGL having a thickness of 8 nm is formed on the ETL1 by co-depositing 90.1 wt.-% of an electron transport compound (ETM of n-CGL) according to Table 12 and 9.9 wt.-% Yb.

Then a p-type CGL having a thickness of 10 nm is formed on the n-type CGL by co-depositing N-([1,1'-biphenyl]-4-yl)-9,9-dimethyl-N-(4-(9-phenyl-9H-carbazol-3-yl)phenyl)-9H-fluoren-2-amine with 10 wt% 2,2',2"-(cyclopropane-1,2,3-triylidene)tris(2-(p-cyanotetrafluorophenyl)acetonitrile) as organic p-dopant.

Then a second hole transport layer having a thickness of 45 nm is formed on the first p-type CGL by depositing N-([1,1'-biphenyl]-4-yl)-9,9-dimethyl-N-(4-(9-phenyl-9H-carbazol-3-yl)phenyl)-9H-fluoren-2-amine.

Then a second electron blocking layer having a thickness of 5 nm is formed on the second hole transport layer by depositing N-([1,1'-biphenyl]-4-yl)-9,9-diphenyl-N-(4-(triphenylsilyl)phenyl}-9H-fluoren-2-amine.

Then 97 wt.-% H09 (Sun Fine Chemicals, Korea) as EML host and 3 wt.-% BD200 (Sun Fine Chemicals, Korea) as fluorescent blue dopant were deposited on the second EBL, to form a second blue-emitting EML with a thickness of 19 nm.

Then 2-(3'-(9,9-dimethyl-9H-fluoren-2-yl)-[1,1'-biphenyl]-3-yl)-4,6-diphenyl-1,3,5-triazine was vacuum deposited to form a second hole blocking layer having a thickness of 5 nm is formed on the second blue-emitting EML.

Then, 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% LiQ were vacuum deposited on the second hole blocking layer to form a second electron transport layer having a thickness of 31 nm.

Then Yb was evaporated at a rate of 0.01 to 1 Å/s at $10^{-7}$ mbar to form an electron injection layer with a thickness of 1 nm on the electron transporting layer.

Ag/Mg (1:8 wt%) is evaporated at a rate of 0.01 to 1 Å/s at $10^{-7}$ mbar to form a cathode with a thickness of 13 nm.

Then, N-({[1,1-'biphenyl]-4-yl)-9,9,dimethyl-N-(4-(9-phenyl-9H-carbazol-3-yl)phenyl)-9H-fluoren-2-amine} was vacuum deposited on the cathode layer to form a capping layer with a thickness of 75 nm.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between oV and 10V. Likewise, the luminance-voltage characteristics and CIE coordinates are determined by measuring the luminance in cd/m$^2$ using an Instrument Systems CAS-140CT array spectrometer (calibrated by Deutsche Akkreditierungsstelle (DAkkS)) for each of the voltage values. The cd/A efficiency at 15 mA/cm2 is determined by interpolating the luminance-voltage and current-voltage characteristics, respectively.

In bottom emission devices, the emission is predominately Lambertian and quantified in percent external quantum efficiency (EQE). To determine the efficiency EQE in % the light output of the device is measured using a calibrated photodiode at 15 mA/cm2.

In top emission devices, the emission is forward directed, non-Lambertian and also highly dependent on the mircocavity. Therefore, the efficiency EQE will be higher compared to bottom emission devices. To determine the efficiency EQE in % the light output of the device is measured using a calibrated photodiode at 15 mA/cm$^2$.

Lifetime LT of the device is measured at ambient conditions (20°C) and 30 mA/cm$^2$, using a Keithley 2400 sourcemeter, and recorded in hours.

The brightness of the device is measured using a calibrated photo diode. The lifetime LT is defined as the time till the brightness of the device is reduced to 97 % of its initial value.

The increase in operating voltage ΔU is used as a measure of the operational voltage stability of the device. This increase is determined during the LT measurement and by subtracting the operating voltage after 1 hour after the start of operation of the device from the operating voltage after 100 hours.

$$\Delta U=[U100\ h)-U(1h)].$$

The smaller the value of ΔU the better is the operating voltage stability.

## Technical Effect of the invention

Table 12:

| Device | ETL | ETL<br>ETL dopant | n-CGL | CIE-Y | Voltage in relative percent to C1 [%] | Ceff/CIE-Y[cd/A] relative to C1 [%] | Voltage rise 30 mA/cm$^2$ (1-100h) [V] |
|---|---|---|---|---|---|---|---|
| C1 | ET-1 | LiQ | C-4 | 0.050 | 100 | 100 | 0.193 |
| C2 | ET-1 | LiQ | I-1 | 0.050 | 98.7 | 101.0 | 0.185 |
| C3 | ET-1 | LiQ | I-2 | 0.050 | 99.0 | 101.0 | 0.159 |
| C4 | ET-1 | --- | ET-2 | 0.050 | 101.3 | 102.1 | 0.233 |
| C5 | ET-1 | --- | C-2 | 0.051 | 100.6 | 99.8 | 0.232 |
| C6 | ET-1 | --- | C-3 | 0.052 | 101.9 | 99.4 | 0.326 |
| C7 | ET-1 | --- | C-4 | 0.050 | 99.0 | 100 | 0.172 |
| C8 | ET-1 | --- | C-1 | 0.052 | 135.7 | 81.4 | 0.463 |
| I1 | ET-1 | --- | I-1 | 0.052 | 98.1 | 99.0 | 0.098 |
| I2 | ET-1 | --- | I-2 | 0.050 | 99.0 | 100 | 0.119 |
| I3 | ET-2 | --- | I-1 | 0.056 | 98.4 | 97.2 | 0.128 |
| I4 | ET-5 | --- | I-1 | 0.056 | 98.4 | 99.2 | 0.146 |
| I5 | ET-4 | --- | I-1 | 0.052 | 97.6 | 96.1 | 0.076 |
| I6 | ET-6 | --- | I-1 | 0.055 | 98.7 | 99.0 | 0.125 |
| I7 | ET-3 | --- | I-1 | 0.055 | 98.5 | 93.4 | 0.102 |
| I8 | ET-7 | --- | I-1 | 0.053 | 100 | 94.8 | 0.091 |

The comparative device C1 comprises an electron transport layer contacting the n-type charge generation layer containing 8-Hydroxyquinolinolato-lithium (LiQ) and compound ET-1, and an n-type charge generation layer containing compound C-4.

The comparative device C2 comprises an electron transport layer contacting the n-type charge generation layer containing 8-Hydroxyquinolinolato-lithium (LiQ) and compound ET-1, and an n-type charge generation layer containing compound I-1.

The comparative device C3 comprises an electron transport layer contacting the n-type charge generation layer containing 8-Hydroxyquinolinolato-lithium (LiQ) and compound ET-1, and an n-type charge generation layer containing compound I-2.

The comparative device C4 comprises an electron transport layer contacting the n-type charge generation layer containing the compound ET-1 and in contrast to comparative device C1 does not contain 8-Hydroxyquinolinolato-lithium (LiQ), and an n-type charge generation layer containing compound ET-2. The compound ET-2 contains two phenthroline groups.

The comparative device C5 comprises an electron transport layer contacting the n-type charge generation layer containing the compound C-2 and does not contain 8-Hydroxyquinolinolato-lithium (LiQ),, and an n-type charge generation layer containing compound C-2. The compound C-2 contains a pyrazine group and a imidazo[1,5-a]pyridine group.

The comparative device C6 comprises an electron transport layer contacting the n-type charge generation layer containing the compound C-3 and does not contain 8-Hydroxyquinolinolato-lithium (LiQ), and an n-type charge generation layer containing compound C-3. The compound C-3 contains a dibenzoacridine group and a imidazo[1,5-a]pyridine group.

The comparative device C7 comprises an electron transport layer contacting the n-type charge generation layer containing the compound C-4 and does not contain 8-Hydroxyquinolinolato-lithium (LiQ), and an n-type charge generation layer containing compound C-4. The compound C-4 contains a dibenzoacridine group and a substituted imidazo[1,5-a] group.

The comparative device C8 comprises an electron transport layer contacting the n-type charge generation layer

containing the compound C-1 and the electron transport layer does not contain 8-Hydroxyquinolinolato-lithium (LiQ), and an n-type charge generation layer containing compound C-1. The compound C-1 contains one phenthroline group.

The inventive device I1 comprises an electron transport layer contacting the n-type charge generation layer containing the compound ET-1, and in contrast to comparative device C3 the electron transport layer does not contain 8-Hydroxyquinolinolato-lithium (LiQ), and the n-type charge generation layer containing compound ET-3. The compound ET-3 contains a phenthroline group and a substituted imidazo[1,5-a] group.

The inventive device I2 comprises an electron transport layer contacting the n-type charge generation layer containing the compound ET-1, and in contrast to comparative device C3 the electron transport layer does not contain 8-Hydroxyquinolinolato-lithium (LiQ), and the n-type charge generation layer containing compound I-2. The compound I-2 contains a phenthroline group and a substituted imidazo[1,5-a] group.

The inventive device I3 comprises an electron transport layer contacting the n-type charge generation layer containing the compound ET-2 instead of the compound ET-1 according to inventive device I1, and the electron transport layer does not contain 8-Hydroxyquinolinolato-lithium (LiQ), and the n-type charge generation layer containing compound ET-3.

The inventive device I4 comprises an electron transport layer contacting the n-type charge generation layer containing the compound ET-5 instead of the compound ET-1 according to inventive device I1, and the electron transport layer does not contain 8-Hydroxyquinolinolato-lithium (LiQ), and the n-type charge generation layer containing compound ET-3.

The inventive device I5 comprises an electron transport layer contacting the n-type charge generation layer containing the compound ET-4 instead of the compound ET-1 according to inventive device I1, and the electron transport layer does not contain 8-Hydroxyquinolinolato-lithium (LiQ), and the n-type charge generation layer containing compound ET-3.

In inventive device 16, the organic electroluminescent device comprises an electron transport layer contacting the n-type charge generation layer containing the compound ET-6 instead of the compound ET-1 according to inventive device I1, and the electron transport layer does not contain 8-Hydroxyquinolinolato-lithium (LiQ), and the n-type charge generation layer containing compound ET-3.

The inventive device I7 comprises an electron transport layer contacting the n-type charge generation layer containing the compound ET-3 instead of the compound ET-1 according to inventive device I1, and the electron transport layer does not contain 8-Hydroxyquinolinolato-lithium (LiQ), and the n-type charge generation layer containing compound ET-3.

The inventive device I8 comprises an electron transport layer contacting the n-type charge generation layer containing the compound ET-7 instead of the compound ET-1 according to inventive device I1, and the electron transport layer does not contain 8-Hydroxyquinolinolato-lithium (LiQ), and the n-type charge generation layer containing compound ET-3.

In the comparative example C1 and C5, compound ET-2 is used in the n-type charge generation layer. In comparative example C5, the electron transport layer contacting the n-type charge generation layer does not contain Hydroxyquinolinolato-lithium (LiQ) in contrast to comparative example C1. However, it is apparent that the voltage rise over time is still very high.

In the comparative device C1 and C5, compound C-4 is used in the n-type charge generation layer. In comparative example C5, the electron transport layer contacting the n-type charge generation layer does not contain Hydroxyquinolinolato-lithium (LiQ) in contrast to comparative example C1. However, it is apparent that the voltage rise over time is still very high.

The inventive device I3 or I4, respectively differs from the comparative device C3 or C4, respectively in that the inventive device I3 or I4, respectively does not contain Hydroxyquinolinolato-lithium in the electron transport layer contacting the n-type charge generation layer.

The inventive device I1 to I7 does not comprise Hydroxyquinolinolato-lithium in the electron transport layer contacting the n-type charge generation layer.

The comparative devices C1 to C5 contains Hydroxyquinolinolato-lithium in the electron transport layer contacting the n-type charge generation layer.

The inventive devices I1 to I7 further differ from the comparative device C1 to C4 in that the inventive devices I1 to I7 contain a compound according to formula (I) in the n-type charge generation layer.

It is apparent that all inventive devices exhibit a remarkable lower voltage rise over time compared to the comparative devices C1 to C4. At the same time the operational voltage is still low, and the current efficiency is still very high.

The inventive device I1 to I7 and the comparative devices C5 to C9, all does not comprise Hydroxyquinolinolato-lithium in the electron transport layer contacting the n-type charge generation layer.

The inventive devices I1 to I7 differ from the comparative device C5 to C9 in that the inventive devices I1 to I7 contain a compound according to formula (I) in the n-type charge generation layer.

It is apparent that all inventive devices exhibit a lower voltage rise over time compared to the comparative devices C5 to C9. At the same time the operational voltage is still low, and the current efficiency is still very high.

Thus, an inventive device comprising a compound of formula (I) in the n-type charge generation layer, and an electron transport layer contacting the n-type charge generation layer which is free of Hydroxyquinolinolato-lithium exhibit a remarkable low voltage rise over time, and at the same time a low operational voltage and a high current efficiency.

A low operating voltage may be important for the battery life of organic electronic devices, in particular mobile devices.

A high efficiency may be beneficial for reduced power consumption and improved battery life, in particular in mobile devices.

A low voltage rise over time may result in improved long-term stability of electronic devices.

The present invention is defined by the appended claims.

## Claims

1. An organic electroluminescent device (100) comprising an anode layer (120), a cathode layer (190), a first emission layer (145), a second emission layer (245), a first charge generation layer (160), and a first electron transport layer (149), wherein

    - the first charge generation layer is arranged between the first emission layer and the second emission layer;
    - the first electron transport layer is arranged between the first emission layer and the second emission layer;
    - the first charge generation layer comprises a first n-type charge generation layer (161) and a first p-type charge generation layer (162),
    - the first n-type charge generation layer is closer to the anode layer than the first p-type charge generation layer and the first p-type charge generation layer is closer to the cathode layer than the first n-type charge generation layer;
    - the first electron transport layer is arranged in direct contact with the first n-type charge generation layer;
    - the first n-type charge generation layer comprises a metal dopant and the metal dopant is Yb;
    - the first n-type charge generation layer comprises a compound of formula (I)

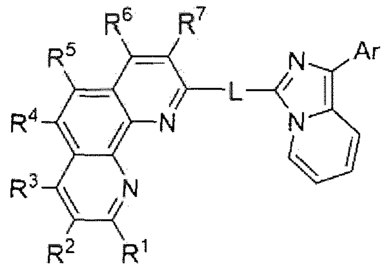

(I);

    wherein in formula (I)

    - Ar is selected from substituted or unsubstituted $C_6$ to $C_{24}$ aryl or substituted or unsubstituted $C_2$ to $C_{24}$ heteroaryl;
    - $R^1$ to $R^7$ are independently selected from H, D, substituted or unsubstituted $C_1$ to $C_{16}$ alkyl, substituted or unsubstituted $C_6$ to $C_{24}$ aryl or substituted or unsubstituted $C_2$ to $C_{24}$ heteroaryl, CN, halogen, F, $C_1$ to $C_{16}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, or $PX^1(R^8)_2$ wherein $R^8$ is independently selected from $C_6$ to $C_{12}$ aryl, $C_3$ to $C_{12}$ heteroaryl, $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy; and $X^1$ is selected from O, S or Se, preferably O; and
    - L is selected from substituted or unsubstituted $C_6$ to $C_{24}$ aryl, substituted or unsubstituted $C_2$ to $C_{24}$ heteroaryl; **characterized in that** the first electron transport layer is free of 8-hydroxyquinolinolato-lithium.

2. The organic electroluminescent device according to claim 1, wherein Ar is selected from substituted or unsubstituted $C_6$ to $C_{12}$ aryl or substituted or unsubstituted $C_2$ to $C_{12}$ heteroaryl.

**3.** The organic electroluminescent device according to claim 1 or 2, wherein Ar is selected from the group consisting of substituted or unsubstituted phenyl or substituted or unsubstituted pyridinyl, substituted or unsubstituted pyrazinyl, substituted or unsubstituted pyrimidyl, substituted or unsubstituted triazinyl, and substituted or unsubstituted quinolinyl.

**4.** The organic electroluminescent device according to any of the preceding claims, wherein $R^1$ to $R^7$ are independently selected from the group consisting of H, D, substituted or unsubstituted $C_1$ to $C_6$ alkyl, substituted or unsubstituted $C_6$ to $C_{12}$ aryl or substituted or unsubstituted $C_2$ to $C_{12}$ heteroaryl, and halogen.

**5.** The organic electroluminescent device according to any of the preceding claims, wherein $R^1$ to $R^7$ are independently selected from the group consisting of H, D, substituted or unsubstituted $C_1$ to $C_4$ alkyl, substituted or unsubstituted $C_6$ aryl or substituted or unsubstituted $C_3$ to $C_5$ heteroaryl, and halogen.

**6.** The organic electroluminescent device according to any of the preceding claims, wherein L is selected from the group consisting of substituted or unsubstituted $C_6$ to $C_{12}$ aryl or substituted or unsubstituted $C_2$ to $C_{12}$ heteroaryl.

**7.** The organic electroluminescent device according to any of the preceding claims, wherein L is selected from the group consisting of substituted or unsubstituted phenyl.

**8.** The organic electroluminescent device according to any of the preceding claims, wherein the compound of formula (I) has a LUMO energy level when calculated with the program package TURBOMOLE V6.5 by applying the hybrid functional B3LYP with a 6-31G* basis set in the gas phase in the range of $\geq$ -1.8 eV to $\leq$ -1.4 eV.

**9.** The organic electroluminescent device according to any of the preceding claims, wherein the compound of formula (I) has a molecular weight in the range of $\geq$400 g/mol to $\leq$2000 g/mol.

**10.** The organic electroluminescent device according to any of the preceding claims, wherein the first electron transport layer is closer to the anode layer than any other electron transport layer within the organic electroluminescent device.

**11.** The organic electroluminescent device according to any of the preceding claims, wherein the first electron transport layer is in direct contact with the first emission layer.

**12.** The organic electroluminescent device according to any of the preceding claims, wherein the first electron transport layer is free of a metal complex.

**13.** The organic electroluminescent device according to any of the preceding claims, wherein the first electron transport layer comprises an electron transport compound, wherein

- the electron transport compound has a molecular weight in the range of $\geq$400 g/mol to $\leq$2000 g/mol; and
- electron transport compound may comprise at least one N-containing heteroaromatic ring.

**14.** The organic electroluminescent device according to any of the preceding claims, wherein the organic electroluminescent device further comprises a hole injection layer (130), a first hole transport layer (141), a second hole transport layer -(241), first electron blocking layer (142), second electron blocking layer (242), optionally a first hole blocking layer, optionally a second hole blocking layer (147), a second electron transport layer (249), and an electron injection layer (180).

**15.** Display device comprising the organic electroluminescent device according to any of the preceding claims.

## Patentansprüche

**1.** Organische Elektrolumineszenzvorrichtung, umfassend eine Anodenschicht, eine Kathodenschicht, eine erste Emissionsschicht, eine zweite Emissionsschicht, eine erste Ladungserzeugungsschicht und eine erste Elektronentransportschicht,
wobei

- die erste Ladungserzeugungsschicht zwischen der ersten Emissionsschicht und der zweiten Emissionsschicht

angeordnet ist;
- die erste Elektronentransportschicht zwischen der ersten Emissionsschicht und der zweiten Emissionsschicht angeordnet ist;
- die erste Ladungserzeugungsschicht eine erste Ladungserzeugungsschicht vom n-Typ und eine erste Ladungserzeugungsschicht vom p-Typ umfasst;
- die erste Ladungserzeugungsschicht vom n-Typ näher an der Anodenschicht als die erste Ladungserzeugungsschicht vom p-Typ ist und die erste Ladungserzeugungsschicht vom p-Typ näher an der Kathodenschicht als die erste Ladungserzeugungsschicht vom n-Typ ist;
- die erste Elektronentransportschicht in direktem Kontakt mit der ersten Ladungserzeugungsschicht vom n-Typ angeordnet ist;
- die erste Elektronentransportschicht frei von 8-Hydroxychinolinolato-Lithium ist;
- die erste Ladungserzeugungsschicht vom n-Typ einen Metalldotierstoff umfasst und der Metalldotierstoff Yb ist;
- die erste Ladungserzeugungsschicht vom n-Typ eine Verbindung der Formel (I) umfasst

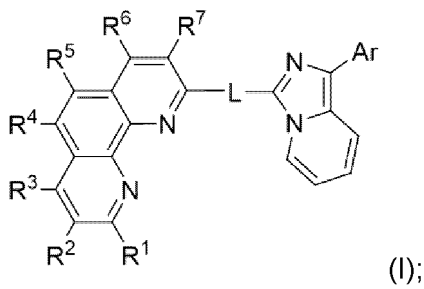

(I);

wobei in Formel (I)

- Ar aus substituiertem oder unsubstituiertem $C_6$ - bis $C_{24}$ -Aryl oder substituiertem oder unsubstituiertem $C_2$ - bis $C_{24}$ -Heteroaryl ausgewählt ist;
- $R^1$ bis $R^7$ unabhängig aus H, D, substituiertem oder unsubstituiertem $C_1$ - bis $C_{16}$ - Alkyl, substituiertem oder unsubstituiertem $C_6$ - bis $C_{24}$ -Aryl oder substituiertem oder unsubstituiertem $C_2$ - bis $C_{24}$ -Heteroaryl, CN, Halogen, F, $C_1$ - bis $C_{16}$ -Alkoxy, verzweigtem $C_3$ - bis $C_{16}$ -Alkyl, cyclischem $C_3$ - bis $C_{16}$ -Alkyl, verzweigtem $C_3$ - bis $C_{16}$ -Alkoxy, cyclischem $C_3$ - bis $C_{16}$ -Alkoxy, teilweise oder perfluoriertem $C_1$ - bis $C_{16}$ -Alkyl, teilweise oder perfluoriertem $C_1$ - bis $C_{16}$ -Alkoxy, teilweise oder perdeuteriertem $C_1$- bis $C_{16}$ -Alkyl, teilweise oder perdeuteriertem $C_1$ - bis $C_{16}$ -Alkoxy oder $PX^1(R^8)_2$ ausgewählt sind, wobei $R^8$ unabhängig aus $C_6$ - bis $C_{12}$ -Aryl, $C_3$ - bis $C_{12}$ -Heteroaryl, $C_1$ - bis $C_{16}$ -Alkyl, $C_1$ - bis $C_{16}$ -Alkoxy, teilweise oder perfluoriertem $C_1$ - bis $C_{16}$ -Alkyl, teilweise oder perfluoriertem $C_1$ - bis $C_{16}$ -Alkoxy, teilweise oder perdeuteriertem $C_1$ - bis $C_{16}$ -Alkyl, teilweise oder perdeuteriertem $C_1$ - bis $C_{16}$ -Alkoxy ausgewählt ist; und $X^1$ aus O, S oder Se, vorzugsweise O, ausgewählt ist; und
- L aus substituiertem oder unsubstituiertem $C_6$ - bis $C_{24}$ -Aryl, substituiertem oder unsubstituiertem $C_2$ - bis $C_{24}$ -Heteroaryl ausgewählt ist.

**2.** Organische Elektrolumineszenzvorrichtung nach Anspruch 1, wobei Ar aus substituiertem oder unsubstituiertem $C_6$ - bis $C_{12}$ -Aryl oder substituiertem oder unsubstituiertem $C_2$ - bis $C_{12}$ -Heteroaryl ausgewählt ist.

**3.** Organische Elektrolumineszenzvorrichtung nach Anspruch 1 oder 2, wobei Ar aus der Gruppe bestehend aus substituiertem oder unsubstituiertem Phenyl oder substituiertem oder unsubstituiertem Pyridinyl, substituiertem oder unsubstituiertem Pyrazinyl, substituiertem oder unsubstituiertem Pyrimidyl, substituiertem oder unsubstituiertem Triazinyl und substituiertem oder unsubstituiertem Chinolinyl ausgewählt ist.

**4.** Organische Elektrolumineszenzvorrichtung nach einem der vorhergehenden Ansprüche, wobei $R^1$ bis $R^7$ unabhängig aus der Gruppe bestehend aus H, D, substituiertem oder unsubstituiertem $C_1$ - bis $C_6$ -Alkyl, substituiertem oder unsubstituiertem $C_6$ - bis $C_{12}$ -Aryl oder substituiertem oder unsubstituiertem $C_2$ - bis $C_{12}$ -Heteroaryl und Halogen ausgewählt sind.

**5.** Organische Elektrolumineszenzvorrichtung nach einem der vorhergehenden Ansprüche, wobei $R^1$ bis $R^7$ unabhängig aus der Gruppe bestehend aus H, D, substituiertem oder unsubstituiertem $C_1$ - bis $C_4$ -Alkyl, substituiertem oder unsubstituiertem $C_6$ -Aryl oder substituiertem oder unsubstituiertem $C_3$ - bis $C_5$ -Heteroaryl und Halogen ausgewählt sind.

**6.** Organische Elektrolumineszenzvorrichtung nach einem der vorhergehenden Ansprüche, wobei L aus der Gruppe bestehend aus substituiertem oder unsubstituiertem $C_6$ - bis $C_{12}$ -Aryl oder substituiertem oder unsubstituiertem $C_2$ - bis $C_{12}$ -Heteroaryl ausgewählt ist.

**7.** Organische Elektrolumineszenzvorrichtung nach einem der vorhergehenden Ansprüche, wobei L aus der Gruppe bestehend aus substituiertem oder unsubstituiertem Phenyl ausgewählt ist.

**8.** Organische Elektrolumineszenzvorrichtung nach einem der vorhergehenden Ansprüche, wobei die Verbindung der Formel (I) ein LUMO-Energieniveau aufweist, wenn sie mit dem Programmpaket TURBOMOLE V6.5 berechnet wird, indem das hybridfunktionelle **B3LYP mit** einem 6-31G*-Basissatz in der Gasphase im Bereich von ≥ -1,8 eV bis ≤ -1,4 eV angewendet wird.

**9.** Organische Elektrolumineszenzvorrichtung nach einem der vorhergehenden Ansprüche, wobei die Verbindung der Formel (I) ein Molekulargewicht im Bereich von ≥ 400 g/mol bis ≤ 2000 g/mol aufweist.

**10.** Organische Elektrolumineszenzvorrichtung nach einem der vorhergehenden Ansprüche, wobei die erste Elektronentransportschicht näher an der Anodenschicht als jede andere Elektronentransportschicht innerhalb der organischen Elektrolumineszenzvorrichtung ist.

**11.** Organische Elektrolumineszenzvorrichtung nach einem der vorhergehenden Ansprüche, wobei die erste Elektronentransportschicht in direktem Kontakt mit der ersten Emissionsschicht ist.

**12.** Organische Elektrolumineszenzvorrichtung nach einem der vorhergehenden Ansprüche, wobei die erste Elektronentransportschicht frei von einem Metallkomplex ist.

**13.** Organische Elektrolumineszenzvorrichtung nach einem der vorhergehenden Ansprüche, wobei die erste Elektronentransportschicht eine Elektronentransportverbindung umfasst, wobei

- die Elektronentransportverbindung ein Molekulargewicht im Bereich von ≥ 400 g/mol bis ≤ 2000 g/mol aufweist; und
- die Elektronentransportverbindung mindestens einen N-haltigen heteroaromatischen Ring umfassen kann.

**14.** Organische Elektrolumineszenzvorrichtung nach einem der vorhergehenden Ansprüche, wobei die organische Elektrolumineszenzvorrichtung ferner eine Lochinjektionsschicht, eine erste Lochtransportschicht, eine zweite Lochtransportschicht, eine erste Elektronenblockierschicht, eine zweite Elektronenblockierschicht, optional eine erste Lochblockierschicht, optional eine zweite Lochblockierschicht, eine zweite Elektronentransportschicht und eine Elektroneninjektionsschicht umfasst.

**15.** Anzeigevorrichtung, umfassend die organische Elektrolumineszenzvorrichtung nach einem der vorhergehenden Ansprüche.

## Revendications

**1.** Dispositif électroluminescent organique (100) comprenant une couche d'anode (120), une couche de cathode (190), une première couche d'émission (145), une seconde couche d'émission (245), une première couche de génération de charge (160) et une première couche de transport d'électrons (149), dans lequel

- la première couche de génération de charge est disposée entre la première couche d'émission et la seconde couche d'émission ;
- la première couche de transport d'électrons est disposée entre la première couche d'émission et la seconde couche d'émission ;
- la première couche de génération de charge comprend une première couche de génération de charge de type n (161) et une première couche de génération de charge de type p (162),
- la première couche de génération de charge de type n est plus proche de la couche anodique que la première couche de génération de charge de type p et la première couche de génération de charge de type p est plus proche de la couche cathodique que la première couche de génération de charge de type n ;
- la première couche de transport d'électrons est en contact direct avec la première couche de génération de

charge de type n ;

- la première couche de génération de charge de type n comprend un dopant métallique et le dopant métallique est Yb ;
- la première couche de génération de charge de type n comprend un composé de formule (I)

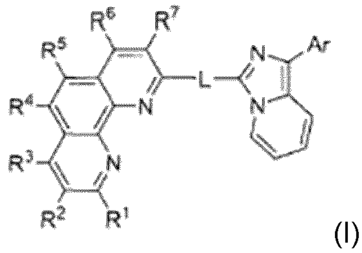

(I) ;

dans laquelle, dans la formule (I)

- Ar est choisi parmi les aryles en $C_6$ à $C_{24}$ substitués ou non substitués ou les hétéroaryles en $C_2$ à $C_{24}$ substitués ou non substitués ;
- les groupes $R^1$ à $R^7$ sont indépendamment choisis parmi H, D, un alkyle en $C_1$ à $C_{16}$ substitué ou non, un aryle en $C_6$ à $C_{24}$ substitué ou non ou un hétéroaryle en $C_2$ à $C_{24}$ substitué ou non, CN, un halogène, F, un alcoxy en $C_1$ à $C_{16}$, un alkyle ramifié en $C_3$ à $C_{16}$, un alkyle cyclique en $C_3$ à $C_{16}$, un alcoxy ramifié en $C_3$ à $C_{16}$, un alcoxy cyclique en $C_3$ à $C_{16}$, un alkyle en $C_1$ à $C_{16}$ partiellement ou perfluoré, un alcoxy en $C_1$ à $C_{16}$ partiellement ou perfluoré, un alkyle en $C_1$ à $C_{16}$ partiellement ou perdeutéré, un alcoxy en $C_1$ à $C_{16}$ partiellement ou perdeutéré, ou $PX^1(R^8)_2$ dans laquelle le groupe $R^8$ est indépendamment choisi parmi un aryle en $C_6$ à $C_{12}$, un hétéroaryle en $C_3$ à $C_{12}$, un alkyle en $C_1$ à $C_{16}$, un alcoxy en $C_1$ à $C_{16}$, un alkyle en $C_1$ à $C_{16}$ partiellement ou perfluoré, un alcoxy en $C_1$ à $C_{16}$ partiellement ou perfluoré, un alkyle en $C_1$ à $C_{16}$ partiellement ou perdeutéré, un alcoxy en $C_1$ à $C_{16}$ partiellement ou perdeutéré ;

et le groupe $X^1$ est choisi parmi O, S ou Se, de préférence O ; et

- le groupe L est choisi parmi un aryle en $C_6$ à $C_{24}$ substitué ou non substitué, un hétéroaryle en $C_2$ à $C_{24}$ substitué ou non substitué ; **caractérisé en ce que** la première couche de transport d'électrons est exempte de 8-hydroxyquinolinolatolithium.

**2.** Dispositif organique électroluminescent selon la revendication 1, dans lequel Ar est choisi parmi un aryle en $C_6$ à $C_{12}$ substitué ou non substitué ou un hétéroaryle en $C_2$ à $C_{12}$ substitué ou non substitué.

**3.** Dispositif électroluminescent organique selon la revendication 1 ou la revendication 2, dans lequel Ar est choisi dans le groupe constitué par un phényle substitué ou non substitué, un pyridinyle substitué ou non substitué, un pyrazinyle substitué ou non substitué, un pyrimidyle substitué ou non substitué, un triazinyle substitué ou non substitué, et un quinolinyle substitué ou non substitué.

**4.** Dispositif électroluminescent organique selon l'une quelconque des revendications précédentes, dans lequel les groupes $R^1$ à $R^7$ sont indépendamment choisis dans le groupe constitué par H, D, un alkyle en $C_1$ à $C_6$ substitué ou non, un aryle en $C_6$ à $C_{12}$ substitué ou non, un hétéroaryle en $C_2$ à $C_{12}$ substitué ou non, et un halogène.

**5.** Dispositif électroluminescent organique selon l'une quelconque des revendications précédentes, dans lequel les groupes $R^1$ à $R^7$ sont indépendamment choisis dans le groupe constitué par H, D, un alkyle en $C_1$ à $C_4$ substitué ou non substitué, un aryle en $C_6$ substitué ou non substitué, un hétéroaryle en $C_3$ à $C_5$ substitué ou non substitué, et un halogène.

**6.** Dispositif organique électroluminescent selon l'une quelconque des revendications précédentes, dans lequel le groupe L est choisi dans le groupe constitué par un aryle $C_6$ à $C_{12}$ substitué ou non substitué ou un hétéroaryle $C_2$ à $C_{12}$ substitué ou non substitué.

**7.** Dispositif organique électroluminescent selon l'une quelconque des revendications précédentes, dans lequel le groupe L est choisi dans le groupe constitué par un phényle substitué ou non substitué.

8. Dispositif électroluminescent organique selon l'une quelconque des revendications précédentes, dans lequel le composé de formule (I) possède un niveau d'énergie LUMO calculé à l'aide du progiciel TURBO MOLE V6.5 en appliquant la fonctionnelle hybride B3LYP avec un ensemble de base 6-31G* en phase gazeuse dans la plage comprise entre ≥ 1,8 eV et ≤ -1,4 eV.

9. Dispositif électroluminescent organique selon l'une quelconque des revendications précédentes, dans lequel le composé de formule (I) a une masse moléculaire dans la plage comprise entre ≥ 400 g/mol et ≤ 2 000 g/mol.

10. Dispositif électroluminescent organique selon l'une quelconque des revendications précédentes, dans lequel la première couche de transport d'électrons est plus proche de la couche anodique que toute autre couche de transport d'électrons dans le dispositif électroluminescent organique.

11. Dispositif organique électroluminescent selon l'une quelconque des revendications précédentes, dans lequel la première couche de transport d'électrons est en contact direct avec la première couche d'émission.

12. Dispositif organique électroluminescent selon l'une quelconque des revendications précédentes, dans lequel la première couche de transport d'électrons est exempte de complexe métallique.

13. Dispositif organique électroluminescent selon l'une quelconque des revendications précédentes, dans lequel la première couche de transport d'électrons comprend un composé de transport d'électrons, dans lequel

- le composé de transport d'électrons possède un poids moléculaire compris entre ≥ 400 g/mol et ≤ 2 000 g/mol ; et
- le composé de transport d'électrons peut comprendre au moins un anneau hétéroaromatique contenant de l'azote.

14. Dispositif électroluminescent organique selon l'une quelconque des revendications précédentes, dans lequel le dispositif électroluminescent organique comprend en outre une couche d'injection de trous (130), une première couche de transport de trous (141), une seconde couche de transport de trous (241), une première couche de blocage d'électrons (142), une seconde couche de blocage d'électrons (242), éventuellement une première couche de blocage de trous, éventuellement une seconde couche de blocage de trous (147), une seconde couche de transport d'électrons (249), et une couche d'injection d'électrons (180).

15. Dispositif d'affichage comprenant le dispositif électroluminescent organique selon l'une quelconque des revendications précédentes.

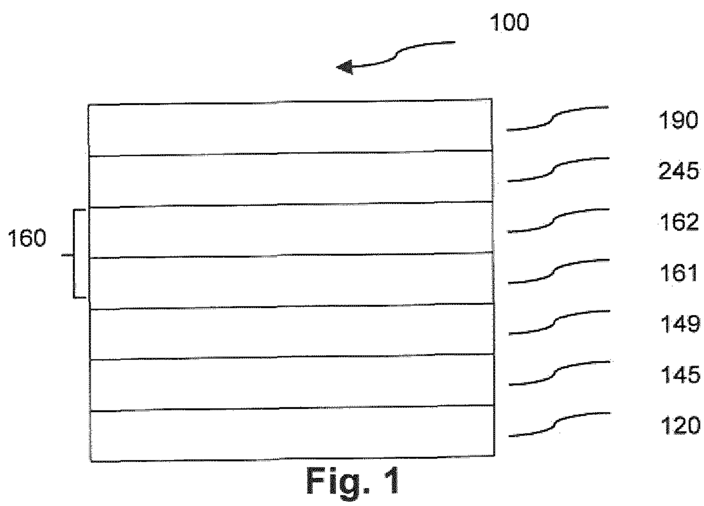


Fig. 1

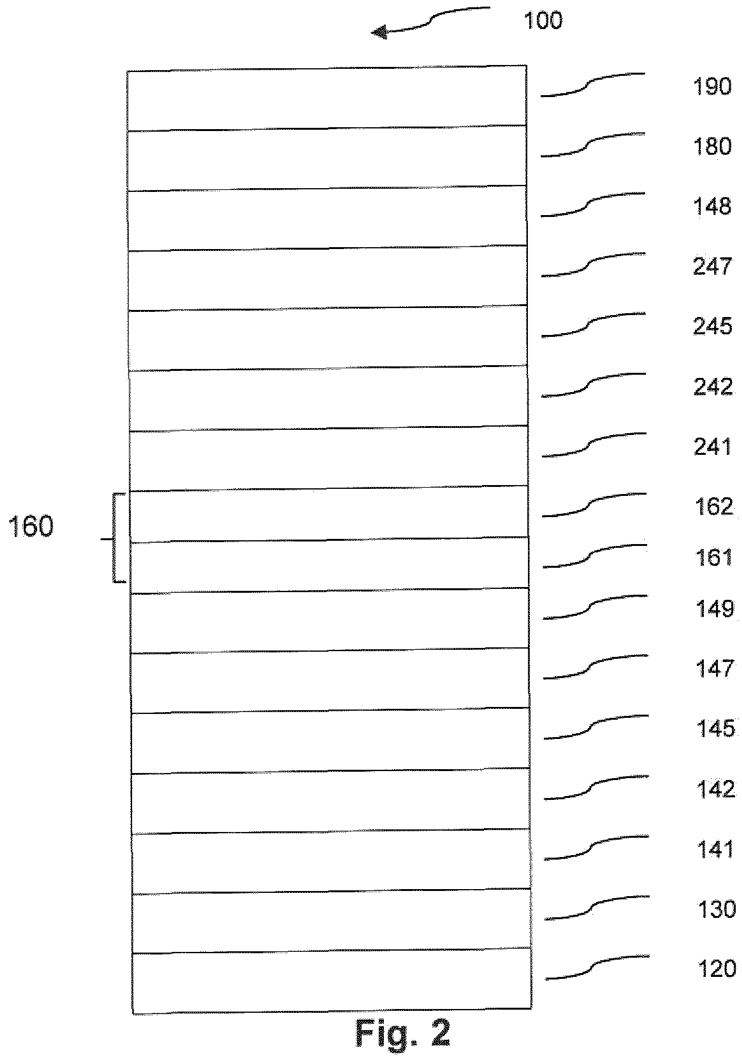
100
190
180
148
247
245
242
241
162
161
149
147
145
142
141
130
120
160

Fig. 2

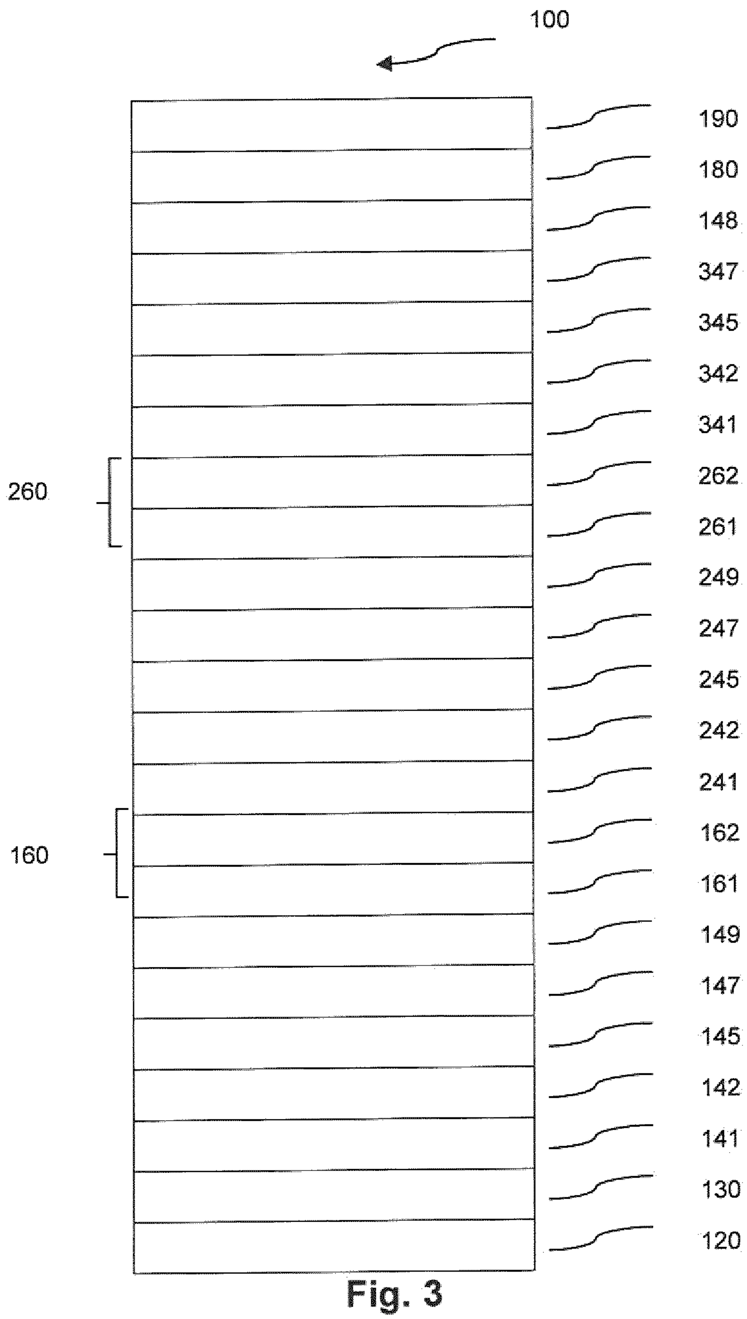
100
190
180
148
347
345
342
341
262
261
249
247
245
242
241
162
161
149
147
145
142
141
130
120
260
160

Fig. 3

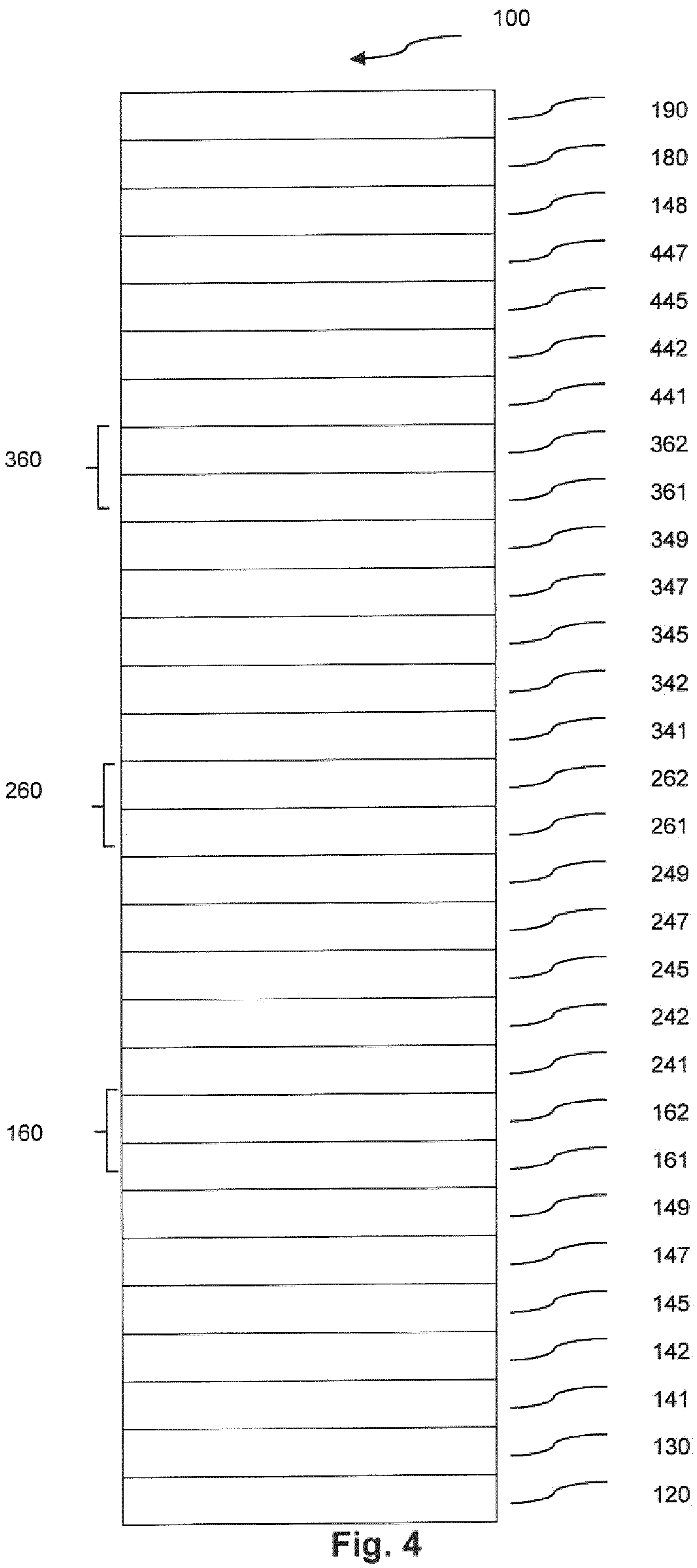
100
190
180
148
447
445
442
441
362
361
349
347
345
342
341
262
261
249
247
245
242
241
162
161
149
147
145
142
141
130
120
360
260
160

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3828951 A1
- EP 1970371 A1
- WO 2013079217 A1
- EP 2722908 A1


### Non-patent literature cited in the description


- **YASUHIKO SHIROTA** ; **HIROSHI KAGEYAMA**. *Chem. Rev.*, 2007, vol. 107, 953-1010